# EUROPEAN PATENT SPECIFICATION

(11) **EP 1 163 256 B1**
(45) Date of publication and mention of the grant of the patent: **18.02.2004**
(21) Application number: 00918365.8
(22) Date of filing: 23.03.2000
(51) Int. Cl.: C07J 1/00, C07J 3/00, A61K 31/565

(54) **HEMIHYDRATE OF 16.ALPHA.-BROMOEPIANDROSTERONE**
HEMIHYDRAT VON 16.ALPHA.-BROMOEPIANDROSTERONE
SEMI-HYDRATE DE 16.ALPHA.-BROMOEPIANDROSTERONE

(30) Priority: 23.03.1999 US 126056 P; 16.06.1999 US 140028 P; 08.10.1999 US 414905; 08.11.1999 US 164048 P
(43) Date of publication of application: 19.12.2001
(73) Proprietor: Hollis-Eden Pharmaceuticals Inc., San Diego, CA 92121 (US)
(72) Inventor: AHLEM, Clarence Nathaniel, San Diego, CA 92122 (US); FRINCKE, James Martin, San Diego, CA 92192 (US); DE CARVALHO DOS ANJOS, Luis Daniel, Paio Pires, 2840 102 Seixal (PT); HEGGIE, William, 2950 656 Palmela (PT); PRENDERGAST, Patrick T., County Kildare (IE); READING, Christopher L., San Diego, CA 92112-3511 (US); THADIKONDA, Krupakar Paul, Gaithersburg, MD 20879 (US); VERNON, Russell Neil, Oak Hills, CA 92345 (US)
(74) Representative: Kaiser, Jürgen, Dr.rer.nat.Dipl.-Chem.
(86) International application number: PCT/US2000/007883
(87) International publication number: WO 2000/056757

(56) References cited:
- EP-A- 0 133 995
- EP-A- 0 289 327
- DE-A- 3 812 595
- US-A- 5 837 269
- JYH-YUAN YANG ET AL: "INHIBITION OF HIV-1 LATENCY REACTIVATION BY DEHYDROEPIANDROSTERONE (DHEA) AND AN ANALOG OF DHEA" AIDS RESEARCH AND HUMAN RETROVIRUSES,US,NEW YORK, NY, vol. 9, no. 8, 1 August 1993 (1993-08-01), pages 747-754, XP000612730 ISSN: 0889-2229
- ZHANG, Z. ET AL: "Prevention of immune dysfunction and vitamin E loss by dehydroepiandrosterone and melatonin supplementation during murine retrovirus infection" IMMUNOLOGY, 96(2), 291-297 , February 1999 (1999-02), XP002141192
- BEBO, BRUCE F., JR. ET AL: "Androgens alter the cytokine profile and reduce encephalitogenicity of myelin-reactive T cells" J. IMMUNOL. (1999), 162(1), 35-40 , XP002141193
- HERNANDEZ-PANDO, R. ET AL: "The effects or androstenediol and dehydroepiandrosterone on the course and cytokine profile of tuberculosis in BALB/c mice" IMMUNOLOGY (1998), 95(2), 234-241 , XP002141194
- INSERRA, PAULA ET AL: "Modulation of cytokine production by dehydroepiandrosterone (DHEA) plus melatonin (MLT) supplementation of old mice" PROC. SOC. EXP. BIOL. MED. (1998), 218(1), 76-82 , XP002141195
- ARAGHI-NIKNAM, MOHSEN ET AL: "Cytokine dysregulation and increased oxidation is prevented by dehydroepiandrosterone in mice infected with murine leukemia retrovirus" PROC. SOC. EXP. BIOL. MED. (1997), 216(3), 386-391 , XP002141196
- ARAGHI-NIKNAM, M. ET AL: "Modulation of immune dysfunction during murine leukemia retrovirus infection of old mice by dehydroepiandrosterone sulfate (DHEAS)" IMMUNOLOGY (1997), 90(3), 344-349 , XP002141197
- CHEMICAL ABSTRACTS, vol. 126, no. 9, 3 March 1997 (1997-03-03) Columbus, Ohio, US; abstract no. 113406, KANG, IN-CHOL ET AL: "Dehydroepiandrosterone and.beta.-endorphin enhance IL-12 gene expression" XP002141202 & TAEHAN MISAENGMUL HAKHOECHI (1996), 31(4), 399-404 ,
- H. P. SIGG ET AL: "3.alpha.-Acetoxyätien-(8:9 oder 8:14)-säure-methylester" HELVETICA CHIMICA ACTA, vol. 39, no. 6, 1956, pages 1507-1525, XP002141198 BASEL CH
- E. HENDERSON ET AL: "Dehydroepiandrosterone and 16.alpha.-bromoepiandrosterone: inhibitors of Epstein-Barr virus induced transformation of human lymphocytes" CARCINOGENESIS, vol. 2, no. 7, 1981, pages 683-686, XP002141199
- MANZ, B. ET AL: "Methyl 17.beta.-carboxyester derivatives of natural and synthetic glucocorticoids: Correlation between receptor binding and inhibition of in vitro phytohaemagglutinin-induced lymphocyte blastogenesis" J. CLIN. CHEM. CLIN. BIOCHEM. (1983), 21(2), 69-75 , XP002141200
- XIA, P. ET AL: "Anti-AIDS agents. Part 36:1 17-carboxylated steroids as potential anti-HIV agents" BIOORG. MED. CHEM. 7(9), 1907-1911 , September 1999 (1999-09), XP002141201
- DeSmidt et al. J. Pharm. Sci. 75:497-501, 1986
- Wadke et al. J. Pharm. Sci. 61:868-871, 1972
- Shefter et al. J. Pharm. Sci. 52:781-791, 1963

## Description

### BACKGROUND OF THE INVENTION

The invention relates to 16α-bromo-3β-hydroxy-5α-androstane-17-one hemihydrate (16α-bromoepiandrosterone or hereafter "BrEA") in accordance with claim 1;a composition comprising BrEA hemihydrate in accordance with claim 5; a method of making BrEA hemihydrate in accordance with claim 9; a use of BrEA hemihydrate according to claim 14; and BrEA hemihydrate for use as a medicament according to claim 56. The steroids are useful for a number of therapeutic and non-therapeutic applications, including their use as immune modulators. The present invention also relates to methods to make the compounds, compositions and formulations.

BrEA and its preparation from the steroid compound 3β-hydroxyandrost-5-en-17-one (dehydroepiandrosterone or "DHEA") have been described (see, e.g., *J. Org. Chem*. 1962 27:2937-2938). Methods to prepare DHEA and other steroids and their biological properties have been described, see, e.g., U.S. patent numbers 2833793, 2911418, 3148198, 3471480, 3976691, 4268441, 4427649, 4542129, 4666898, 4956355, 5001119, 5043165, 5077284, 5028631, 5110810, 5157031, 5162198, 5175154, 5277907, 5292730, 5296481, 5372996, 5387583, 5407684, 5424463, 5461042, 5478566, 5506223, 5518725, 5527788, 5527789, 5532230, 5559107, 5562910, 5583126, 5585371, 5587369, 5591736, 5593981, 5610150, 5635496, 5641766, 5641768, 5656621, 5660835, 5686438, 5696106, 5700793, 5707983, 5709878, 5710143, 5714481, 5728688, 5736537, 5744462, 5753237, 5756482, 5776921, 5776923, 5780460, 5795880, 5798347, 5798348, 5804576, 5807848, 5807849, 5811418, 5824313, 5824668, 5824671, 5827841, 5837269, 5837700, 5843932, 5846963, 5859000, 5872114 and 5872147; German patent numbers 2035738 and 2705917; PCT publication numbers WO 95/21617, WO 97/48367, WO 98/05338, WO 98/50040, WO 98/50041, WO 98/58650; European publication number 0020029; Ben-David, et al., *Proc. Soc. Expt. Biol. Med.* 1967 125:1136-1140, Coleman et al., *Diabetes* 1982 31:830, Oertel, et al., *J. Steroid Biochem*. 1972 3:493-496, Pashko, et al., *Carcinogenesis* 1981 2:717-721, Schwartz et al., *Nutr. Cancer* 1981 3:46-53; Dyner et al., *J. Acquired Immune Defrciency Syndromes* 1993 6:459-465; A.A. Afanasii and Y.A. Titov, *Total Steroid Synthesis,* Plenum Press, New York, 1970, see, e.g., p 1-304.

The use DHEA and other steroids in various applications, e.g., modulating immune responses have been described, e.g., U.S. patent numbers 5869090, 5863910, 5856340, 5824668, 5804576, 5753237, 5714481, 5709878, 5407684, 5206008, 5077284, 4978532, 4898694, 4542129, 3711606 and 3710795. U.S. patent 4956355 and PCT publication number WO 97/48367, have described the use of BrEA and certain steroid compounds to treat certain virus or bacterial infections, such as human immunodeficiency virus ("HIV") infection.

Various biological effects and/or metabolic conversions of steroid compounds have been described, e.g., Batta et al., *J. Biol. Chem.* 1986 25:127-133, Belli at al., *Liver* 1991 11:162-169, Bhattacharjee et al., *Anal. Biochem.* 1992 201:233-236, Blake et al., *Int. J. Peptide Protein Res.* 1982 20:97-101, 1986 25:127-133, Bonaventura, *Am. J. Obstet. Gynecol*. 1978 131:403-409, Bucala et al., *J. Steroid Biochem.* 1986 25:127-133, Carey et al., *Biochem.* 1981 20:3637-3648, Chen et al., *Carcinogenesis* 1999 20:249-254, Chen et al., *Carcinogenesis* 1998 19:2187-2193, Chow et al., *Antisense Res. Dev.* 1994 4:81-86, Citro et al., *Dis. Colon Rectum* 1994 37(2 Suppl):S127-S132, Cleary, *Proc. Soc. Exp. Biol. Med.* 1991 196:8-16, Cleary, *Int. J. Biochem.* 1990 22:205-210, Crawford et al., *Lab. Invest.* 1994 71:42-51, Danenberg et al., *Antimicrob. Agents Chemother.* 1992 36:2275-2279, Dotzlaw et al., *Cancer Res.* 1999 59:529-532, Falany et al., *J. Steroid Biochem. Mol. Biol.* 1994 48:369-375, Faredin et al., *J. Investigative Dermatol.* 1969 52:357-361, Galigniana et al., *Mol. Pharmacol*. 1999 55:317-323, Goto et al., *J. Chromatogr.* 1983 276:289-300, Grenot *Biochem.* 1992 31:7609-7621, Hofbauer et al., *Life Sci.* 1999 64:671-679, Huijghebaert et al., *J. Lipid Res.* 1986 27:742-752, Hurd et al., *Oncogene* 1999 18:1067-1072, lida et al., *J. Lipid Res.* 1995 36:628-638, Jellinck et al., *Steroids* 1967 10:329-346, Jonsson et al., *J. Pediatr. Gastroenterol. Nutr.* 1995 20:394-402, Kalimi et al, *Mol. Cell. Biochem.* 1994 131:99-108, Kramer et al., *J. Biol. Chem.* 1994 269:10621-10627, LaRochelle et al., *Steroids* 1984 43: 209-217, Liao et al., *Carcinogenesis* 1998 19:2173-2180, Lillienau et al., *J. Clin. Invest.* 1992 89:420-431, Loria, *Psychoneuroendocrinology* 1997 22:S103-S108, Luscher et al *Mol. Immunol.* 1983 20:1099-1105, Manna et al., *J. Biol. Chem.* 1999 274:5909-5918, Marschall et al., *J. Biol. Chem.* 1989 264:12989-12993, Medh et al., *Cancer Res.* 1998 15:3684-3693, Mohan et al., *Steroids* 1992 57:244-247, Munoz de Toro et al., *J. Steroid Biochem. Mol. Biol*. 1998 67:333-339, Padgett et al., *J. Neuroimmunol.* 1998 84:61, Padgett et al., *Ann. N.Y. Acad. Sci*. 1995 774:323, Padgett et al., *J. Immunol.* 1994 153:1544-1552, Pashko et al., *Carcinogenesis* 1984 5:463-466, Pashko et al., *Carcinogenesis* 1981 2:717, Petrylak et al., *J. Clin. Oncology* 1999 17:958-967, Podesta et al., *Steroids* 1996 61:622-626, Regelson et al., *Ann. N. Y. Acad. Sci.* 1994 719:564, Schmassmann et al., *Gastroenterology* 1993 104:1171-1181, Schmassmann et al., *Hepatology* 1990 11:989-996, Schreiber et al., *Lancet* 353:459-461, Schreiber, *Neth. J. Med.* 1998 53:S24-31, Schwartz et al., *Cancer Res.* 1988 48:4817, Shahidi et al., *Biochem. Biophys. Res. Commun.* 1999 254:559-565, Steer et al., *Ann. Rheum. Dis.* 1998 57:732-737, Suzuki et al., *Steroids* 1998 63:672-677, Suzuki et al., *Steroids* 1996 61:296-301, Swaan et al., *Bioconjugate Chem.* 1997 8:520-525, Tang et al, *Anticancer Drug Res.* 199813:815-824, Thomas et al., *J. Steroid Biochem.* 1986 25:103-108, Utsumi et al., *Cancer Res.* 1999 59:377-381, Vanden Heuvel, *J. Nutr.* 1999 129(2S Suppl.):575S-580S, Wang et al., *Endocrinology* 1998 139:3903-3912, Wong et al., *J. Biol. Chem.* 1999 274:5443-5453, Xie et al., *Endocrinology* 1999 140:219-227, Yen et al., *Lipids* 197712:409-413, Zackheim et al., *Arch. Dermatology* 1998 134:949-954, Zhang et al., *Biochim. Biophys. Acta* 1991 1096:179-186, Zhu et al., *Carcinogenesis* 1988 19:2101-2106.

Compositions containing BrEA that were used to deliver the compound to cells or cell extracts usually included a significant amount of water. Such compositions contained solvents such as dioxane or dimethylsulfoxide ("DMSO"), which contained water, or an aqueous cyclodextrin solutions to facilitate compound delivery to cells, see, e.g., *J. Pharmacol Exp. Ther.* 1998, 285:876-83, *Cancer Res.* 1986 46:3389-95, *Caicinogenesis* 1985 6:333-35, *Carcinogenesis* 1981 2:717-721, *Carcinogenesis* 1981 2:683-86. Such compositions are typically delivered to animals by injection or to cells in tissue culture by addition to cell culture medium. European publication number EP 429 187 describes formulations that contain DHEA or BrEA and polyvinylpyrrolidone and crosslinked polyvinylpyrrolidone. Some of these compositions may have undesired or suboptimal properties. For example, solvents such as dioxane, DMSO or chloroform are generally not preferred or suitable parenteral excipients, particularly for human use. Formulations that contain BrEA or related steroids and that have improved properties, e.g., lower toxicity, improved chemical stability or desirable characteristics for large-scale synthesis are needed.

Mammalian immune responses to infections or other conditions are often characterized by responses mediated by different effector cell populations. In some situations, helper T cells designated Th1 in the murine system, facilitate immune effector functions that are typically dominated by cell-mediated responses. In other cases, helper T cells designated Th2 cells facilitate immune effector functions that are typically dominated by humoral responses. A vigorous Th1 response is usually needed to clear infections or to slow the progression of an infection. When a subjects immune response is biased to, or dominated by, a Th2-type response, the cytokines associated with the Th2 response tend to suppress the immune system's capacity to mount a vigorous Th1 response at the same time. The converse is also generally true. When mammalian immune responses begin to result in an increasing Th2 response, the Th1 response to the same condition tends to weaken. Weak Th1 responses may be associated with progression of some infections or other conditions, see, e.g., M. Clerici and G.M. Shearer, *Immunol*. *Today* 14:107-111, 1993; M. Clerici and G.M. Shearer, *Immunol. Today* 15:575-581, 1994. The invention provides compounds and compositions useful to enhance Th1 immune responses.

Additionally, WO 98/47516 A1 discloses a method of enhancing the Th 1 immune protective response when using 17-ketosteroidal compounds as anti-viral, anti-bacterial, anti-mycoplasma, or anti-intracellular parasitic agent, wherein 16 α-bromoepiandrosterone is a preferred compound.

### OBJECTS OF THE INVENTION

The invention compositions, formulations, uses or methods accomplish one or more of the following objects.

One object of the invention is to provide new steroid compounds or analogs that are suitable for therapeutic and other applications, such as immune modulators. Invention objects further include providing BrEA hemihydrate (BrEA₂ ^{·}H₂O), compositions that comprise BrEA hemihydrate and methods to make and use it. Another object of the invention is to provide liquid compositions and formulations that comprise a formula 1 compound(s), and that comprise about 3% (v/v) or less of water. Another object is to provide compositions one can use as intermediates to prepare human pharmaceutical and veterinary formulations containing a formula 1 compound(s). Another object is to provide intermittent dosing methods to deliver a formula 1 compound to a subject to enhance Th1 immune responses. Further objects are to provide methods to modulate innate immunity or to enhance Th1 immune responses in a subject by administering to the subject a formula 1 compound(s) such as BrEA. Other objects are to provide methods to inhibit pathogen, e.g., viral, replication in a subject by administering to the subject a formula 1 compound(s) such as BrEA. Invention objects include providing formula 1 compounds or formulations useful to ameliorate one or more symptoms of a pathological condition associated with immune suppression or with deficient Th1 immune responses. Other objects are to provide methods to make and use compositions and formulations comprising a formula 1 compound(s).

### BRIEF DESCRIPTION OF THE FIGURES

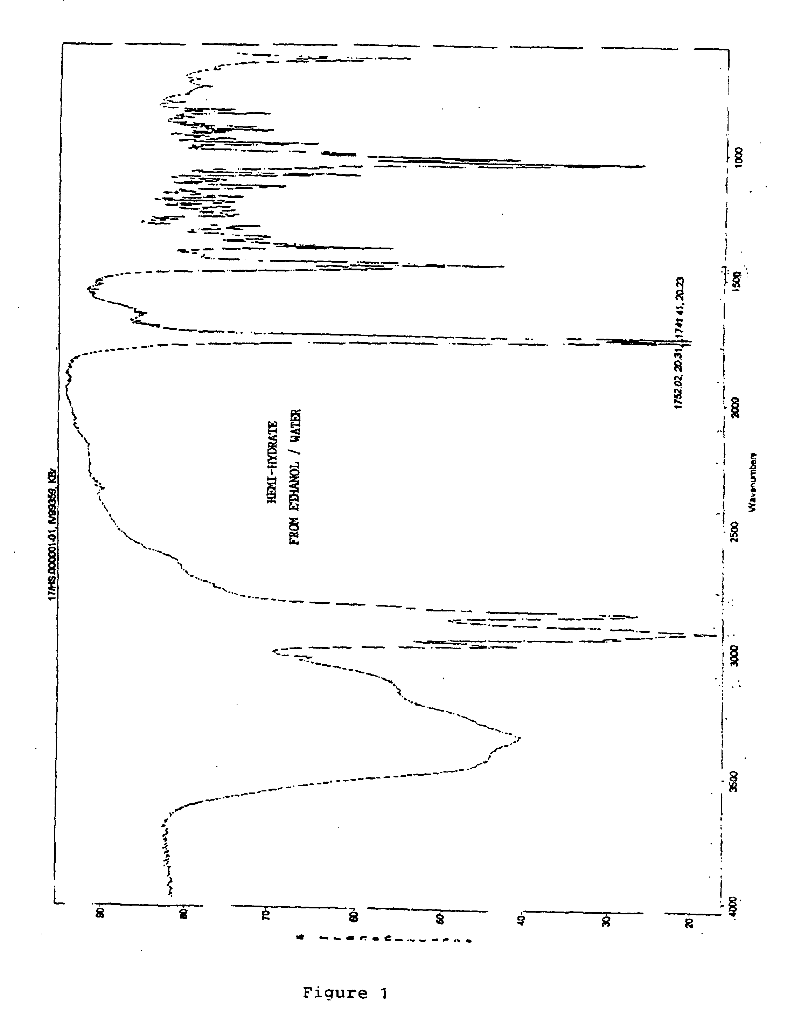

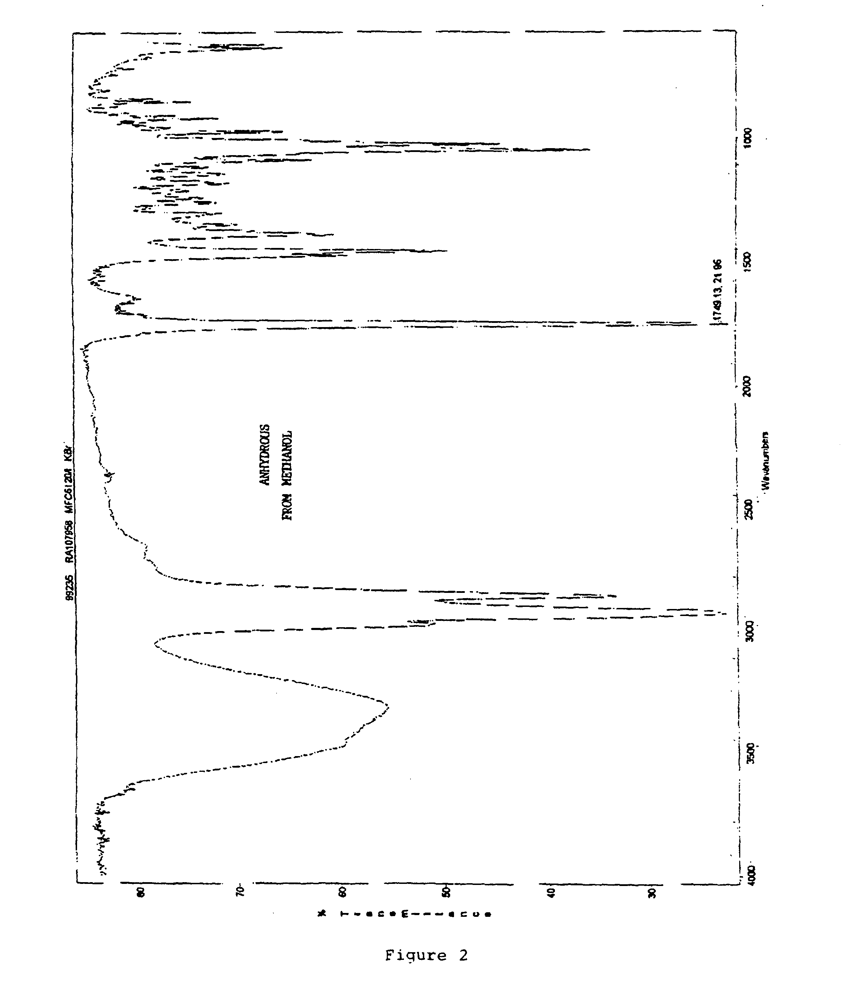

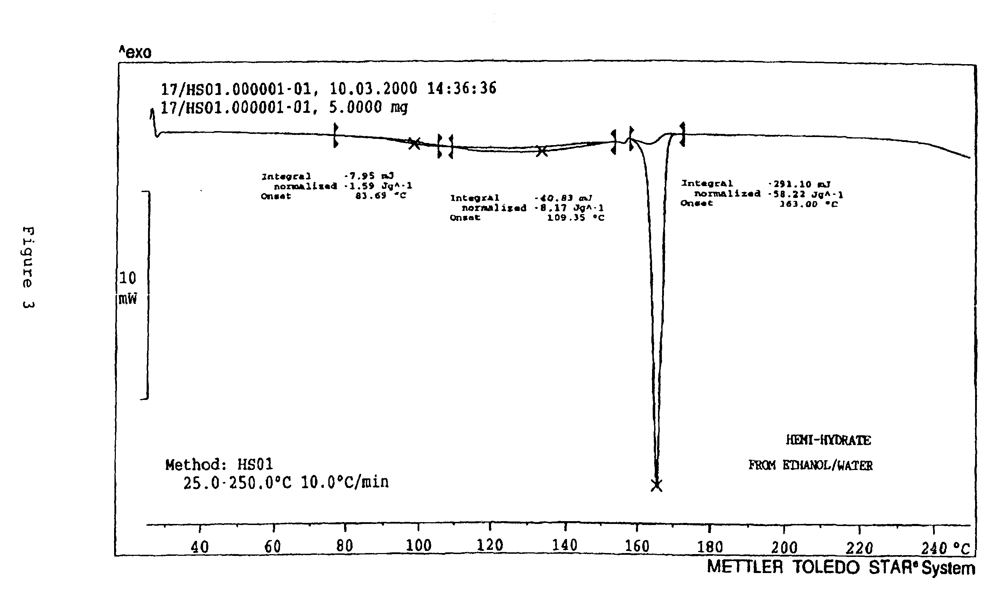

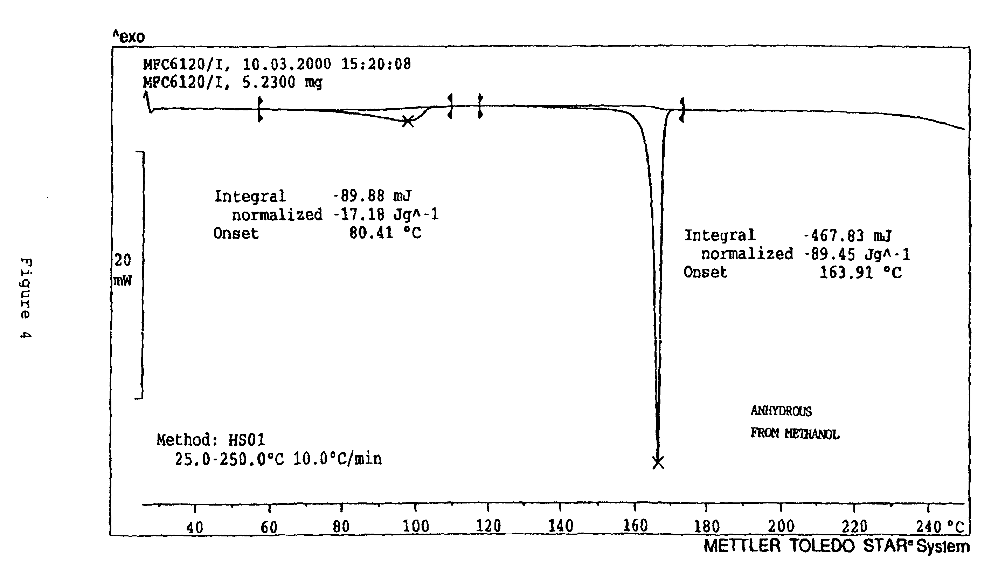

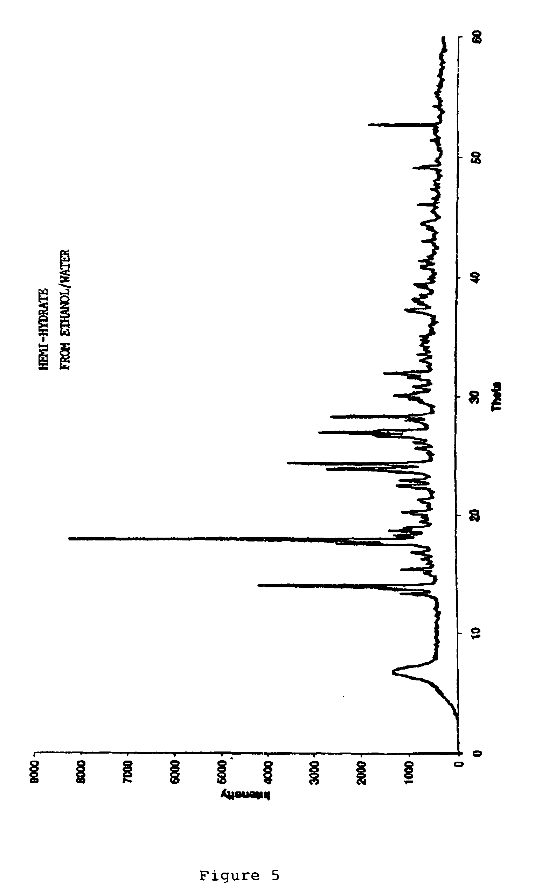

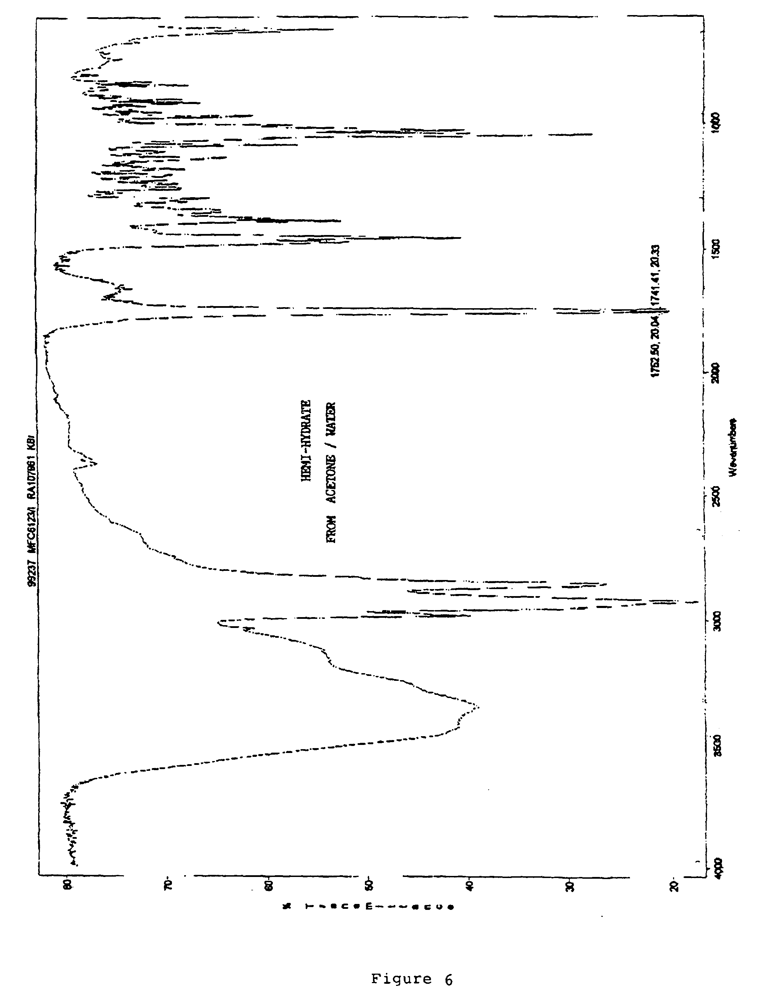

Figure 1 is an FTIR (Fourier transform infrared) spectrum obtained by USP method <197> of BrEA hemihydrate that was prepared by precipitation of BrEA from ethanol and water. Figure 2 is a FTIR spectrum obtained by USP method <197> of anhydrous BrEA that was prepared by precipitation of BrEA from anhydrous methanol. Figure 3 shows a DSC endotherm of BrEA hemihydrate that was prepared by precipitation of BrEA from ethanol and water. Figure 4 shows a DSC endotherm of anhydrous BrEA that was prepared by precipitation of BrEA from anhydrous methanol. Figure 5 is an XRD (powder X-ray diffraction) spectrum of BrEA hemihydrate that was prepared by precipitation of BrEA from ethanol and water. Figure 6 is a FTIR spectrum obtained by USP method <197> of BrEA hemihydrate that was prepared by precipitation of BrEA from acetone and water.

### SUMMARY OF THE INVENTION

In accordance with the objects, the invention provides BrEA hemihydrate 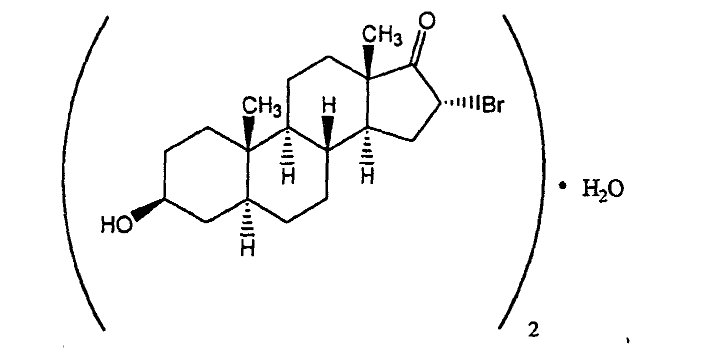 which is optionally characterized by reference to one or more physical properties such as its melting point, infrared absorption spectrum or its powder X-ray diffraction spectrum.

Related embodiments include BrEA hemihydrate and one or more excipients suitable for human pharmaceutical use or for veterinary use. Another related embodiment is a method to make BrEA hemihydrate comprising precipitating BrEA from a solution comprising ethanol and water.

BrEA is included in a compound according to formula 1 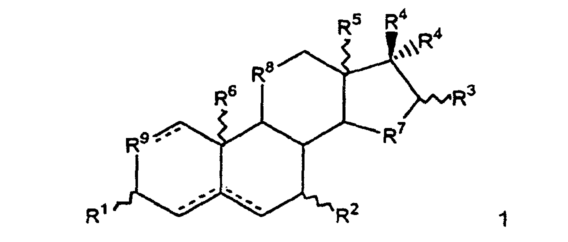 and one or more nonaqueous liquid excipients, wherein the composition comprises less than about 3% v/v water and wherein,
R¹, R², R³, R⁴, R⁵, R⁶ and R¹⁰ independently are -H, -OR^{PR}, -SR^{PR}, -N(R^{PR})₂, -O-Si-(R¹³)₃, -CN, -NO₂, an ester, a thioester, a phosphoester, a phosphothioester, a phosphonoester, a phosphiniester, a sulfite ester, a sulfate ester, an amide, an amino acid, a peptide, an ether, a thioether, an acyl group, a thioacyl group, a carbonate, a carbamate, a thioacetal, a halogen, an optionally substituted alkyl group, an optionally substituted alkenyl group, an optionally substituted alkynyl group, an optionally substituted aryl moiety, an optionally substituted heteroaryl moiety, an optionally substituted monosaccharide, an optionally substituted oligosaccharide, a nucleoside, a nucleotide, an oligonucleotide, a polymer, or,
one more of R¹, R², R³, R⁴, R⁵, R⁶, R¹⁰, R¹⁵, R¹⁷ and R¹⁸ are =O or =S and the hydrogen atom that is bonded to the same carbon atom is absent.

Embodiments include liquid formulations that comprise BrEA, one or more excipients and less than about 3% water, wherein the formulation is optionally disposed in containers that exclude water.

Another embodiment is a method comprising intermittent administration of BrEA to a subject having a pathological condition, such as a viral or parasite infection.

A further embodiment is a method to modulate a subject's innate immunity, Th1 immune responses or Th2 immune responses comprising administering BrEA to a subject

Other embodiments are as described in the specification including the appended numbered embodiments and the claims.

### DETAILED DESCRIPTION OF THE INVENTION

**Definitions.** As used herein and unless otherwise stated or implied by context, the following terms have the meanings defined here.

An "invention formulation" or "formulation" means an invention composition that one can administer parenterally to a human or animal without further manipulations that change the ingredients or the ingredient proportions that are present. Formulations are suitable for human or veterinary applications.

An "invention composition" is a composition, that is an intermediate one can use to make the invention formulations, i.e., a change(s) in an ingredient(s) or its amount(s) is needed to make a formulation. Thus, invention compositions include compositions where further processing is required before it is a formulation, e.g., mixing or addition of a desired amount of an ingredient

An "excipient" means a component or an ingredient that is acceptable in the sense of being compatible with the other ingredients of invention compositions or formulations and not overly deleterious to the patient or animal to which the formulation is to be administered. As used here, "excipients" include liquids, such as benzyl benzoate, cottonseed oil, N,N-dimethylacetamide, a C₂₋₁₂ alcohol (e.g., ethanol), glycerol, peanut oil, a polyethylene glycol ("PEG"), vitamin E, poppyseed oil, propylene glycol, safflower oil, sesame oil, soybean oil and vegetable oil. Excipients, as used herein will optionally exclude chloroform, dioxane, vegetable oil, DMSO or any combination of these. Excipients comprise one or more components typically used in the pharmaceutical formulation arts, e.g., fillers, binders, disintegrants and lubricants.

A "subject" means a human or animal. Usually the animal is a vertebrate such as a primate, rodent, domestic animal or game animal. Primates include chimpanzees, cynomologous monkeys, spider monkeys, and macaques, e.g., *Rhesus.* Rodents include mice, rats, woodchucks, ferrets, rabbits and hamsters. Domestic and game animals include cows, horses, pigs, deer, bison, buffalo, felines, e.g., domestic cat, canines, e.g., dog, avians, e.g., chicken, emu, ostrich, and fish, e.g., trout, catfish and salmon. Subject includes any subset of the foregoing, e.g., all of the above, but excluding one or more groups or species such as humans, primates or rodents.

Unless otherwise stated or implied by context, expressions of a percentage of a liquid ingredient, e.g., an excipient, in an invention composition or formulation mean the ingredient's percent by volume (v/v). Thus, 20% propylene glycol means 20% v/v propylene glycol is present in an invention composition or formulation. The amount of excipient indicated in invention compositions is not affected by the form used, e.g., NF or USP grade solvent or excipient. Thus, an invention composition that comprises about 30% polyethylene glycol 300 NF can instead comprise a USP counterpart, provided that other limitations, such as the amount of water present, are not exceeded.

As used herein, "innate immunity" refers to one or more components typically associated with nonspecific immune defense mechanisms in a subject. These components include the alternate complement pathway, e.g., Factor B, Factor D and properdin; NK cells, phagocytes (monocytes, macrophages), neutrophils, eosinophils, dendritic cells, fibrocytes; anti-microbial chemicals, e.g., defensins; physical barriers - skin, mucosal epithelium; and certain interleukins, chemokines and cytokines. Innate immunity plays a role in resistance to intracellular parasite infections, e.g., white blood cell infection, a liver infection, and other infections, e.g., lymph node infections. Enhancement of innate immunity mechanism by formula 1 compounds or method described herein may enhance phagolysosome fusion or movement, which some pathogens, e.g., intracellular bacteria such as mycobacteria, or *Listeria* inhibit.

As used herein, references to CD molecules, specific immune cell subsets, immune responses and the like, generally use nomenclature that applies to molecules, cells or the like that are found in humans. Analogs or counterparts of such molecules, cells or the like in other species may have a differing nomenclature, but are included in this invention. A description of the nomenclature and function of various CD molecules and immune cell subsets are as found in the scientific literature. References to Th0, Th1 or Th2 cells and references to Th1 or Th2 immune responses in the context of human patients refers to the human counterparts of the murine Th0, Th1 or Th2 immune cells or responses. For reviews see, e.g., A.K. Abbas et al., editors, *Cellular and Molecular Immunology*, W.B. Saunders Company, third edition, 1997, ISBN 0-7216-4024-9, pages 4-469, and I. Kimber and M. K. Selgrade, editors, *T Lymphocyte Subpopulations in Immunotoxicology*, John Wiley & Sons Ltd., 1998, ISBN 0-471-97194-4, pages 1-53.

"Immunosuppressive molecule" means molecules such as cyclosporin, cyclohexamide, mitomycin C, adriamycin, taxol and amphotericin B. These molecules tend to have toxicities toward the immune system and are directly or indirectly immunosuppressive, i.e., toxic to dividing cells or they can downregulate immunity.

"Steroid receptor" means a gene product, typically a protein monomer or dimer that can bind to a ligand, e.g., a natural steroid or an analog thereof, such as formula 1 compounds. Steroid receptors include orphan steroid receptors. Orphan steroid receptors are proteins for which the natural ligand or biological function is at least partially unknown. As used here, steroid receptors include homodimers, e.g., SXR and (CARβ)₂, and heterodimers, e.g., PXR-CARβ or RXR-CARβ. Steroid receptors also include isoforms, e.g., PXR.1 and PXR.2 for the PXR receptor, and homologs of the steroid receptors, e.g., the homolog of CARβ known as MB67. Isoforms are typically generated by different splicing pathways for a nuclear RNA from one gene, while homologs are typically a distinct copy of a steroid receptor gene, where the gene copy encodes only relatively small differences compared to the reference steroid receptor gene product. Such differences are most often found in areas other than the dimerization region and the steroid binding region of the steroid receptor's structure. Typically isoforms and homologs bind the same or similar ligands as the reference gene product or steroid receptor. Steroid receptors may be of human or animal origin, e.g., obtained from cells, tissues or cDNA expression libraries derived from cells or tissues of any primate, rodent (including murine), avian, ovine, bovine, equine, canine or feline species or any of the species or any species within any group (e.g., Family or Genus) of species mentioned elsewhere herein or in any reference cited herein.

In the context of a combination of molecules that includes a steroid receptor and a formula 1 compound, "invention complexes" or "complexes" means a complex that comprises a steroid receptor and a formula 1 compound and optionally other molecules. These other molecules include (i) a DNA recognition sequence ("DNARS" hereafter), i.e., a sequence that the steroid receptor specifically recognizes and binds to and (ii) a transcription factor that can bind to the steroid receptor-formula 1 compound complex. As used herein, these complexes can arise in cells *in vitro* or *in vivo*, or in cell-free systems. Complexes include, for example, steroid receptor heterodimer-formula 1 compound combinations, steroid receptor homodimer-formula 1 compound combinations, steroid receptor monomer-formula 1 compound combinations, steroid receptor heterodimer-formula 1 compound-DNA (or DNARS) combinations, steroid receptor homodimer-formula 1 compound-DNA (or DNARS) combinations, steroid receptor heterodimer-formula 1 compound-transcription factor combinations, steroid receptor homodimer-formula 1 compound-transcription factor combinations, steroid receptor heterodimer-formula 1 compound-DNA (or DNARS)-transcription factor combinations and steroid receptor homodimer-formula 1 compound-DNA (or DNARS)-transcription factor combinations.

An "agonist" or an "antagonist" is a compound or composition that respectively, either increases or decreases the activity of a receptor, which can lead to increased or decreased transcription of a regulated gene. Receptors (and transcription factors) can modulate transcription of their target gene(s) by enhancing transcription or decreasing it.

**General methods.** Methods have been described, for example Karl Fischer (KF) and loss on drying (LOD), to determine the content of water or solvents in various compositions. LOD measures all volatiles in a sample, while KF is typically used to measure all water. When water is the only volatile present, LOD values are equal to or less than KF values for a given composition. KF measures water in hydrates of a compound and LOD determines both water and the amount of other volatiles that may be present. Invention compositions and formulations are conveniently assayed for water content by KF titration (e.g., using a Metrohm 684 KF Coulometer or equivalent) according to a published procedure (*U.S. Pharmacopoeia,* vol. 23, 1995, chapter <921>, U.S. Pharmacopeial Convention, Inc., Rockville, MD) and manufacturer's Coulometer instructions. The amount of material used in the assay, about 50-100 mg, is measured using a five place analytical balance (Sartorius, Model RC210D, or a suitable equivalent). The amounts of water specified in invention compositions and formulations is the amount obtained by KF analysis.

Powder X-ray diffraction (XRD) methods have been used to characterize various crystalline compounds (see, e.g., *U.S. Pharmacopoeia*, volume 23, 1995, <941>, p.1843-1845, U.S. Pharmacopeial Convention, Inc., Rockville, MD; Stout et al., *X-Ray Structure Determination; A Practical Guide,* MacMillan Co., New York, N.Y., 1986). The diffraction pattern, or portions thereof, obtained from a crystalline compound is usually diagnostic for a given crystal form, although weak or very weak diffraction peaks may not always appear in replicate diffraction patterns obtained from successive batches of crystals. Also, the relative intensities of XRD bands, particularly at low angle X-ray incidence values (low Theta), may vary due to preferred orientation effects arising from differences in, e.g., crystal habit, particle size or other measurement conditions. Peaks on XRD spectra are typically defined at a given Theta value +/- about 0.1 to 0.2. XRD information from the 1, 2, 3, 4, 5 or more main intensity XRD peaks, optionally combined with one or more other diagnostic data (melting point, DSC, IR), is usually suitable to characterize or describe a crystalline material such as BrEA hemihydrate from other crystal forms that contain the same compound.

Other techniques that are used to identify or describe a crystalline material such as BrEA hemihydrate include melting point (MP), differential scanning calorimetry (DSC) and infrared absorption spectroscopy (IR) data. DSC measures thermal transition temperatures at which a crystal absorbs or releases heat when its crystal structure changes or it melts. MP data and DSC thermal transition temperatures are typically reproducible within about 1 or 2°C on successive analyses. IR measures absorption of infrared light that is associated with the presence of particular chemical bonds that are associated with groups, e.g., hydroxyl, that vibrate in response to particular light wavelengths.

**Invention Embodiments.** The invention provides BrEA hemihydrate, which is typically substantially free of other forms of BrEA, such as amorphous BrEA or anhydrous BrEA. As used herein, BrEA hemihydrate or crystalline BrEA hemihydrate refers to solid BrEA and water having an ordered arrangement of substantially all of the constituent molecules in a defined three-dimensional spatial pattern or lattice. Crystalline BrEA hemihydrate may comprise one or more crystal habits, e.g., tablets, rods, plates or needles. BrEA hemihydrate that is substantially free of other forms of BrEA means a dry or substantially dry (where a liquid(s) comprises less than about 10% w/w of the total weight) solid preparation where more than about 55% w/w of the BrEA in the preparation is present as BrEA hemihydrate. Such compositions typically comprise at least about 60% w/w, or at least about 70% w/w, or at least about 80% w/w, usually at least about 90% w/w or at least about 95% w/w, or at least about 98% w/w of BrEA hemihydrate, with the remaining BrEA being present as other forms of BrEA such as the amorphous or anhydrous BrEA. Solid BrEA hemihydrate will typically comprise at least about 90% w/w, usually at least about 97% or about 98% w/w of the compound and less than about 10% w/w, usually less than about 3% or 2% w/w of by-products, which may include the 16β isomer of BrEA or one or more by-products of BrEA synthesis. Often the amount of solid BrEA that is present in a solid or a liquid medium will not contain detectable amounts of other forms of BrEA (using standard analytical methods such as, e.g., FTIR, DSC or XRD) and the hemihydrate will may thus comprise about 99-100% w/w of the total amount of BrEA that is present.

Other invention embodiments include compositions that comprise a substantial amount of BrEA hemihydrate that is present in compositions that comprise one or more other forms of BrEA, e.g., amorphous BrEA or anhydrous BrEA, and optionally one or more additional components, such as any excipient described herein. As used herein, the "substantial amount" of BrEA hemihydrate in these compositions comprises at least about 15-20% w/w or at least about 20% w/w of BrEA hemihydrate of the total amount of BrEA that is present, typically at least about 25% w/w, more typically at least about 30% w/w, often at least about 35% w/w and usually at least about 45% w/w. These compositions are generally solids, e.g., formulations or unit dosages, but they also include suspensions, precipitates, gels and colloids that contain solid BrEA. Such suspensions or precipitates may arise from, e.g., precipitation of BrEA hemihydrate from a solution that contains water or from addition of solid BrEA to a liquid excipient(s). Obviously, compositions that comprise a substantial amount of BrEA may be substantially free of other forms of solid BrEA as discussed above.

BrEA hemihydrate may conveniently be identified by reference to BrEA hemihydrate characterized by one or more of (1) its melting or decomposition point or range (optionally expressed as +/- 2°C), (2) one or more BrEA hemihydrate DSC transition temperatures or ranges (any of which may be optionally expressed as +/- 2°C), (3) one or more characteristic BrEA hemihydrate IR absorption bands, (4) 1, 2, 3, 4, 5, 6 or more of the highest intensity XRD peaks (any one or more of which are optionally expressed as +/-0.1° Theta or +/- 0.2° Theta) obtained from an XRD spectrum of BrEA hemihydrate using Cu-Kα radiation (e.g., obtained essentially according to the method described at *U.S. Pharmacopoeia,* volume 23, 1995, <941>, p.1843-1845), (5) the presence of less than about 3% or less than about 2% w/w of other compounds, (6) a water content of dry BrEA hemihydrate of about 2.5% w/w (e.g., 2.3-2.7% w/w), where dry BrEA hemihydrate means compound dried by filtration, optionally washed once with an anhydrous solvent such as hexane, filtered again and dried *in vacuo* at about 60°C until no further weight loss occurs over 24 hours at about 60°C (e.g., where water content is determined essentially by the Karl Fisher or other method described at *U.S. Pharmacopoeia*, vol. 23, 1995, p 1801-1802 or 1840-1843 methods <731> or <921>), (7) cell constants and the orientation matrix obtained from single crystal X-ray crystallography of BrEA hemihydrate (obtained, e.g., essentially as described in WO 99/04774 at example 13), (8) a description of crystal shapes as observed at about 100X magnification to about 150X magnification by polarized light microscopy or (9) average BrEA hemihydrate crystal size and shape descriptions.

Thus, for example, BrEA hemihydrate may be characterized by or one or more of its IR absorption bands, e.g., the carbonyl peaks at 1741 cm⁻¹ and 1752 cm⁻¹, and either its melting or decomposition point or range and/or 1, 2, 3, 4, 5, 6 or more of the XRD peaks (usually the highest intensity peaks) at Theta (X-ray diffraction angle) values of 17.8, 23.8, 24.2, 26.9-27.2, 28.6, 30.1 and 32.2.

BrEA hemihydrate is suitable to prepare compositions comprising an excipient(s) suitable for human pharmaceutical use or for veterinary use. Such compositions are used to prepare formulations and unit dosages. Unit dosages typically comprise tablets, capsules, lozenges or sterile solutions, including sterile solutions for parenteral administration. Solid unit dosage forms typically comprise about 5-1000 mg of BrEA hemihydrate, typically about 20-400 mg, e.g., about 25 mg, about 50 mg, about 100 mg, about 150 mg or about 250 mg per unit dose.

The invention provides a method to make BrEA hemihydrate comprising contacting water, 16α-bromo-3β-hydroxy-5α-androstan-17-one and a C1-C6 alcohol (e.g., methanol, ethanol, propanol, isopropanol, butanol) and water. Typically the only one C1-C6 alcohol is present, e.g., ethanol, which is anhydrous or which may comprise up to about 2% w/w water. In some embodiments, the method utilizes a solution that comprises about 5-25% w/w water, about 30-45% w/w ethanol and about 30-45% w/w of a BrEA preparation. Typical BrEA preparations are solid preparations that comprise at least about 80% w/w, usually at least about 90% w/w or at least about 95% w/w of BrEA. The solutions may comprise about 18-22% w/w water, about 37-43% w/w ethanol and about 37-43% w/w of a BrEA preparation. In conducting the precipitation or crystallization method, the solution will typically be at a temperature of about -20°C to about 45°C, usually at about 0°C to about 20°C. The solution is maintained at this temperature range for about 30 minutes to about 12 hours and the solution is optionally agitated using slow to moderate agitation during crystallization.

A related embodiment comprises a method to prepare BrEA hemihydrate comprising precipitating BrEA from a solution comprising at least about 15-25% w/w water, about 35-45% w/w of a BrEA preparation and at least about 35-45% w/w of one or more water-miscible solvents, typically C₁₋₆ alcohols (methanol, ethanol, propanol, isopropanol, butanol). The BrEA preparation may optionally comprise one or more by-products of BrEA synthesis. Typical BrEA hemihydrate preparations or batches comprise less than about 5% w/w, usually less than about 3% or about 2% w/w of other compounds, such as by-products of BrEA synthesis. Aspects of this method include contacting water with an organic solution that comprises BrEA and an organic solvent such as a C1-C6 alcohol (e.g., ethanol) or acetone. Addition of water to such solutions leads to precipitation of BrEA hemihydrate. Solutions that contain BrEA hemihydrate crystals or precipitate are invention embodiments that are used to prepare solid BrEA that is later dried and stored, typically at ambient temperatures.

Precipitation of BrEA hemihydrate from water-containing solutions is accomplished by known methods, e.g., reducing the solution's temperature, using saturated or nearly saturated BrEA solutions, vacuum concentration of saturated or nearly saturated BrEA solutions (which is typically conducted at a relatively low temperature, usually about 15-25°C), seeding with saturated or nearly saturated BrEA solutions with BrEA hemihydrate crystals (e.g., about 10-100 mg per 1-10 L of solution), by heating a saturated or nearly saturated BrEA solution (about 25-35°C for a few minutes followed by allowing the temperature to fall or by actively cooling the solution) and optionally seeding the solution with BrEA hemihydrate crystals or by addition of a liquid, e.g., additional water or ethanol, to a saturated or nearly saturated BrEA ethanol-water solution, which causes the solution to become supersaturated. BrEA may also be precipitated from other solvents or solvent systems, including acetone and acetone-ethanol. Such solvents are typically water miscible. Two-stage precipitation of BrEA may also be used to recover solid BrEA hemihydrate, e.g., initial precipitation and recovery of the solid, followed by either cooling and seeding of the mother liquor or by allowing the mother liquor to stand, e.g., for about one, two or more days at ambient temperature, to obtain a second crop of crystals. Also, BrEA hemihydrate crystals may optionally be recrystallized, essentially as described herein, to further increase the purity of the final solid. Methods for crystallizing organic compounds have been described, e.g., A.S. Myerson, *Handbook of Industrial Crystallization,* 1993, Butterworth-Heinemann, Stoneham, MA, p 1-101.

Other related embodiments comprise a product produced by the process of contacting a solution comprising BrEA and an organic solvent with water. Typically the solutions are as described above, e.g., a solution comprising about 3-5% v/v water and at least about 40% v/v of one or more water-miscible solvents, typically polar solvents such as C₁₋₆ alcohols or ketones (e.g., methanol, ethanol, propanol, isopropanol, butanol, typically ethanol or acetone). Such processes are accomplished by any one or more of the techniques described in the paragraph above, e.g., cooling of a saturated or nearly saturated BrEA water-ethanol solution and optionally seeding the cooled solution with BrEA hemihydrate. An embodiment related to this comprises solutions or solids that comprise wet BrEA hemihydrate crystals or wet filtered or centrifuged BrEA hemihydrate cakes, which may be obtained after crystallization. Examples of these embodiments include adding water to a BrEA-alcohol solution, e.g., slow addition of about 0.5-1.5 volumes or about 0.8-1.2 volumes of water to about 6 volumes of a BrEA-ethanol solution to obtain BrEA hemihydrate. Other examples of these embodiments include adding water to a BrEA-ketone solvent solution, e.g., slow addition of about 0.5-1.5 volumes or about 0.8-1.2 volumes of water to about 10 volumes of a BrEA-acetone solution to obtain BrEA hemihydrate.

Another related embodiment is BrEA hemihydrate that is milled to an average particle size of about 0.01-200 µM, or about 0.1-10 µM or about 0.5-5 µM. Average particle size or diameter for milled BrEA hemihydrate may thus be relatively small, e.g., about 0.03-2.0 µM or about 0.1-1.0 µM, or somewhat larger, e.g., about about 0.5-5.0 µM or about 1-5.0 µM. Milled BrEA hemihydrate is suitable for preparing solid formulations and parenteral formulations for human or veterinary use. The milled material facilitates dissolution of BrEA hemihydrate in solvents or excipients and facilitates mixing with solids or solid excipients.

While it is possible to administer BrEA hemihydrate as a pure compound to a subject, it is usually presented as a solid formulation or used to prepare a liquid formulation. Formulations will typically be used to prepare unit dosages, e.g., tablets, capsules or lozenges for oral, buccal or sublingual administration, that comprise about 10-1000 mg or typically about 25-400 mg of BrEA hemihydrate. Alternatively, embodiments include a product for parenteral (e.g., subcutaneous, subdermal, intravenous, intramuscular, intraperitoneal) administration made by the process of contacting BrEA hemihydrate and a liquid excipient, e.g., any one, two, three or more of PEG 100, PEG 200, PEG 300, PEG 400, propylene glycol, benzyl benzoate, benzyl alcohol or ethanol, and optionally sterilizing the solution and optionally dispensing the solution into vials or ampules (typically amber glass), which may be single-use or multi-use and optionally storing the formulation at reduced temperature (about 0-12°C, or about 2-10°C). Such products for parenteral administration typically comprise BrEA at a concentration of about 10-170 mg/mL, usually at about 20-110 mg/mL or about 30-100 mg/mL, and optionally one or more of a salt, buffer or bacteriostat or preservative (e.g., NaCl, BHA, BHT or EDTA).

Other embodiments include a product produced by the process of contacting BrEA hemihydrate, which may be substantially free of other forms of BrEA, with an excipient suitable for human pharmaceutical use or for veterinary use. The product is useful to make formulations or unit dosage forms that contain the hemihydrate.

Formulations made from or containing BrEA hemihydrate will usually be stored under conditions that limit the amount of water that reaches the formulation, e.g., silica gel in a sealed container that holds a formulation. Water permeation characteristics of containers have been described, e.g., Containers - Permeation, Chapter, USP 23, 1995, U.S. Pharmacopeial Convention, Inc., Rockville, MD, p.1787.

Embodiments include invention compositions that transiently occur when a method step or operation is performed. For example, when a formula 1 compound such as BrEA, containing less than about 3% water is contacted with an excipient, e.g., a PEG, an alcohol, propylene glycol or benzyl benzoate, the composition before addition of one ingredient with another is a non-homogenous mixture. As the ingredients are contacted, the mixture's homogeneity increases and the proportion of ingredients relative to each other approaches a desired value. Thus, invention compositions, which contain less than about 3% water can comprise about 0.0001-99% of a formula 1 compound such as BrEA and one or more excipients. These transient compositions are intermediates that necessarily arise when one makes an invention composition or formulation and they are included in invention embodiments to the extent that they are patentable.

In general, the formula 1 compound that is present in invention compositions and formulations is completely dissolved in the non-aqueous excipients. However, in some embodiments, e.g., transient compositions and some formulations, the formula 1 compound is partially dissolved while the remaining portion is present as a solid, which can be a suspension or a colloid.

Invention compositions and formulations suitable for parenteral delivery of formula 1 compounds to humans or animals typically comprise two, three or more excipients. Exemplary embodiments include (1) any two, three or four of propylene glycol, PEG200, PEG300, ethanol and benzyl benzoate and (2) any two, three or four of propylene glycol, PEG100, PEG200, PEG300, PEG400 and benzyl benzoate.

Invention compositions and formulations generally comprise about 0.01-10% of BrEA, usually about 1-5%, and about 0.01-3% water, typically about 0.05-3%, usually about 0.1-1 %. The invention formulations are usually presented as unit or multi-unit dosages suitable for parenteral administration once or twice per day or once per 2-3 days. Unit dosages comprise about 3-1000 mg of BrEA per unit dose, typically about 5-500 mg, usually about 10-200 mg. For treating retroviruses such as HIV in humans, a unit dose usually comprises about 10-250 mg of BrEA, usually about 100-200 mg, in a volume of about 1-6 mL, usually about 2-4 mL.

Invention embodiments include the product made by a process of combining, mixing or otherwise contacting BrEA and one, two or more excipients. Such products are produced by routine methods of contacting the ingredients. Such products optionally also contain a diluent, a disintegrant and a binder, or other excipients described herein or in references cited herein.

BrEA in the presence of significant amounts of water was found to epimerize at the bromine atom, leading to a mixture of the 16α- and 16β-BrEA isomers. Invention compositions and formulations comprising BrEA or BrEA hemihydrate will usually have a water content of less than about 3%, typically less than about 0.3%, usually less than about 0.1%. These compositions and formulations were found to have a good stability when stored at ambient temperature (about 5-40°C as used herein) in closed containers compared to control compositions and formulations having more water. Such liquids are also characterized by an unexpected reduction in precipitation of the compound, which water appears to induce.

Invention embodiments include compositions that comprise less than about 3% water, a formula 1 compound and a compound that is not generally considered suitable for human use but is useful to make an invention formulation for veterinary use. Veterinary formulations are compositions useful for the purpose of administering invention compositions to primates, cats, dogs, horses, cows, rabbits and other subjects and may contain excipients acceptable in the veterinary art and are compatible with formula 1 compounds such as BrEA. These veterinary compositions may not always be suitable for human use because they contain an excipient that is not suitable for human use, e.g., an alcohol other than ethanol such as methanol, propanol or butanol. Typically such excipients will be present at relatively low levels, e.g., about 1-30%, usually about 1-5%.

Invention embodiments include compositions and formulations, e.g., unit dosage forms and sterile solutions, that comprise (1) about 1-100 mg/mL of a formula 1 compound(s), about 57.5% propylene glycol, about 25% PEG300, about 12.5% ethanol and about 5% benzyl benzoate; (2) about 1-60 mg/mL of a formula 1 compound(s), about 70% propylene glycol, about 25% PEG300 and about 5% benzyl benzoate; (3) about 1-60 mg/mL of a formula 1 compound(s), about 25% PEG300, about 35% propylene glycol, about 35% mannitol and about 5% benzyl benzoate; (4) about 1-60 mg/mL of a formula 1 compound(s), about 57.5% propylene glycol, a mixture comprising about 25% PEG300 and PEG200 (e.g., PEG300:PEG200 in a ratio of about 1:10 to about 10:1). about 12.5% ethanol and about 5% benzyl benzoate; (5) about 1-60 mg/mL of a formula 1 compound(s), about 75% propylene glycol, a mixture comprising about 25% PEG300 and PEG200 (e.g., a PEG300:PEG200 in a ratio of about 1:10 to about 10:1) and about 5% benzyl benzoate; (6) about 1-60 mg/mL of a formula 1 compound(s), about 25% PEG300 and PEG200 (e.g., PEG300:PEG200 in a ratio of about 1:10 to about 10:1), about 35% propylene glycol, about 35% mannitol and about 5% benzyl benzoate; (7) any of formulations (1) through (6) where the formula 1 compound(s) is about 40-55 mg/mL; (8) any of formulations (1) through (6) where the formula 1 compound(s) is about 30-100 mg/mL; (9) any of formulations (1) through (8) where 1, 2, 3 or 4 formula 1 compounds are present; (10) any of formulations (1) through (8) where 1 or 2 formula 1 compounds are present; (11) any of formulations (1) through (8) where 1 formula 1 compound is present; (12) any of formulations (1) through (11) where the formula 1 compound comprises independently at 1, 2 or 3 of any of R¹-R⁶, R¹⁰, R¹⁵, R¹⁷ or R¹⁸ an independently selected ester, thioester, carbonate, carbamate, amino acid or peptide of 1 or 2 independently selected formula 1 compounds; (13) any of formulations (1) through (12) where the formula 1 compound comprises or is BrEA or BrEA hemihydrate; (14) any of formulations (1) through (13) where the formula 1 compound comprises or is an ester, a sulfate ester or phosphoester of BrEA.

Other embodiments include the product obtained by storing invention compositions or formulations, e.g., unit dosage forms, any of embodiments (1)-(14) above, or compositions used to make formulations, at about 10-40°C for at least about 3 days, e.g., storage at ambient temperature for about 1-24 months. Invention formulations will typically be stored in hermetically or induction sealed containers for these time periods. Invention compositions will typically be held in closed containers. The specification and claims disclose exemplary suitable formulations and unit dosage forms for these embodiments.

**Intermittent dosing methods.** One can intermittently administer the formula 1 compound(s), e.g., BrEA hemihydrate, to a subject without some of the undesired aspects normally associated with discontinuous dosing. Such undesired aspects include development of resistance of a pathogen (virus such as HIV or a parasite such as a *Plasmodium* parasite) to the therapeutic agent or failure of the patient or subject to adhere to a dosing regimen. Intermittent dosing protocols include administration of a formula 1 compound, e.g., orally, topically or parenterally as follows: (1) dosing for about 3 to about 20 days, (2) no dosing of the formula 1 compound for about 4 to about 20 days, (3) dosing for about 4 to about 20 days and (4) optionally repeating the dosing protocol 1, 2, 3, 4, 5, 6, 10, 15, 20, 30 or more times. Often, the dosing of steps (1) and (3) will be maintained for about 3-15 days, usually about 3-5 days. In general, steps (1)-(3) of the dosing protocol recited above, will be repeated at least one time, typically at least 2, 3, 4, 5 or 6 times. For infections that tend to remain chronic, e.g., HIV, HCV or other chronic virus or parasite infection, the intermittent dosing protocol is typically maintained over a relatively long time period, e.g., for at least about 6 months to about 5 years.

In these intermittent dosing protocols, the formula 1 compound(s) can be administered by any suitable route, e.g., intramuscular (i.m. or I.M.), subcutaneous (s.c. or S.C.), intravenous (i.v. or I.V.), intradermal, other parenteral route, aerosol using about 0.1 to about 10 mg/kg/day, usually about 0.2-4 mg/kg/day. Alternatively, one can administer the formula 1 compound(s) orally using about 4 to about 40 mg/kg/day, usually about 6-20 mg/kg/day. In some embodiments, the intermittent dosing methods exclude dosing protocols that are commonly used to deliver contraceptive steroids to, e.g., human females, such as daily dosing for 21 days, followed by no dosing for 7 days. In general, the non-aqueous formulations described herein that contain formula 1 compound(s) are administered i.m. or s.c., while aqueous formulations that contain formula 1 compound(s) is administered by i.v., i.m., s.c. or other parenteral routes. The daily doses can be administered as a single dose, especially for doses given parenterally, or the dose can be subdivided into two, three or four subdoses, usually two, especially for doses given orally.

Exemplary embodiments are (a) administering a formula 1 compound(s), e.g., BrEA hemihydrate, once every other day for 20 days, followed by (b) no dosing for 1, 2, 3, 4, 5, 6, 10, 15, 20, 25, 30, 35, 40, 45, 50, 55, 60 or more days and then (c) administering the formula 1 compound(s) at least once more on one day, e.g., administering the formula 1 compound(s) once every other day for 20 days and (d) optionally repeating (a), (b) and (c) 1, 2, 3, 4, 5 or 6 times or more. A subset of these embodiments are (a) administering a formula 1 compound(s), e.g., BrEA hemihydrate, once every other day for 20 days, followed by (b) no dosing for about 10-40 days and then (c) administering the formula 1 compound(s) at least once more on one day, e.g., administering the formula 1 compound(s) once every other day for 20 days and (d) optionally repeating (a), (b) and (c) 1, 2, 3, 4, 5 or 6 times or more. In any of these embodiments, one can administer the formula 1 compound(s) in 2 or 3 subdoses per day.

Other embodiments are (a) administering a formula 1 compound(s), e.g., BrEA, once every day for about 8-12 days, followed by (b) no dosing for 1, 2, 3, 4, 5, 6, 10, 15, 20, 25, 30, 35, 40, 45, 50, 55, 60 or more days and then (c) administering the formula 1 compound(s) at least once more on one day, e.g., administering the formula 1 compound(s) once per day for about 8-12 days and (d) optionally repeating (a), (b) and (c) 1, 2, 3, 4, 5 or 6 times or more. A subset of these embodiments are (a) administering a formula 1 compound(s), e.g., BrEA hemihydrate, once every day for about 10 days, followed by (b) no dosing for about 10-40 days and then (c) administering the formula 1 compound(s) at least once more on one day, e.g., administering the formula 1 compound(s) once per day for about 10 days and (d) optionally repeating (a), (b) and (c) 1, 2, 3, 4, 5 or 6 times or more. In any of these embodiments, one can administer the formula 1 compound(s) in 2 or 3 subdoses per day.

One aspect of invention intermittent dosing is monitoring the subject's response to dosing. For example, while dosing a subject who has a viral infection (e.g., HCV, HIV, SIV, SHIV), one can measure the subject's or pathogen's response, e.g., amelioration of one or more symptoms or a change in infectious particles or viral RNA in the serum. Once a response is observed dosing can be continued for one, two or three additional days, followed by discontinuing the dosing for at least one day (at least 24 hours), usually for at least 2 or 3 days. Once the subject's response shows signs of remission (e.g., viral serum RNA begins to increase), dosing can be resumed for another course. An aspect of the subject's response to formula 1 compound(s) is that the subject may show a measurable response within a short time, usually about 5-10 days, which allows straightforward tracking of the subject's response, e.g., by monitoring viral titer in peripheral white blood cells ("PBMC") or by measuring viral nucleic acid levels in the blood. One may monitor one or more immune cell subsets, e.g., NK, LAK, dendritic cells or cells that mediate ADCC immune responses, during and after intermittent dosing to monitor the subject's response and to determine when further administration of the formula 1 compound is indicated. These cell subsets are monitored as described herein, e.g., by flow cytometry.

For any of the treatments or methods described herein, prolonged beneficial effects or a sustained immune response by a subject may result from a single administration or a few daily administrations of the formula 1 compound for from intermittent treatment with the formula 1 compound. A single administration means that a formula 1 compound is administered to the subject in one, two, three or more doses within a 24 hour period and no further administration of any formula 1 compound to the subject occurs for at least about 45 days to about 2 months, e.g., for 3, 4, 5, 6 or more months. Prolonged beneficial effects or immune responses may also persist after a short course of treatment has been completed (e.g., daily dosing for 2, 3, 4, 5 or 6 days) and the subject is no longer receiving any formula 1 compound, or, in some cases, any other therapeutic treatment to treat the primary cause of the subject's pathological condition. Such beneficial effects can persist for more than about 5-30 days.

In some cases, beneficial effects from treatment have been observed for more than 3 months (4 or 5 or more months) after a short course of treatment of a subject with a formula 1 compound. Thus, administration of a formula 1 compound provides a method to effectively protect a subject against progression of an infection or against adverse consequences of unwanted immune reactions (e.g., inflammation) or against immunosuppression (from infection, chemotherapy, etc), without any dosing of the compound for at least 3 months after an initial dosing protocol, which could be an intermittent or a continuous dosing protocol over, e.g., 1 day to about 4 months (1-15 days, about 1 month, about 2 months, etc).

**Formulations and compositions for preparing formulations.** While it is possible for the active ingredient(s) to be administered alone it is usual to present them as pharmaceutical formulations. The formulations, both for veterinary and for human use, of the invention comprise at least one active ingredient, i.e., a formula 1 compound, together with one or more acceptable excipients therefor and optionally other therapeutic ingredients.

Another aspect of the invention relates to compositions comprising one or more pharmaceutically acceptable excipients or carriers. One or more formula 1 compound(s) (also referred to as the "active ingredient(s)") are administered by any route appropriate to the condition to be treated. Suitable routes for the non-aqueous liquid formulations and other formula 1 compound formulations include oral, rectal, nasal, topical (including buccal and sublingual), vaginal and parenteral (including subcutaneous, intramuscular, intravenous, intradermal, intrathecal and epidural). In general, the non-aqueous liquid formulations are delivered by a parenteral route. In other embodiments, such as the invention intermittent dosing methods, the formula 1 compound(s) may be present as a non-aqueous liquid formulation, a dry solid formulation that is an oral, topical, parenteral formulation, or as an aqueous liquid formulation that is used parenterally, orally or topically. It will be appreciated that the preferred route may vary with, for example, the subject's pathological condition or weight or the subject's response to therapy with a formula 1 compound or other therapy appropriate to the circumstances.

The formulations include those suitable for the foregoing administration routes. The formulations may conveniently be presented in unit dosage form and may be prepared by any of the methods well known in the art of pharmacy. Techniques, excipients and formulations generally are found in, e.g., *Remington's Pharmaceutical Sciences*, Mack Publishing Co., Easton, PA 1985, 17^{th} edition, Nema et al., *PDA J. Pharm. Sci. Tech*. 1997 51:166-171. Methods to make invention formulations include the step of bringing into association an active ingredient(s) with the excipient(s). In general the formulations are prepared by uniformly and intimately bringing into association the active ingredient with liquid excipients or finely divided solid excipients or both, and then, if appropriate, shaping the product.

Formulations of the invention suitable for oral administration are prepared as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient(s) may also be presented as a bolus, electuary or paste.

A tablet is made by compression or molding, optionally with one or more accessory ingredients. Compressed tablets may be prepared by compressing in a suitable machine the active ingredient(s) in a free-flowing form such as a powder or granules, optionally mixed with a binder, lubricant, inert diluent, preservative, surface active or dispersing agent Molded tablets may be made by molding in a suitable machine a mixture of the powdered active ingredient(s) moistened with an inert liquid diluent The tablets may optionally be coated or scored and optionally are formulated so as to provide slow or controlled release of the active ingredient(s) therefrom.

For infections of the eye or other external tissues e.g. mouth and skin, the formulations are typically applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc.), often 0.2 to 15% w/w and most often 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound that enhances absorption or penetration of the active ingredient(s) through the skin or other affected areas. Examples of such dermal penetration enhancers include dimethyl sulphoxide and related analogs.

The oily phase of the emulsions of this invention may be constituted from known excipients in a known manner. While the phase may comprise merely an emulsifier (otherwise known as an emulgent), it desirably comprises a mixture of at least one emulsifier with a fat or an oil or with both a fat and an oil. A hydrophilic emulsifier may be included together with a lipophilic emulsifier, which acts as a stabilizer. Some embodiments include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

Emulgents and emulsion stabilizers suitable for use in the formulation of the invention include Tween60™, Span80™, cetostearyl alcohol, benzyl alcohol, myristyl alcohol, glyceryl mono-stearate and sodium lauryl sulfate.

The choice of suitable oils or fats for the formulation is based on achieving the desired cosmetic properties. Creams are generally a non-greasy, non-staining and washable products with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used. These may be used alone or in combination depending on the properties required. Alternatively, high melting point lipids such as white soft paraffin and/or liquid paraffin or other mineral oils are used.

Formulations suitable for topical administration to the eye also include eye drops wherein the active ingredient(s) is dissolved or suspended in a suitable excipient(s), especially an aqueous solvent for active ingredient(s) that comprise one or more charges at pH values near neutrality, e.g., about pH 6-8. The active ingredient(s) is typically present in such formulations in a concentration of about 0.5-20% w/w, typically about 1-10% w/w, often about 2-5% w/w.

Formulations suitable for topical administration in the mouth include lozenges comprising the active ingredient in a flavored basis, usually sucrose and acacia or tragacanth; pastilles comprising the active ingredient(s) in an inert basis such as gelatin and glycerin, or sucrose and acacia; and mouthwashes comprising the active ingredient in a suitable liquid excipient(s).

Formulations for rectal administration may be presented as a suppository with a suitable base comprising for example cocoa butter or a salicylate.

Formulations suitable for intrapulmonary or nasal administration have a particle size for example in the range of 0.01 to 500 microns (including average particle sizes in a range between 0.01 and 500 microns in 0.1 micron or other increments, e.g., 0.05, 0.1, 0.5, 1, 1.5, 2.0, 2.5, 3.0, 3.5, 4.0, 4.5, 5.0, 6, 7, 8, 9, 10, 20, 25, 30, 35, 50, 75, 100, etc. microns), which is administered by rapid inhalation through the nasal passage or by inhalation through the mouth so as to reach the alveolar sacs. Suitable micronized formulations include aqueous or oily solutions or suspensions of the active ingredient(s). Formulations suitable for aerosol, dry powder or tablet administration may be prepared according to conventional methods and may be delivered with other therapeutic agents such as compounds heretofore used in the treatment or prophylaxis of viral or other infections as described herein. Such formulation may be administered, e.g., orally, parenterally (i.v., i.m., s.c.), topically or by a buccal route.

Formulations suitable for vaginal administration may be presented as pessaries, tampons, creams, gels, pastes, foams or spray formulations containing in addition to the active ingredient(s) such excipients as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations are presented in unit-dose or multi-dose containers, for example sealed ampules and vials, and may be stored in a freeze-dried (lyophilized) condition requiring only the addition of the sterile liquid excipient, for example water for injection, immediately prior to use. Extemporaneous injection solutions and suspensions are prepared from sterile powders, granules and tablets of the kind previously described. Unit dosage formulations are those containing a daily dose or unit daily sub-dose, as recited herein, or an appropriate fraction thereof, of the active ingredient(s).

It should be understood that in addition to the ingredients particularly mentioned above the formulations of this invention may include other agents or excipients conventional in the art having regard to the type of formulation in question, for example those suitable for oral administration may include flavoring agents.

The invention further provides veterinary compositions comprising at least one active ingredient as above defined together with a veterinary excipient(s) therefor. Veterinary excipients are materials useful for the purpose of administering the composition and may be solid, liquid or gaseous materials that are otherwise inert or acceptable in the veterinary art and are compatible with the active ingredient(s). These veterinary compositions may be administered orally, parenterally or by any other desired route.

Invention formulations include controlled release pharmaceutical formulations containing an active ingredient(s) ("controlled release formulations") in which the release of the active ingredient(s) is controlled and regulated to allow less frequency dosing or to improve the pharmacokinetic or toxicity profile of a given active ingredient(s).

An effective dose of active ingredient(s) depends at least on the nature of the condition being treated, toxicity, whether the compound(s) is being used prophylactically (lower doses) or against an active infection or condition, the method of delivery, and the pharmaceutical formulation, and will be determined by the clinician using conventional dose escalation studies. It can be expected to be from about 0.05 to about 30 mg/kg body weight per day. For example, for topical delivery the daily candidate dose for an adult human of approximately 70 kg body weight will range from about 1 mg to about 500 mg, generally between about 5 mg and about 40 mg, and may take the form of single or multiple doses or administration sites.

Embodiments include formulations that comprise a liposome or lipid complex that comprises a formula 1 compound(s), e.g., carbamate, carbonate, amino acid or peptide thereof. Such formulations are prepared according to known methods, e.g., U.S. patents 4427649, 5043165, 5714163, 5744158, 5783211, 5795589, 5795987, 5798348, 5811118, 5820848, 5834016 and 5882678. The liposomes optionally contain an additional therapeutic agent(s), e.g., amphotericin B, cis-platin, adriamycin, a protease inhibitor, a nucleoside or a nucleotide analog, such as one of those mentioned herein. Formulations that comprise liposomes can be delivered to a subject by any standard route, e.g., oral, aerosol or parenteral (e.g., s.c., i.v. or i.m.).

**Therapeutic applications.** The formula 1 compound, or the biologically active substances produced from these compounds by hydrolysis or metabolism *in vivo*, have a number of clinical and non-clinical applications. The compounds are generally useful to enhance Th1 immune responses or to reduce Th2 immune responses. As used herein, reference to Th1 or Th2 immune responses means such responses as observed in mammals generally and not as observed in the murine system, from which the Th1 and Th2 terminology originated. Thus, in humans, Th1 cells preferentially display chemokine receptors CXCR3 and CCR5, while Th2 cells preferentially express the CCR4 molecule and a smaller amount of the CCR3 molecule.

Aspects of the invention include compositions that comprise an amount of at least one formula 1 compound effective to enhance the relative proportion of a desired immune cell subset, e.g., CD4⁺ T cells, NK cells or dendritic cells, or to modulate one or more functions of immune cell subsets and a pharmaceutically acceptable carrier. Typical immune modulation centers on modulating expression of gene(s) that enhance of Th1 immune responses or reduces of Th2 immune responses. Functions that the formula 1 compound affected include expression of CD molecules or alteration of the proportion of cell subsets, e.g., CD4⁺ or CD8⁺ T cells, or their relative numbers in a subject's blood or tissues. CD molecules participate in the function of various immune cell subsets and can be useful as markers for immune function *in vivo*. In some aspects, the formula 1 compound activate immune cells which generally alters (increases or decreases) expression of, or changes the numbers of cells that express combinations of, CD4, CD6, CD8, CD25, CD27, CD28, CD30, CD38, CD39, CD43, CD45RA, CD45RO, CD62L, CD69, CD71, CD90 or HLA-DR molecules. Often, the numbers of cells that express these molecules are increased, e.g., CD25, CD16 or CD69. Typically such increases are observed as an increased proportion of circulating white blood cells that express one or more of these molecules. In some cases the number of such molecules per cell is detectably altered.

Expression of one or more adhesion molecules CD2, CD5, CD8, CD11a, CD11b, CD11c, CD18, CD29, CD31, CD44, CD49a, CD49b, CD49c, CD49d, CD49e, CD49f, CD50, CD54, CD58, CD103 or CD104 are also detectably affected after administration of the formula 1 compounds to a subject. Often, the numbers of cells that express these molecules are increased, e.g., CD5 or CD56. The adhesion molecules function in various aspects of immune responses, such as binding to class I MHC molecules, transducing signals between cells or binding to molecules in the extracellular matrix associated with endothelial or other cell types. Administration of the formula 1 compounds to a subject also affects the numbers of certain immune cell subsets, e.g., NK cells (e.g., CD8⁻, CD56⁺ or CD8⁺, CD56⁺) or lymphokine activated killer cells (LAK). Increased circulating NK or LAK cells are typically observed, which is reflected in increased numbers of cells that express one or more of CD16, CD38, CD56, CD57 or CD94. Also, increased numbers of circulating dendritic cell precursors are observed, as shown by increases in cells that express one or more of CD11c, CD80, CD83, CD106 or CD123. Although one can observe an increased proportion of circulating white blood cells that express one or more of these molecules, in some instances the number of such molecules per cell is detectably altered. Both the cell numbers and the density of CD molecule per cell can also be detectably modulated. Modulation of immune cell subsets typically occurs on intermittent dosing of a formula 1 compound.

Expression of one or more homing receptors such as CD62L is may also be detectably affected after administration of the formula 1 compounds to a subject. Often, the numbers of cells that express these molecules are increased, e.g., CD62L.

Other CD molecules that are modulated by the presence of the formula 1 compounds in a subject include cytokine receptor molecules such as CD115, CDW116, CD117, CD118, CDW119, CD120a, CD120b, CD121a, CD121b, CD122, CD123, CD124, CD125 CD126, CDW127, CDW128 or CDW130. Often, the numbers of receptor molecules per cell will be modulated. For example, receptors for cytokines that mediate Th1 immune responses (e.g., IL-2, γIFN) will typically increase in or on cells that mediate Th1 immune responses. Modulation of these molecules may be by direct interactions with a receptor(s) in the cell that expresses the cytokine receptor or indirectly by modulation of cytokine synthesis in the affected cells or in other cells, typically immune cells, that may interact with the cells whose receptor synthesis is being modulated.

Treatment of a subject with a formula 1 compound can result in a change of at least 25-50% above or below (e.g., at least 30% or at least 40% above or below) the control or basal level of some immune cell subsets. For example, increases of more than about 30% in the total numbers of activated CD8⁺ T cells, e.g., CD8⁺, CD69⁺, CD25⁺ T cells, CD8⁺, CD69⁺, CD25⁻ T cells or CD8⁺, CD69⁻, CD25⁺ T cells, usually occurs by 7 days after a single dose of a formula 1 compound to a subject. Such increases may be greater than 50%, 60% or 100% in the total numbers of activated CD8⁺ T cells or subsets of activated CD8⁺ T cells in individual subjects. Typically such increases are about in the total numbers of activated CD8⁺ T cells or subsets of activated CD8⁺ T cells averages about 30-40%, with individual subjects experiencing increases over 100% in the numbers of activated CD8⁺ T cells per unit blood volume compared to the basal level.

Administration of the formula 1 compounds can affect other immune cell subsets. For example, the concentration of circulating CD4⁺, CD69⁺, CD25⁻ (Th1 helper cells) and CD8⁺, CD16⁺, CD38⁺ LAK cells or CD8⁻, CD16⁺, CD38⁺ LAK cells typically increases during or after the course of dosing a subject with a formula 1 compound. Also, CD8⁻, CD16⁺, CD38⁺ and CD8⁺, CD16⁺, CD38⁺ (ADCC effector cells) and low side scatter Lin⁻, DR⁺, CD123⁺ (dendritic precursors) or low side scatter Lin⁻, DR⁺, CD11c⁺ (dendritic cells or precursors) may show modest to significant increases.

In subjects that are immunosuppressed, e.g., from infection (e.g., viral (HIV, HCV), bacterial infection or parasite infection) or from chemotherapy (e.g., an antiviral therapy, a cancer chemotherapy or a radiation therapy), administration of the formula 1 compounds to the subject results in a favorable shift in the balance of Th1 or Th2 responses the subject can mount in the face of immunosuppression. When Th1 responses are suboptimal or insufficient, treatment with a formula 1 compound results in enhancement of Th1 responses or a reduction in Th2 responses. Conversely, when Th2 responses are suboptimal or insufficient, treatment with a formula 1 compound results in enhancement of Th2 responses or a reduction in Th1 responses. The formula 1 compounds can thus be used to shift the nature of a subject's immune response to result in a more balanced immune response from immunosuppression. Alternatively, the compounds can selectively suppress inappropriate or unwanted immune responses. Enhanced Th1 responses appears to be at least partly due to one or more of (i) a reduction in biological restraints, e.g., high levels of IL-4 or IL-10, on Th1 functions by preexisting primed Th1 effector cells, (ii) enhanced differentiation of Th0 cells to Th1 cells or enhanced responses mediated by Th1 cells, (iii) enhanced function of accessory cell function, e.g., antigen presentation by dendritic precursor cells or by macrophages, (iv) enhanced proliferation and differentiation of Th1 precursor or progenitor cells, (v) enhanced IL-12 expression in dendritic cells or their precursors, which results in enhanced differentiation of Th1 cells from Th0 precursors, (vi) enhanced expression or activity of factors associated with Th1 functions, e.g., IL-2, gamma interferon (γIFN) or lymphotoxin.

An aspect of the invention methods is an alteration in the expression of IL-4 or IL-10 that occurs after administration of a formula 1 compound to a subject. A consistent observation is that extracellular IL-4 or IL-10 levels rapidly decrease to levels that are undetectable by ELISA. Intracellular IL-10 levels are reduced to levels that are near or below the limits of detection by flow cytometry. The administration of a formula 1 compound to a subject thus provides a means to inhibit either or both of these interleukins. Such inhibition may be associated with enhancement of Th1 immune responses relative to Th2 or Th0 responses, e.g., in subjects where Th1 responses are suppressed (e.g., from viral, bacterial or parasite infection (HIV, HCV, etc) or chemotherapy) or are otherwise suboptimal. In many subjects, levels of either IL-4 or IL-10, usually IL-10, before dosing with a formula 1 compound is low or undetectable. In these subjects, dosing with the formula 1 compound results in a rapid drop in the interleukin that is detectable, usually IL-4.

In some embodiments, the formula 1 compound is administered to a subject who has a pathogen infection, such as a viral, bacterial or parasite infection. The formula 1 compounds can be considered for use in a broad scope of infections (see, e.g., J.B. Peter, editor, *Use and Interpretation of Laboratory Tests in Infectious Disease,* 5^{th} edition, Specialty Laboratories, Santa Monica, CA 90404, 1998, pages 1-271), since the compounds generally enhance Th1 immune responses and/or reduce Th2 immune responses. Difficulty in treating some infections, e.g., progressive toxoplasmic encephalitis, malaria, tuberculosis, leishmaniasis. and schistosomiasis, often appear to be associated with unwanted Th2 immune responses. Typically unwanted Th2 immune responses are associated with, or caused by, increased expression of one or more cytokines or inteneukins such as IL-4 and IL-10. Administration of a formula 1 compound, or other compounds disclosed herein, will generally reduce the expression of one or more of the Th2-associated cytokines or interleukins. At the same time, the compounds enhance the expression of one or more cytokines or interleukins associated with Th1 immune responses. Because of their capacity to modulate Th1 and Th2 immune responses, the compounds are useful for a variety of clinical conditions, e.g., infection, immunosuppression or cancer, where an enhanced Th1 immune response is desired. For example, in disseminated or diffuse tuberculosis, a reduced Th2 response would be desirable to allow a patient to slow progression of the disease or to clear infected cells more efficiently.

An aspect of the invention provides embodiments where a formula 1 compound and a glutathione reductase inhibitor such as buthathione sulfoximine [CH₃-(CH₂)₃-S(=O)(=NH)-(CH₂)₂-CHNH₂-C(O)-OH] are administered to a subject to treat infections, e.g., a parasite infection such as malaria, *Toxoplasma, Cryptosporidium*, or to treat a cancer or malignancy. The decreased supply of reduced glutathione may enhance phagocytosis by macrophage, possibly due to enhanced oxidative damage in infected cells or in replicating malignant cells. Alternatively, the use of a glutathione reductase inhibitor may result in improved recognition of infected or malignant cells by the immune system. A formula 1 compound and buthathione sulfoximine are used, e.g., to enhance clearance of ring stage malaria from infected cells or to enhance immune system recognition of malignant cells compared to the use of the formula 1 compound alone. The infections and malignancies where these embodiments apply are as described herein.

Another aspect of the invention provides for the use of a formula 1 compound and a flavonoid, e.g., a naragin flavonoid, to enhance the bioavailability of the formula 1 compound. In these embodiments, the an effective amount of a flavonoid is administered to a subject who is receiving a formula 1 compound. Typically about 1-10 mg of flavonoid per kg of body weight is administered to the subject a flavonoid such as bavachinin A, didymin (isosakuranetin-7-rutinoside or neoponcirin), flavanomarein (isookanine-7-glucoside), flavanone azine, flavanone diacetylhydrazone, flavanone hydrazone, silybin, silychristin, isosilybin or silandrin. The flavonoid compound is typically administered with the formula 1 compound or a few hours, e.g., about 1, 2 or 3 hours, before the formula 1 compound is administered to the subject.

Liposome formulations can be used to enhance delivery of the formula 1 compound(s) to certain cell types such as tumor cells (see e.g., U.S. patent 5714163) or to cells of the reticuloendothelial system ("RES"). The RES includes macrophages, mononuclear phagocytic cells, cells lining the sinusoids of the spleen, lymph nodes, and bone marrow, and the fibroblastic reticular cells of hematopoietic tissues. In general, RES cells are phagocytic and they are targets for targeted delivery of a formula 1 compound(s) *in vitro* or *in vivo* using liposomes, or other compositions or formulations. Thus, one can deliver formula 1 compound to a neoplasm that is derived from reticuloendothelial tissue (reticuloendothelioma). The liposomes may also optionally comprise a peptide from an infectious agent such as a malaria parasite. The peptides may facilitate the generation of a MHC class II and B cell response.

Other uses for the formula 1 compound include administering the compound to a subject who suffers from a pathological condition(s). The treatment may treat or ameliorate the source of the condition(s) and/or symptoms associated with the pathological condition(s) such as infection with a pathogen(s) (viruses, bacteria, fungi), a malignancy, unwanted immune response, i.e., an immune response that causes pathology and/or symptoms, e.g., autoimmune conditions or allergy or conditions such as hypoproliferation conditions, e.g., normal or impaired tissue growth, or wound healing or bum healing, or in immunosuppression conditions, e.g., conditions characterized by an absence of a desired response and/or an inadequate degree of a desired response.

Many cancers or malignancies are associated with an unwanted Th2 immune response or a deficient Th1 response. An insufficient Th1 immune response may play a role in the capacity of malignant cells to escape immune surveillance. These conditions include non-small cell lung cancer, bronchogenic carcinoma, renal cell cancer or carcinoma, lymphoma, glioma, melanoma, pancreatic or gastric adenocarcinoma, human pappilomavirus associated cervical intraepithelial neoplasia, cervical carcinoma, hepatoma and cutaneous T-cell lymphoma (mycosis fungoides, Sezary syndrome).

In some of these embodiments, the subject's hyperproliferation or malignant condition may be associated with one or more pathogens. For example hepatocellular carcinoma associated with HCV or HBV, Kaposi's sarcoma associated with HIV-1 or HIV-2, T cell leukemia associated with HTLV I, Burkitt's lymphoma associated with Epstein-Barr virus or papillomas or carcinoma associated with papilloma viruses (HPV 6, HPV 11, HPV 16, HPV 18, HPV 31, HPV 45) or gastric adenocarcinoma or gastric MALT lymphoma associated with *Helicobacter pylori* infection. In other embodiments, the formula 1 compound(s) is administered to a subject who has a hyperproliferation condition that appears to not be associated with a pathogen, e.g., melanoma, or a cancer or precancer arising in the throat, esophagus, stomach, intestine, colon, ovary, lung, breast or central nervous system.

In an exemplary embodiment, human patients suffering from melanoma or melanoma precursor lesions are treated with a topical cream formulation containing 2-20% BrEA (w/w). The cream is applied to primary nevi (dysplastic nevi or common acquired nevi), primary cutaneous melanomas, secondary cutaneous melanomas and the skin surrounding the nevi or melanomas. The areas to be treated are washed with soap or swabbed with an alcohol (e.g., ethanol or isopropanol) prior to administering the cream, when this is practical. About 0.1-0.4 g of cream, depending on the size of the treated area, is applied once or twice per day per treated region or lesion for about 10-20 days. The cream is left undisturbed at the administration site for about 15-30 minutes before the patient resumes normal activity. Progression of the nevi and melanomas is retarded in the majority of patients and significant regression is observed for some lesions. Following initial treatment, the formulation is administered every other day for at least 1 to 4 months using the same dosing described for the initial round of treatment. For these patients, standard therapy to treat precursor lesion or melanoma, e.g., dimethyl triazeno imidazole carboxamide or nitrosoureas (e.g., BCNU, CCNU), is optionally started or continued according to the recommendations of the patient's doctor and with the patient's informed approval. In cases where a tumor or precursor lesion is surgically removed and the site has sufficiently healed, the patient optionally continues using the topical formulation at the site and the adjacent surrounding area every other day for at least 1 to 4 months. In some of these embodiments, a formula 1 compound(s) is administered daily continuously as an oral composition or formulation, e.g., for a formula 1 compound(s) that is a new compound *per se.* BrEA is optionally also administered systemically using, e.g., a formulation described in the examples below to deliver 1-5 mg/kg/day every other day for about 1 week to about to 4 months, e.g., in the case of malignant melanoma.

Insufficient Th1 immune responses are ofter associated with viral infection. Viral infections may arise from DNA or RNA viruses, e.g., herpesviruses, hepadnaviruses, adenoviruses, retroviruses, togaviruses, alphaviruses, arboviruses, flaviviruses, rhinoviruses, papillomaviruses and/or pestiviruses. Exemplary viruses have been described. See, for example B. N. Fields, et al., editors, *Fundamental Virology,* 3^{rd} edition, 1996, Lippencott-Raven Publishers, see chapter 2 at pages 23-57, including table 4 at pages 26-27, table 5 at pages 28-29, chapter 17 at pages 523-539, chapters 26-27 at pages 763-916, chapter 32 at pages 1043-1108 and chapter 35 at pages 1199-1233. As used herein, retroviruses include human and animal viruses, e.g., HIV-1, HIV-2, LAV, human T-cell leukemia virus I ("HTLV I"), HTLV II, HTLV III, SIV, SHIV, FIV, FeLV. Additional viruses, including their genogroups, clades, isolates, strains and so forth, that may establish a virus infection include human hepatitis C virus ("HCV"), human hepatitis B virus ("HBV"), human hepatitis A virus ("HAV"), duck hepatitis virus, woodchuck hepatitis virus, human ("HPV", e.g., HPV 6, HPV 11, HPV 16, HPV 18, HPV 31, HPV 45) or animal papilloma viruses, Poliovirus, Herpes simplex virus 1 ("HSV-1"), Herpes simplex virus 2 ("HSV-2"), human Herpesvirus 6 ("HHV-6"), human Herpesvirus 8 ("HHV-8"), Dengue virus (types 1-4), Western Equine Encephalitis Virus, Japanese Encephalitis Virus, Yellow Fever Virus and Bovine Viral Diarrhea Virus.

Other conditions where an immune imbalance or an excessive Th2 immune response is involved include autoimmune diseases such as SLE (systemic lupus erythematosus), osteoporosis, multiple sclerosis, myasthenia gravis, Graves disease, mite-associated ulcerative dermatitis, rheumatoid arthritis and osteoarthritis. Excessive Th2 immune responses are also associated with an unwanted symptom or pathology, e.g., fatigue, pain, fever or an increased incidence of infection, that is associated with aging, allergy and inflammation conditions such as allergic bronchopulmonary aspergillosis in cystic fibrosis patients, atopic asthma, allergic respiratory disease, allergic rhinitis, atopic dermatitis, subepithelial fibrosis in airway hyperresponsiveness, chronic sinusitis, perennial allergic rhinitis, Crohn's disease (regional enteritis), ulcerative colitis, inflammatory bowel disease, fibrosing alveolitis (lung fibrosis).

Other clinical indications that have an association with or have a symptom(s) that is consistent with an excessive Th2 immune response, e.g., fatigue, pain, fever or an increased incidence of infection, are schizophrenia, acute myelitis, sarcoidosis, bums, trauma (e.g., bone fracture, hemorrhage, surgery) and immune responses to xenotransplantation. This common underlying immune component in at least part of the pathology of all of these conditions allows a single agent to be effectively used to treat the condition or to treat one or more symptoms that are associated with insufficient Th1 responses or with excessive Th2 responses. In all of the conditions where an insufficient Th1 response or an unwanted Th2 response is present, amelioration of one or more symptoms associated with the condition is accomplished by administering an effective amount of a formula 1 compound according to the methods described herein. Thus, one may intermittently administer a formula 1 compound using a formulation and a route of administration as described herein.

In some applications, the formula 1 compound may directly and/or indirectly interfere with replication, development or cell to cell transmission of a pathogen such as a virus or a parasite (malaria). Improvement in a subject's clinical condition may arise from a direct effect on an infectious agent or on a malignant cell. Interference with cellular replication can arise from inhibition of one or more enzymes that a parasite or an infected cell uses for normal replication or metabolism, e.g., glucose-6-phosphate dehydrogenase, which affects cellular generation of NADPH (see, e.g., Raineri et al., *Biochemistry* 1970 9: 2233-2243). This effect may contribute to cytostatic effects that some formula 1 compound can have. Modulation of cellular enzymes expression or activity may also interfere with replication or development of a pathogen, e.g., HIV or malaria parasites or with replication or development of neoplastic cells, e.g., inhibition of angiogenesis. Clinical improvement will also generally result from an enhanced Th1 immune response.

In other applications, embodiments are a method comprising contacting a formula 1 compound with a cell(s), whereby the formula 1 compound forms a complex with a steroid hormone receptor or results in the modulation of a biological activity. The steroid hormone receptor may be an orphan nuclear hormone receptor that displays a moderate or high binding affinity for the formula 1 compound(s). In some embodiments, the steroid receptor is a known steroid receptor. Biological effects from interaction of a formula 1 compound and a receptor can lead to interference with the replication or development of a pathogen or the cell(s) itself. For example, expression of HIV transcripts in HIV-infected cells may be altered. The receptor-formula 1 compound complex may directly interfere with LTR-dependent transcription of HIV genes, leading to reduced viral replication.

The formula 1 compounds may be used to identify receptors that modulate biological responses, e.g., receptors that participate in effecting enhanced Th1 cytokine synthesis. Invention embodiments include a method, "Method 1", which permits the determination of one or more effects of a test compound on a steroid receptor in various biological systems. Generally, the test compound is a formula 1 compound. Such systems include cells containing a DNA construct that constitutively or inducibly expresses a steroid receptor(s) of interest, e.g., SXR, CARβ, RXR, PXR, PPARα or mixtures or dimers thereof, e.g., SXR/RXR. In other biological systems, the steroid receptor can be under the transcriptional control of a regulatable promoter. Alternatively, the expression another gene such as a steroid-inducible gene, e.g., a steroid-inducible cytochrome P-450. For this method, a source of steroid receptors is generally combined with a means of monitoring them, e.g., by measuring the transcription of a gene regulated by the receptor. Cells that comprise the steroid receptor and optional monitoring means are sometimes referred to herein as the "biological system." Sources of steroid receptors include cell lines and cell populations that normally express the steroid receptor of interest and extracts obtained from such cells. Another source for a useful biological system for purposes of this method is tissues from experimental animals that express the receptor.

In one aspect, method 1 allows one to determine one or more effects of a formula 1 compound on a steroid receptor using a method that comprises (a) providing a biological system, e.g., a cell extract, cells or tissue, comprising cells having a plurality of steroid receptors that comprise monomers, homodimers or heterodimers that comprise a steroid receptor, e.g., SXR, CAR-β, RXR, PPARα, PXR or dimers that comprise one or more of these; (b) activating or inhibiting the plurality of monomers, homodimers or heterodimers that comprise the steroid receptor by contacting the cells with a steroid receptor (e.g., SXR, CAR-β, RXR, PPARα or PXR) agonist or antagonist; (c) removing substantially all of the steroid receptor agonist or antagonist from the cells; (d) determining an activity of the plurality of monomers, homodimers or heterodimers that comprise the steroid receptor while in an activated state in the absence of agonist or antagonist; (e) exposing the cells to the test compound; (f) determining at least one effect of the test compound on the activity of the plurality of monomers, homodimers or heterodimers that comprise one or more of the steroid receptors while they remain substantially free of agonist or antagonist; and (g) optionally classifying the test compound as an agonist or an antagonist of the steroid receptor, or a neutral compound having little or no detectable effect.

The effects that method 1 can measure includes determining or measuring an effect on a gene whose expression is affected by the steroid receptor. The gene could be a gene associated with a pathological condition such as an infectious agent, an immune disorder or a hyperproliferation condition.

Thus, another aspect of method 1, "method 1A", is determining if a chemical not previously known to be a modulator of protein biosynthesis can transcriptionally modulating the expression of a gene that encodes a protein associated with the maintenance or treatment of one or more symptoms of a pathological condition. This method comprises: (a) contacting a sample which comprises eucaryotic cells with a formula 1 compound, wherein the eucaryotic cells comprise a plurality of steroid receptor proteins and a DNA construct containing in 5' to 3' order (i) a modulatable transcriptional regulatory sequence of the gene encoding the protein of interest, (ii) a promoter of the gene encoding the protein of interest, and (iii) a reporter gene which expresses a polypeptide capable of producing a detectable signal, coupled to, and under the control of, the promoter, under conditions such that the chemical, if capable of acting as a transcriptional modulator of the gene encoding the protein of interest, causes a measurable or detectable signal to be produced by the polypeptide expressed by the reporter gene; (b) quantitatively determining the amount of the signal so produced; and (c) optionally comparing the amount so determined with the amount of produced signal detected in the absence of any chemical being tested or upon contacting the sample with other chemicals so as to identify the chemical as one that causes a change in the detectable signal produced by the polypeptide, and determining whether the chemical specifically transcriptionally modulates expression of the gene associated with the maintenance or treatment of one or more symptoms of the pathological condition.

In conducting method 1A, one typically contacts a sample that contains a predefined number of identical or essentially identical eucaryotic cells with a predetermined concentration of a compound of formula 1. The eucaryotic cells comprise a DNA construct that is made using conventional molecular biology methods and protocols. Generally the DNA construct contains in 5' to 3' order (i) a transcriptional regulatory sequence that participates in modulating expression of the gene that is associated with maintaining or treating the pathological condition, (ii) the gene's promoter, and (iii) a reporter gene which expresses a polypeptide capable of producing a detectable signal, coupled to, and under the control of, the promoter. The construct is maintained under conditions such that the formula 1 compound, if capable of acting as a transcriptional modulator of the gene encoding the protein of interest, causes a measurable or detectable signal to be produced by the polypeptide expressed by the reporter gene. Once sufficient time for generation of a detectable response or signal has passed, one can determine the amount of the signal produced. Typically the response or signal is measured quantitatively, but a qualitative measurement can be useful for rapid screening purposes.

For method 1A, one can also optionally compare the detectable signal with the amount of produced signal that (i) one detects in the absence of any formula 1 compound or (ii) when contacting the sample with other chemicals, which identifies the formula 1 compound as a chemical that causes a change in the detectable signal the polypeptide produces. One then typically determines whether the formula 1 compound specifically transcriptionally modulates expression of the gene associated with the maintenance or treatment of one or more symptoms of the pathological condition.

Other aspects of the method 1 and 1A include a screening method comprising separately contacting each of a plurality of identical, essentially identical or different samples, each sample containing a predefined number of such cells with a with a predetermined concentration of each different formula 1 compound to be tested, e.g., wherein the plurality of samples comprises more than about 1x10³ or more than about 1x10⁴ samples or about 0.5-5x10⁵ samples. In other aspects one determines the amount of RNA by quantitative polymerase chain reaction. In any of methods 1 or 1A, a formula 1 compound such as any one of those described or named herein may be utilized.

Aspects of the invention include another method, "method 2", which centers on identifying a gene whose expression is modulated by a candidate binding partner for infectious disease therapeutic agents. Typically the binding partner is a steroid receptor, e.g., a monomer, homodimer or heterodimer that comprises SXR, CAR-β, PXR, PPARα or RXR or a homolog or isoform thereof. The steroid receptor is typically present as a complex that comprises, e.g., the formula 1 compound and the regulated gene's DNARS, which the steroid receptor, or a complex that comprises the steroid receptor, recognizes and specifically binds to. Such complexes can also comprise a transcription factor that binds to the steroid receptor or to nucleic acid sequences adjacent to or near the DNARS. Exemplary transcription factors that may be present include one or more of ARA54, ARA55, ARA70, SRC-1, NF-κB, NFAT, AP1, Ets, p300, CBP, p300/CBP, p300/CPB-associated factor, SWI/SNF and human homologs of SWI/SNF, CBP, SF-1, RIP140, GRIP1 and Vpr. In general, one provides a first and a second group of cells *in vitro* or *in vivo* and contacts the first group of cells with the infectious disease therapeutic agent, but does not contact the second group of cells *in vitro* or *in vivo* with the infectious disease therapeutic agent. Recovering RNA from the cells, or generating cDNA derived from the RNA, is accomplished by conventional protocols. Analysis of the RNA, or cDNA derived from the RNA, from the first and the second group of cells identifies differences between them, which one can use to identify a gene whose regulation is modulated by the candidate binding partner for the infectious disease therapeutic agent or any DNARS associated with that gene.

An aspect of method 2 is determining the capacity of a formula 1 compound to modulate, or participate in the modulation of, the transcription of a gene associated with the maintenance or treatment of one or more symptoms of a pathological condition. It is expected that in general, the formula 1 compounds will cause an increase in the transcription of such genes. The pathological condition is typically one associated with an infectious agent, e.g., virus, parasite or bacterium, but can also include an immune condition, e.g., an autoimmune condition or an immune deficiency. The pathological condition may also be an insufficient immune response to an infection or an insufficient response to a hyperproliferation condition or malignancy. Other pathological conditions that one can apply the method to are inflammation conditions.

In some aspects, the formula 1 compounds used in method 2 will be labeled. Such compounds are prepared by conventional methods using standard labels, such as radiolabels, fluorescent labels or other labels as described herein and in the cited references.

An embodiment of method 2 involves analyzing the RNA, or cDNA derived from it, by subtraction hybridization. In this embodiment, the RNA or cDNA obtained from the first and second groups of cells is hybridized and the resulting duplexes are removed. This allows recovery of nucleic acids that encode genes whose transcription is modulated by the candidate binding partner, which is usually a steroid receptor. One can use conventional methods to amplify and obtain nucleic acid and protein sequence information from the nucleic acids recovered by this method. The nucleic acid sequences that are transcriptionally induced or repressed by the formula 1 compound are candidate binding partners.

A transcriptionally induced gene(s) will be enriched in the group 1 cells treated with the formula 1 compound, while any repressed gene(s) will be depleted or absent. In these embodiments, the RNA recovered after removal of duplexes is typically amplified by standard RT-PCR or PCR protocols. These protocols typically use specific sets of random primer pairs, followed by analysis of the amplified nucleic acids by gel electrophoresis. Nucleic acids that are induced by the formula 1 compound will appear as a band(s), usually duplex DNA, that is not present in the control or second set of cells. Nucleic acids that are transcriptionally repressed by the formula 1 compound's binding partner will be depleted or absent in the first group of cells. Once such gene candidates are identified, they can be cloned and expressed and the capacity of the DNARS associated with the gene to form a complex that comprises a candidate binding partner and an optionally labeled formula 1 compound is analyzed by conventional methods, e.g., equilibrium dialysis, affinity chromatography using, e.g., the DNARS immobilized on a column, or coprecipitation of complexes that comprise an optionally labeled DNARS and candidate binding partner using anti-binding partner antibodies. Nucleic acid sequence analysis is usually used to identify regions adjacent to the coding regions of the regulated gene to identify any DNARS associated with the gene. The identity of a DNARS can be established by the binding to the DNARS of complexes that comprise a candidate binding partner, e.g., a steroid receptor, and optionally also comprise a formula 1 compound. The location and identity of the DNARS can be accomplished by DNA footprinting or other methods for detecting binding interactions. The DNARS, the receptor or the formula 1 compound can be labeled in these variations of method 2.

In general, the second group of cells will be identical or essentially identical to the first group of cells. In embodiments (for both methods 1 and 2) where the cells are "essentially identical", the first or the second group of cells may differ from each other by the presence or absence of a DNA construct(s) that expresses (i) a steroid receptor and/or (ii) an easily detected protein, e.g., a β-galactosidase, a peroxidase, a phosphatase, or a chloramphenicol acetyltransferase, whose transcriptional regulation is usually modulated by a steroid receptor. In these embodiments, the difference between the first and the second group of cells is used to facilitate the analysis of the biological effects of the formula 1 compound and the steroid receptor binding partner. Groups of cells are considered "identical" if they do not display known or obvious morphological or genetic differences.

Usually, the second group of cells will serve as a control, and they will thus not be exposed to any formula 1 compound before obtaining the RNA or cDNA. But, for some embodiments, one can expose the second group of cells to a known agonist or antagonist of the steroid receptor binding partner. This allows one to compare the potency of the formula 1 compound with the potency of the agonist or antagonist.

In other embodiments, one can modify method 2 by providing a third group of cells, which is optionally used as an untreated control when the second group of cells is treated with a steroid receptor agonist or antagonist. In these embodiments, one will typically compare the effect of the formula 1 compound and the agonist or the antagonist of the expression of a gene or DNA construct. The DNA construct would comprise a promoter or other regulatory sequences that are subject to transcriptional modulation, usually increase transcription, by the formula 1 compound in concert with its binding partner.

Exemplary mammalian and other steroid receptors, including orphan steroid receptors, their homologs, isoforms and co-factors (e.g., co-repressors, transcription factors, gene promoter regions or sequences) that these complexes can comprise are steroidogenic factor-1 (SF-1), chicken ovalbumin upstream promoter-transcription factor (COUP-TFI) and its mammalian homologs, silencing mediator for retinoid and thyroid hormone receptor (SMRT) and its mammalian homologs, NF-E3, COUP-TFII and its mammalian homologs, testicular orphan receptor TR2, thyroid hormone α1 (TR α1), retinoid X receptor α, TR α1/RXR α heterodimer, direct repeat-4 thyroid hormone response element (DR4-TRE), estrogen receptor (ER), estrogen receptor related α (ERRα), estrogen receptor related β (ERRβ), steroid xenobiotic receptor (SXR), hepatocyte nuclear factor 4 (HNF-4), hepatocyte nuclear factor 3 (HNF-3), liver X receptors (LXRs), LXRα, estrogen receptor α (ERα), constitutive androstane receptor-β (CAR-β), RXR/CAR-β heterodimer, short heterodimer partner (SHP), SHP/ERα heterodimer, estrogen receptor β, SHP/ERβ heterodimer, testicular orphan receptor TR4, TR2/TR4 heterodimer, pregnane X receptor (PXR) and isoforms, cytochrome P-450 monooxygenase 3A4 gene promoter region and isoforms, HNF-4/cytochrome P-450 monooxygenase 3A4 gene promoter region and isoforms complex, HIV-1 long terminal repeat (LTR), HIV-2 LTR, TR2/HIV-1 LTR complex, TR4/HIV-1 LTR complex, TR4/HIV-1 LTR complex, TR α1/TR4/HIV-1 LTR complex, TR2 isoforms (TR2-5, TR7, TR9, TR11), DAX-1, DAX-1/steroidogenic acute regulatory protein gene promoter region, RevErb, ReverbA α, Rev-erb β, steroid receptor coactivator amplified in breast cancer (AIB 1), p300/CREB binding protein-interacting protein (p/CIP), thyroid hormone receptor (TR, T3R), thyroid hormone response elements (T3REs), constitutive androstane receptor (CAR), Xenopus xSRC-3 and mammalian (human) homologs, TAK1, TAK1/peroxisome proliferator-activated receptor α (PPARα) complex, PPARα/RXRα complex, TAK-1/RIP-140 complex, retinoic acid receptor (RAR), RARβ, TR4/RXRE complex, SF-1/steroid hydroxylase gene promoter region, SF-1/oxytocin gene promoter region, SF-1/ACTH receptor gene promoter region, rat Ear-2 and mammalian homologs, human TR3 orphan receptor (TR3), RLD-1, OR-1, androgen receptor, glucocorticoid receptor, estrogen receptor, progesterone receptor, mineralcorticoid receptor, OR1, OR1/RXRα complex, TIF-1, CBP/P300 complex, TRIP1/SUG-1 complex, RIP-140, SRC1α/P160 complex and TIF-2/GRIP-1 complex, RAR/N-CoR/RIP13 complex, RAR/SMRT/TRAC-2 complex, and the DNARS 5' AGGTCANAGGTCA 3' or 5' TGCACGTCA 3'. One of these complexes can be included in invention methods when, e.g., they are performed in cell-free assays. Formation of these complexes in cells is facilitated by inserting into the cells a DNA construct(s) that expresses one or more of these proteins, e.g., mammalian or yeast cells containing a stable DNA construct or a construct used for transient transfection assays. Methods to perform assays or to induce biological responses *in vitro* or *in vivo* using the formula 1 compounds as agonists, antagonists or as reference standards are essentially as described, see, e.g., U.S. patents 5080139, 5696133, 5932431, 5932555, 5935968, 5945279, 5945404, 5945410, 5945412, 5945448, 5952319, 5952371, 5955632, 5958710, 5958892, 5962443; International Publication Numbers WO 96/19458, WO 99/41257, WO 99/45930. The complexes or assay systems, that comprise a formula 1 compound and that are employed in the practice of these methods are included as aspects of the invention.

The formula 1 compound typically interact with one or more biological ligands to efect a biological response. To facilitate the identification of candidate binding partners for the formula 1 compound, one can use a radiolabeled formula 1 compound that is linked to a support, usually a solid support, as a means to recover the candidate binding partners. The formula 1 compound can be linked to the support through, e.g., the 3-, 7-, 16- or 17-position of the steroid nucleus. Linking agents are known for such uses and include homobifunctional and heterobifunctional agents, many of which are commercially available. The linker one uses will typically comprise about 2-20 linked atoms. The linked atoms usually comprise mostly carbon, with one, two or three oxygen, sulfur or nitrogen atoms that replace one or more carbon atoms. One can use a cDNA expression library that one has made from suitable cells or tissues as a source of candidate binding partners. The cells or tissues can be obtained from a mammalian or a vertebrate host, e.g., human, mouse, bird, primate, or from other sources, e.g., insects (e.g., Drosophila), other invertebrates (e.g., yeast, bacteria, *Mycoplasma sp., Plasmodium sp., Tetrahymena sp., C. elegans*) or other organism groups or species listed herein or in the cited references. Suitable tissues include skin, liver tissue or cells, including hepatocytes and Kupfer cells, fibrocytes, monocytes, dendritic cells, kidney cells and tissues, brain or other central nervous system cells or tissues, including neurons, astrocytes and glial cells, peripheral nervous system tissues, lung, intestine, placenta, breast, ovary, testes, muscle, including heart or myocyte tissue or cells, white blood cells, including T cells, B cells, bone marrow cells and tissues, lymph tissues or fluids and chondrocytes.

Typically a candidate binding partner that one isolates from a non-human source will have a human homolog that has similar binding properties for the formula 1 compound. Non-human candidate binding partners can thus be used to facilitate recovery of the human homologs, e.g., by preparing antiserum for precipitating the human homolog from a solution that comprises the human homolog or by comparing the sequence of the non-human candidate binding partner with known human gene sequences. Once a source of the candidate binding partner is obtained, it can be contacted with labeled formula 1 compound, usually radiolabeled with, e.g., ¹⁴C or ³H, and complexes that comprise the labeled formula 1 compound and the candidate binding partner is recovered using, e.g., affinity chromatography or antibody precipitation methods. The recovery of the complex provides a source of at least partially purified candidate binding partner, i.e., the candidate binding partner is enriched, e.g., at least 10-fold enriched, or at least 100-fold enriched, or at least 500-fold enriched, compared to its abundance in the original candidate binding partner source material.

Other aspects include a method to determine a biological activity of a formula 1 compound comprising: (a) contacting the formula 1 compound(s) with a cell or cell population; (b) measuring one or more of (i) a complex between a binding partner and the formula 1 compound, (ii) proliferation of the cell or cell population, (iii) differentiation of the cell or cell population (iv) an activity of a protein kinase C, (v) a level of phosphorylation of a protein kinase C substrate, (vi) transcription of one or more target genes, (vii) inhibition of the cellular response to steroids, e.g., glucocorticoids, (viii) inhibition of steroid-induced transcription, e.g., glucocorticoids, sex steroids or (ix) inhibition of HIV LTR-driven transcription; and (c) optionally comparing the result obtained in step (b) with an appropriate control. Aspects of this embodiment include (i) the method wherein the binding partner is a steroid receptor, a transcription factor or a DNARS, (ii) the method wherein the biological activity determined is a modulating activity of the formula 1 compound for replication or cytopathic effects associated with a retrovirus, a hepatitis virus or a protozoan parasite, (iii) the method wherein the biological activity determined is a modulating activity of the formula 1 compound for replication, cytopathic effects associated with the retrovirus, the hepatitis virus or the protozoan parasite or the biological activity determined is metabolism (assay by ³H-thymidine uptake or other assay as referenced or described herein) of a cell or cell population comprising NK cells, phagocytes, monocytes, macrophages, basophils, eosinophils, dendritic cells, synoviocytes, microglial cells, fibrocytes, transformed (neoplastic) cells, virus-infected cells, bacteria-infected cells or parasite-infected cells, and (iv) the method wherein the target gene is a virus gene, a bacterial gene, a parasite gene, a gene associated with cancer, e.g., wherein the virus gene is a DNA or an RNA polymerase gene, a reverse transcriptase gene, an envelope gene, a protease gene or a gene associated with viral nucleic acid replication or a viral structural gene.

An embodiment is a method comprising contacting a complex that comprises a steroid receptor and a formula 1 compound with a transactivator protein, whereby a complex comprising the steroid receptor protein, the formula 1 compound and the transactivator protein forms, wherein the transactivator protein is in (1) a cell or tissue extract (e.g., nuclei, lysate containing nuclei or lysate without nuclei from a cell(s) or tissue(s)), (2) a partially purified or purified cell or tissue extract, (3) a cell(s) in tissue culture or (4) a cell(s) in a subject, where any of (1)-(4) optionally comprises a target gene (native gene or introduced by standard gene manipulation techniques) whose level of expression is optionally assayed after the complex forms. In some of these embodiments, the transactivator protein is partially purified or purified and is in the cell or tissue extract or the partially purified or purified cell or tissue extract The transactivator protein may be TIF-1, CBP/P300, TRIP1/SUG-1, RIP-140, SRC1α/P160, or TIF-2/GRIP-1. In any of these embodiments the complex comprising the steroid receptor protein, the formula 1 compound and the transactivator protein may increase or decrease transcription of the target gene compared to a suitable control (e.g., control under same conditions, but lacking any added compound that corresponds to the formula 1 compound, or where another compound (e.g., a steroid that is known to bind to the steroid receptor) is used as a benchmark or reference standard against which altered target gene expression is measured). In these methods, the target gene may be a pathogen gene (e.g., virus, bacterium, parasite, fungus, yeast) or a gene associated with a pathological condition (autoimmunity, inflammation, hyperproliferation).

The formula 1 compounds are suitable for use in certain described methods that use steroids to modulate biological activities in cells or tissues. For example, a formula 1 compound(s) can be used to selectively interact with specific steroid receptors or steroid orphan receptor, or their subtypes, that are associated with a pathological condition(s) in a subject, essentially as described in U.S. patent 5668175. In these applications, the formula 1 compound may act as a ligand for the receptor to modulate abnormal expression of a gene product(s) that correlates with the pathological condition (a steroid hormone responsive disease state). Such genes are normally regulated by steroid hormones. In other applications, one can use the formula 1 compounds to screen for ligands that bind to a steroid receptor or steroid orphan receptor and one or more transcription factors (or cofactors) such as AP-1 and/or with a DNA sequence(s), essentially as described in U.S. patent 5643720. Similarly, the formula 1 compounds can be used essentially as described in U.S. patents 5597693, 5639598, 5780220, 5863733 and 5869337. In some of these embodiments, the formula 1 compound(s) is labeled to facilitate its use. Suitable labels are known in the art and include radiolabels (e.g., ³H, ¹⁴C, ³²P, ³⁵S, ¹³¹I, ⁹⁹Tc and other halogen isotopes), fluorescent moieties (e.g., fluorescein, resorufin, Texas Red, rhodamine, BODIPY, arylsulfonate cyanines), chemiluminescent moieties (e.g., acridinium esters), metal chelators, biotin, avadin, peptide tags (e.g., histidine hexamer, a peptide recognized by monoclonal or polyclonal antibodies), covalent crosslinking moieties. One prepares the labeled compounds according to known methods.

Methods suitable to measure the biological effects of various compounds, e.g., activation, on immune system cells (e.g., NK cells, phagocytes, monocytes, macrophage, neutrophils, eosinophils, dendritic cells, synoviocytes, microglial cells, fibrocytes) have been described, e.g., Jakob et al., *J. Immunol*. 1998 161:3042-3049, Pierson et al., *Blood* 1996 87:180-189, Cash et al., *Clin. Exp. Immunol*. 1994 98:313-318, Monick et al., *J. Immunol*. 1999 162:3005-3012, Rosen et al., *Infect. Immun*. 1999 67:1180-1186, Grunfeld et al., *J. Lipid Res*. 1999 40:245-252, Singh et al., *Immunol. Cell Biol*. 1998 76:513-519, Chesney et al., *Proc. Natl. Acad. Sci. USA* 1997 94:6307-6312, Verhasselt et al., *J. Immunol*. 1999162:2569-2574, Avice et al., *J. Immunol*. 1999 162:2748-2753, Cella et al., *J. Exp. Med.* 1999 189:821-829, Rutalt et al., *Free Radical Biol. Med.* 1999 26:232-238, Akbari et al., *J. Exp. Med.* 1999189:169-178, Hryhorenko et al., *lmmunopharmacology* 1998 40:231-240, Femvik et al., *Inflamm. Res.* 1999 48:28-35, Cooper et al., *J. Infect. Dis.* 1999 179:738-742, Betsuyaku et al., *J. Clin. Invest*. 1999 103:825-832, Brown et al., *Toxicol. Sci.* 1998 46:308-316, Sibelius et al., *Infect. Immunol*. 1999 67:1125-1130. The use of formula 1 compounds in such methods are aspects of the invention and they permit, e.g., measurement of the biological effects of formula 1 compounds on genes whose expression is regulated by the formula 1 compound and the steroid receptor.

Embodiments include any of the methods described above, e.g., method 1, wherein the cells or biological system comprises NK cells, phagocytes, monocytes, macrophage, neutrophils, eosinophils, dendritic cells, synoviocytes, microglial cells, glial cells, fibrocytes or hepatocytes, that optionally comprise a DNA construct that expresses one or two cloned steroid receptors. The method optionally analyzes the effect of a formula 1 compound on the cells compared to controls. Controls include the use of a known agonist or antagonist for the steroid receptor or the comparison of cells exposed to a formula 1 compound with control cells (usually the same cell type as the treated cells) that are not exposed to the formula 1 compound. A response, e.g., activation of the steroid receptor can be measured by known assays compared to controls.

The formula 1 compound will, in some cases modulate (increase or decrease) transcription of one or more genes in the cells. In other cases, the formula 1 compound will enhance lysosome movement in one or more of the subject's NK cells, phagocytes, monocytes, macrophages, neutrophils, eosinophils, dendritic cells synoviocytes, microglial cells or fibrocytes. Such effects will typically be mediated directly or indirectly through steroid receptors that act to modulate gene transcription, e.g., cause enhances protein kinase C (PKC) activity in the cells used in the assay, e.g., PKCα, PKCβ, PKCγ or PKCζ.

Other related embodiments are a composition comprising a partially purified or a purified complex comprising a formula 1 compound and a steroid receptor, a serum steroid-binding protein (e.g., human serum albumin, α1-acid glycoprotein, sex hormone-binding globulin, testosterone-binding globulin, corticosteroid-binding globulin, androgen binding protein (rat)) or another binding partner, e.g., transcription factor or DNARS. An aspect of these compositions includes a product produced by the process of contacting the partially purified or the purified composition with one or more cells, one or more tissues, plasma or blood.

Another embodiment comproses a method to determine a biological activity of a formula 1 compound comprising: (a) contacting the formula 1 compound(s) with a cell or cell population; (b) measuring one or more of (i) a complex between a binding partner and the formula 1 compound, (ii) proliferation of the cell or cell population, (iii) differentiation of the cell or cell population (iv) an activity of a protein kinase C, (v) a level of phosphorylation of a protein kinase C substrate, (vi) transcription of one or more target genes, (vii) inhibition of the cellular response to steroids, e.g., glucocorticoids, (viii) inhibition of steroid-induced transcription, e.g., glucocorticoids, sex steroids or (ix) inhibition of HIV. LTR-driven transcription; and (c) optionally comparing the result obtained in step (b) with an appropriate control. Aspects of this embodiment include (i) the method wherein the binding partner is a steroid receptor, a transcription factor or a DNARS, (ii) the method wherein the biological activity determined is a modulating activity of the formula 1 compound for replication or cytopathic effects associated with a retrovirus, a hepatitis virus or a protozoan parasite, (iii) the method wherein the biological activity determined is a modulating activity of the formula 1 compound for replication, cytopathic effects associated with the retrovirus, the hepatitis virus or the protozoan parasite or the biological activity determined is metabolism (assay by ³H-thymidine uptake or other assay as referenced or described herein) of a cell or cell population comprising NK cells, phagocytes, monocytes, macrophages, basophils, eosinophils, dendritic cells, synoviocytes, microglial cells, fibrocytes, transformed (neoplastic) cells, virus-infected cells, bacteria-infected cells or parasite-infected cells, and (iv) the method wherein the target gene is a virus gene, a bacterial gene, a parasite gene, a gene associated with cancer, e.g., wherein the virus gene is a DNA or an RNA polymerase gene, a reverse transcriptase gene, an envelope gene, a protease gene or a gene associated with viral nucleic acid replication or a viral structural gene.

Another embodiment is a method comprising contacting a complex that comprises a steroid receptor and a formula 1 compound with a transactivator protein, whereby a complex comprising the steroid receptor protein, the formula 1 compound and the transactivator protein forms, wherein the transactivator protein is in (1) a cell or tissue extract (e.g., nuclei, lysate containing nuclei or lysate without nuclei from a cell(s) or tissue(s)), (2) a partially purified or purified cell or tissue extract, (3) a cell(s) in tissue culture or (4) a cell(s) in a subject, where any of (1)-(4) optionally comprises a target gene (native gene or introduced by standard gene manipulation techniques) whose level of expression is optionally assayed after the complex forms. In some of these embodiments, the transactivator protein is partially purified or purified and is in the cell or tissue extract or the partially purified or purified cell or tissue extract The transactivator protein may be TIF-1, CBP/P300, TRIP1/SUG-1, RIP-140, SRC1α/P160, or TIF-2/GRIP-1. In any of these embodiments the complex comprising the steroid receptor protein, the formula 1 compound and the transactivator protein may increase or decrease transcription of the target gene compared to a suitable control (e.g., control under same conditions, but lacking any added compound that corresponds to the formula 1 compound, or where another compound (e.g., a steroid that is known to bind to the steroid receptor) is used as a benchmark against which altered target gene expression is measured). In these methods, the target gene may be a pathogen gene (e.g., virus, bacterium, parasite, fungus, yeast) or a gene associated with a pathological condition (autoimmunity, inflammation, hyperproliferation).

The biological effects observed while performing the methods described herein are expected to usually involve the formation of complexes that contain two or more components. These components can include one or more transcription factors or co-regulators or co-repressors of transcription and their homologs and isoforms. These factors and complexes containing them include members of the steroid receptor coactivator-1 family (SRC-1, SRC-1/serum response factor), NF-κB, NFAT, p300, CBP, p300/CBP, p300/CPB-associated factor, SWI/SNF and human and other homologs, BRG-1, OCT-1/OAF, AP1, Ets, androgen receptor associated protein 54 (ARA54), androgen receptor associated protein 55 (ARA55), androgen receptor associated protein 70 (ARA70), RAC3/ACTR, CREB-binding protein (CPB), SRC-1α, receptor interacting protein-140 (RIP-140), transcription factor activator protein-1, activation function-2, glucocorticoid receptor-interacting protein-1 (GRIP-1), receptor interacting protein-160 (RIP-160), suppressor of gal4D lesions (SUG-1), transcription intermediary factor-1 (TIF-1), transcription intermediary factor-2 (TIF-2), SMRT, N-CoR, N-CoA-1, p/CIP, stroidogenic factor-1 (SF-1), p65 (ReIA), and Vpr encoded by the human immunodeficiency virus and its isoforms and homologs. One or more of these factors can be present in complexes that comprise a formula 1 compound and a steroid receptor, such as SXR, PPARα, CAR-β, RXR and/or PXR.

In a related embodiment, a formula 1 compound is used to exert a cytostatic effect on mammalian cells *in vitro.* Typically such cells are lymphoid cells, e.g., T cell populations from, e.g., blood or organs that are rich in lymphoid cells (e.g., spleen, lymph tissue or nodes), or transformed T cell lines. Such activity provides an estimate of the potency of formula 1 compounds to mediate immunological effects, such as enhancing Th1 immune responses or suppressing expression of one or more Th2-associated cytokines. Thus, an invention method comprises (a) contacting a formula 1 compound and lymphoid cells *in vitro,* (b) determining the degree of cytostasis that the compound exerts to identify a cytostatic compound and (c) optionally administering the cytostatic compound to an immune suppressed subject to determine the effect of the compound on one or more of the subject's immune responses as described herein, e.g., enhanced Th1 cytokine or cell response or decreased Th2-associated cytokine expression. Typically, such methods are conducted using a range of formula 1 compound concentrations and suitable controls, such as a known cytostatic agent or a blank that contains solvent that lacks the formula 1 compound. Inhibition of cell proliferation is measured by standard methods. Methods to measure the cytoststic effects of the compounds includes measuring viable cell numbers in treated and untreated cultures or by measuring DNA synthesis using e.g., ³H-thymidine incorporation into DNA in treated and untreated cultures. Typical ranges of formula 1 concentrations in the cell growth medium are about 0.1 µM to about 100 µM, using about 4-6 different concentrations of compounds with a fixed number of cells (e.g., about 0.4 x 10⁵ to about 5 x 10⁵). The formula 1 compound is left in contact with the cells in tissue culture for a sufficient time to observe cytostasis, e.g., about 16 hours to about 6 days, typically about 24-72 hours. In these embodiments, one may optionally screen for modulation of a biological activity of a steroid receptor, e.g., activation of PPARα, which may be associated with the cytostasis the compound induced.

In some applications, the formula 1 compound(s) appears to bring about an improvement of one or more of the symptoms associated with an infection or a condition. For example, treatment of subjects who are immune suppressed, e.g., from a retrovirus infection, cancer chemotherapy or other cause, generally show improvement of one or more associated symptoms, such as weight loss, fever, anemia, fatigue or reduced infection symptoms that are associated with a secondary infection(s), e.g., HSV-1, HSV-2, papilloma, human cytomegalovirus ("CMV"), *Pneumocystis* (e.g., *P. carinii*) or *Candida (C. albicans, C. krusei, C. tropicalis*) infections. The formula 1 compounds are also useful to facilitate immune system recovery in autologous bone marrow transplant or stem cell transplant situations. In some embodiments, the formula 1 compound(s) is administered as a nonaqueous liquid formulation as described herein or the formula 1 compound(s) is administered according to any of the intermittent dosing protocols described herein using a solid or liquid formulation(s). In the case of a subject who has a retroviral infection with symptoms that include one or more of, a relatively low CD4 count (e.g., about 10-200, usually about 20-100), one or more additional pathogen infections (HSV-1, HSV-2, HHV-6, HHV-8, CMV, HCV, a HPV, *P. carinii* or *Candida* infection) and one or more of anemia, fatigue, Kaposi's sarcoma, fever or involuntary weight loss (at least about 5% of body weight), administration of about 0.1 to about 10 mg/kg/day (usually about 0.4 to about 5 mg/kg/day) of a formula 1 compound(s) to the subject typically results in noticeable improvement of one or more of the symptoms within about 1-4 weeks. In other embodiments, the formula 1 compound(s) is administered to a subject who has a condition that appears to be associated with a viral infection, e.g., pneumonia or retinitis associated with CMV, nasopharyngeal carcinoma or oral hairy leukoplakia associated with Epstein-Barr virus, progressive pancephalitis or diabetes associated with Rubella virus or aplastic crisis in hemolytic anemia associated with Parvovirus 19.

In an exemplary embodiment, human patients infected with HCV are dosed with an aqueous isotonic α-cyclodextrin or β-cyclodextrin formulation containing about 20 mg/mL BrEA hemihydrate. The formulation is delivered intravenously in a single daily dose or two subdoses per day. The patients are dosed with 1 to 10 mg/kg/day for 4 to 10 days, followed by no dosing for 5 to 30 days, followed by dosing again with the cyclodextrin formulation for 4 to 10 days. The dosing regimen is repeated one, two or more times. Clinical markers for HCV infection are followed during treatment, e.g., viral nucleic acid in the blood or plasma, liver enzyme levels in the blood or plasma (e.g., AST/SGOT, ALT/SGPT, alkaline phosphatase). For these patients, a standard anti-HCV treatment(s), e.g., interferon and/or ribavirin, is optionally started or continued according to the recommendations of the patient's doctor and with the patient's informed approval. In some of these embodiments, a formula 1 compound(s) is administered daily continuously as a component in an oral or parenteral composition or formulation, e.g., for a formula 1 compound(s) that is a new compound *per se.* BrEA hemihydrate is optionally also administered systemically using, e.g., the formulation of example 1 to deliver 1-5 mg/kg/day every other day for about 1 to 4 months, or an oral formulation to deliver about 5-40 mg/kg/day every other day for about 1 to 4 months.

In any of the embodiments disclosed herein, one can optionally administer an additional therapeutic treatment in conjunction with, i.e., before, during or after, administration of a formula 1 compound(s) to a subject(s). For example, in subjects who have a viral or parasite infection and are in the course of administration of a formula 1 compound, other treatments can also be administered to the subject, e.g., nucleoside analogs for viral infections or chloroquine for malaria. Such additional treatments will typically include standard therapies for the subject's pathological condition(s), but they can also include experimental or other treatments. For example, one can coadminister vitamins (multivitamins, individual vitamins), antioxidants or other agents (vitamin E, allopurinol); nutritional supplements (liquid protein or carbohydrate preparations) or other therapies as the patient's medical condition warrants and the patient's doctor recommends. Any of these additional treatments can be coupled with the administration of any of the formula 1 compounds, e.g., BrEA hemihydrate, an ester, carbamate, carbonate or amino acid or peptide conjugate thereof, in any of the embodiments described herein.

Such additional treatments are apparent to the skilled artisan. Such treatments are selected based on the condition(s) to be treated, cross-reactivities of ingredients and pharmaco-properties of the combination. For example, when treating retroviral infections in a human or other subject, the formula 1 compounds are combined with one or more reverse transcriptase inhibitors, protease inhibitors, antibiotics or analgesics. Suitable formula 1 compounds include those described, e.g., in compound groups 1 through 42-25-20-6 and elsewhere herein. Exemplary reverse transcriptase inhibitors are AZT, 3TC, D4T, ddl, ddC, adefovir dipivoxil, 9-[2-(*R*)-[[bis[[(isopropoxycarbonyl)oxy]methoxy]-phosphinoyl]methoxy]propyl]adenine, (*R*)-9-[2-(phosphonomethoxy)propyl]-adenine and adefovir. Exemplary protease inhibitors are indinavir, nelfinavir, ritonavir, crixivan and sequanavir. Fusion inhibitors may also be used, e.g., HIV fusion inhibitors. When treating viral infections of the respiratory system or other systems, e.g., hepatitis C virus ("HCV") or influenza virus infection (e.g., influenza A or B), the compositions of the invention are optionally used in conjunction with antivirals (such as γ-interferon, amantidine, rimantadine, ribavirin or compounds disclosed in U.S. patents 5763483 (especially compounds recited in claims 1 and 2) and 5866601), mucolytics, expectorants, bronchodilators, antibiotics, antipyretics, or analgesics.

In some embodiments, formula 1 compound is administered to subjects who have a parasite or bacterial infection, to slow the progression of infection, interfere with replication or development of the infectious agent or to ameliorate one or more of the associated symptoms, e.g., weight loss, anemia or secondary infections. Parasites are malaria parasites, sleeping sickness parasites and parasites associated with gastrointestinal infections. Parasites and bacteria include species, groups, genotypes, strains or isolates of gastroentestinal helminths, microsporidia, isospora, cryptosporidia (*Cryptosporidium parvum*), *Mycobacterium* (*M. avium, M. bovis, M. leprae, M. tuberculosis, M. pneumoniae. M. penetrans*), *Mycoplasma* (*M. fermentans, M. penetrans, M. pneumoniae), Trypanosoma* (*T. brucei, T. gambiense, T. cruzi, T. evansi*), *Leishmania* (*L. donovani, L. major, L. braziliensis), Plasmodium (P. lalciparum, P. knowlesi, P. vivax, P.berghei*), *Ehrlichia (E. canis, E. chaffeensis, E. phagocytophila, E. equi,* E. *sennetsu), Babesia microti, Haemophilus (H. somnus, H. influenzae*), *Brucella (B. militensis, B. abortus*), *Bartonella (B. henselae), Bordetella (B. bronchiseptica, B. pertussis), Escherichia (E. coli), Salmonella (S. typhimurium), Shigella (S. flexnen), Pseudomonas (P. aeruginosa), Neisseria (N. gonorrhoeae, N. meningitidis), Streptococcus, Staphylococcus (S. aureus), Rickettsia (R. rickettsii*), *Yersinia (Y. enterocolitica), Legionella pneumonia* and *Listeria (L. monocytogenes)*.

One or more of the invention intermittent dosing protocols or one or more of the liquid non-aqueous formulations described herein can be applied by routine experimentation to any of the uses or applications described herein. For a formula 1 compound that is a new compound *per se,* the compound can be administered to a subject according to an invention intermittent dosing protocol(s) or by other protocols, e.g., continuous daily dosing of a single dose or two or more subdoses per day. In addition any of the formula 1 compounds, e.g., one or more formula 1 compounds that are new *per se*, can be present in any solid or liquid formulation described herein. These formulations and dosing protocols can be applied by routine experimentation to any of the uses or applications described herein.

Numbered embodiments. Several aspects of the invention and related subject matter includes the following numbered embodiments.

In some aspects, the invention relates to non-aqueous liquid formulations that comprise a formula 1 compound. Exemplary embodiments are as follows.
1. A composition comprising one or more compounds of formula 1 and one or more nonaqueous liquid excipients, wherein the composition comprises less than about 3% v/v water.
5. The composition of embodiment 1 wherein the composition comprises less than about 0.3% v/v water.
6. The composition of embodiments 1 or 5 wherein the one or more nonaqueous liquid excipients is one, two or more of an alcohol, a polyethylene glycol, propylene glycol or benzyl benzoate.
9. The composition of any of embodiments 1, 5 or 6 wherein the composition comprises two, three, four or five nonaqueous liquid excipients.
10. The composition of embodiment 9 wherein the composition comprises three or more nonaqueous liquid excipients.
11. The composition of any of embodiments 1 5, 6, 9 or 10 wherein the formula 1 compound comprises about 0.0001-99% w/v of the composition.
12. The composition of any of the foregoing embodiments wherein the composition comprises a unit dose.
13. The composition of embodiment 12 wherein the unit dose comprises about 0.5-100 mg/mL of the formula 1 compound.
14. The composition of embodiment 10 wherein the composition comprises about 1.0-60 mg/mL of the formula 1 compound.
16. The composition of embodiment 1 wherein the one or more nonaqueous liquid excipients comprise a polyethylene glycol, propylene glycol and benzyl benzoate.
17. The composition of embodiment 16 wherein the composition comprises less than about 0.3% v/v water.
20. The composition of embodiment 16 that further comprises an alcohol.
22. The composition of embodiment 1 wherein the one or more nonaqueous liquid excipients comprise benzyl benzoate, a polyethylene glycol, an alcohol and optionally an additional nonaqueous liquid excipient.
23. The composition of embodiment 22 wherein the composition comprises less than about 0.3% v/v water.
26. The composition of embodiment 22 or 23 wherein the polyethylene glycol is polyethylene glycol 300 and/or polyethylene glycol 200.
27. The composition of embodiment 26 wherein the alcohol is polyethylene glycol is polyethylene glycol 300.
28. The composition of embodiments 22 or 23 that comprises about 2.5-25% v/v ethanol, about 1-10% v/v benzyl benzoate, about 10-35% v/v polyethylene glycol 300, about 40-65% v/v propylene glycol and about 2-60 mg/mL 16α-bromo-3β-hydroxy-5α-androstan-17-one.
28A. The composition of embodiments 22, 23 or 26 that comprises about 0.1-10% v/v benzyl benzoate, about 0.1-10% v/v benzyl alcohol, about 1-95% v/v polyethylene glycol 200, about 1-95% v/v propylene glycol and about 2-60 mg/mL 16α-bromo-3β-hydroxy-5α-androstan-17-one. The embodiment 28A composition may comprise about 2% v/v benzyl benzoate, about 2% v/v benzyl alcohol, about 40% v/v polyethylene glycol 200, about 51% v/v propylene glycol (qs) and about 50 mg/mL 16α-bromo-3β-hydroxy-5α-androstan-17-one.
29. The composition of embodiment 28 that comprises about 12.5% v/v ethanol, about 5% v/v benzyl benzoate, about 25% v/v polyethylene glycol 300, about 57.5% v/v propylene glycol and about 50 mg/mL 16α-bromo-3β-hydroxy-5α-androstan-17-one.
30. The composition of any of the foregoing embodiments that further comprises a local anesthetic.
31. The composition of embodiment 30 wherein the local anesthetic is procaine, benzocaine or lidocaine.
32. The composition of any of the foregoing embodiments wherein the composition comprises a solvate, a suspension, a colloid, a gel or a combination of any of the foregoing.
33. A product produced by the process of contacting a composition comprising one or more compounds of formula 1 and a first nonaqueous liquid excipient with a second nonaqueous liquid excipient wherein the product comprises less than about 3% water and the salts, analogs, configurational isomers and tautomers thereof.
34. The product of embodiment 33 wherein the product comprises less than about 0.3% water.
35. The product of embodiments 33 or 34 wherein the first nonaqueous liquid excipient is a polyethylene glycol (e.g., PEG300 or PEG 200) or propylene glycol.
36. The product of embodiments 33, 34 or 35 wherein the second nonaqueous liquid excipient is a polyethylene glycol (e.g., PEG300 or PEG 200) or propylene glycol.
38. A product produced by the process of contacting a composition comprising one or more compounds of formula 1 and two nonaqueous liquid excipients with a third nonaqueous liquid excipient wherein the product comprises less than about 3% water and the salts, analogs, configurational isomers and tautomers thereof.
39. The product of embodiment 38 wherein the product comprises less than about 0.3% water.
40. The product of embodiments 38 or 39 wherein the two nonaqueous liquid excipients are selected from a polyethylene glycol (e.g., PEG300 or PEG 200), propylene glycol, benzyl benzoate and an alcohol (e.g., ethanol).
41. The product of embodiments 38, 39 or 40 wherein the third nonaqueous liquid excipient is a polyethylene glycol (e.g., PEG300 or PEG 200), propylene glycol, benzyl benzoate or an alcohol (e.g., ethanol).
42. A product produced by the process of contacting a composition comprising one or more compounds of formula 1 and three nonaqueous liquid excipients with a fourth nonaqueous liquid excipient wherein the product comprises less than about 3% water and the salts, analogs, configurational isomers and tautomers thereof.
43. The product of embodiment 42 wherein the product comprises less than about 0.3% water.
44. The product of embodiments 42 or 43 wherein the three nonaqueous liquid excipients are selected from a polyethylene glycol (e.g., PEG300 or PEG 200), propylene glycol, benzyl benzoate and an alcohol (e.g., ethanol).
45. The product of embodiments 42, 43 or 44 wherein the fourth nonaqueous liquid excipient is a polyethylene glycol (e.g., PEG300 or PEG 200), propylene glycol, benzyl benzoate or an alcohol (e.g., ethanol).
46. The product of any of embodiments 33-45 wherein the product has been stored at reduced temperature (about 4°C to about 8°C) or at ambient temperature for about 30 minutes to about 2 years.
65. A method comprising administering the composition or product of any of the foregoing embodiments to a subject suffering from a pathogen infection or a malignancy or an immune suppression or disregulation condition, e.g., a suppressed Th1 immune response or an unwanted Th2 immune response.
66. The method of embodiment 65 wherein the pathogen infection is a DNA virus infection or an RNA virus infection.
67. The method of embodiment 66 wherein the RNA virus infection is a retrovirus infection or a hepatitis virus infection.
68. The method of embodiment 67 wherein the retrovirus infection or hepatitis virus infection is an HIV, FIV, SIV, SHIV or hepatitis C virus infection.
69. The method of embodiment 65 wherein the pathogen infection is an intracellular parasite infection.
70. The method of embodiment 69 wherein the intracellular parasite infection is a malaria infection.

In other aspects, the invention provides dosing methods suitable to treat the conditions described herein. The following embodiments describe some of these methods.
1B. A method comprising intermittently administering BrEA hemihydrate (or a composition comprising BrEA hemihydrate) to a subject or delivering to the subject's tissues BrEA hemihydrate (or a composition comprising BrEA hemihydrate).
2B. The method of embodiment 1B wherein the subject has an infection, a hyperproliferation disorder, a hypoproliferation condition, an immunosuppression condition, an unwanted immune response or wherein the subject has recently experienced or will shortly experience trauma, surgery or a therapeutic treatment wherein the therapeutic treatment is one other than the method of embodiment 1B.
3B. The method of embodiment 2B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a bum, the presence of an immunosuppressive molecule, gastrointestinal irritation, or any combination of the foregoing.
4B. The method of embodiment 3B wherein the subject's immunosuppression condition is ameliorated or the unwanted immune response (e.g., a Th2 response) is reduced or wherein the subject's Th1 immune response is enhanced.
5B. The method of embodiment 3B wherein the subject's innate immunity, specific immunity or both is enhanced.
6B. The method of embodiment 5B wherein the subject's innate immunity is enhanced.
7B. The method of embodiment 6B wherein the subject's specific immunity is enhanced, e.g., wherein the subject's Th2 immune response is reduced or wherein the subject's Th1 immune reponse is enhanced.
8B. The method of embodiment 2B wherein the BrEA hemihydrate is administered according to the a dosing regimen comprising the steps,
   (a) administering BrEA hemihydrate to the subject at least once per day for at least 2 days;
   (b) not administering the BrEA hemihydrate to the subject for at least 1 day;
   (c) administering BrEA hemihydrate to the subject at least once per day for at least 2 days; and
   (d) optionally repeating steps (a), (b) and (c) at least once or variations of steps (a), (b) and (c) at least once.
9B. The method of embodiment 8B wherein step (c) comprises the same dosing regimen as step (a).
10B. The method of embodiment 9B wherein step (a) of the dosing regimen comprises administering BrEA hemihydrate once per day, twice per day, three times per day or four times per day.
11 B. The method of embodiment 10B wherein step (a) of the dosing regimen comprises administering BrEA hemihydrate compounds once per day or twice per day.
12B. The method of embodiment 10B wherein step (a) comprises administering BrEA hemihydrate for about 3 to about 24 days.
13B. The method of embodiment 12B wherein step (a) comprises administering BrEA hemihydrate for about 4 to about 12 days.
14B. The method of embodiment 13B wherein step (a) comprises administering BrEA hemihydrate compounds for about 4 to about 8 days.
15B. The method of embodiment 14B wherein step (b) comprises not administering BrEA hemihydrate for about 3 to about 120 days.
16B. The method of embodiment 15B wherein step (b) comprises not administering BrEA hemihydrate for about 4 to about 60 days.
17B. The method of embodiment 16B wherein step (b) comprises not administering BrEA hemihydrate for about 5 to about 30 days.
18B. The method of embodiment 16B wherein step (b) comprises not administering BrEA hemihydrate for about 8 to about 60 days.
19B. The method of embodiment 15B wherein steps (a), (b), and (c) are repeated at least about 4 times.
20B. The method of embodiment 15B wherein steps (a), (b), and (c) are repeated about 5 times to about 25 times.
21B. The method of embodiment 15B wherein steps (a), (b), and (c) and repetitions of steps (a), (b), and (c) occur over a time period of at least about 2 months.
22B. The method of embodiment 15B wherein steps (a), (b), and (c) and repetitions of steps (a), (b), and (c) occur over a time period of at least about 12 months.
23B. The method of embodiment 8B wherein step (b) comprises not administering the BrEA hemihydrate for about 3 to about 120 days.
24B. The method of embodiment 23B wherein step (b) comprises not administering BrEA hemihydrate for about 4 to about 60 days.
25B. The method of embodiment 24B wherein step (b) comprises not administering BrEA hemihydrate for about 5 to about 30 days.
26B. The method of embodiment 23B wherein step (b) comprises not administering BrEA hemihydrate for about 8 to about 60 days.
27B. The method of embodiment 8B wherein step (d) comprises repeating steps (a), (b), and (c) at least once.
28B. The method of embodiment 27B wherein step (d) comprises repeating steps (a), (b), and (c) about 3 times to about 25 times.
29B. The method of embodiment 1 B wherein steps (a), (b), and (c) and repetitions of steps (a), (b), and (c) occur over a time period of at least about 2 months.
30B. The method of embodiment 29B wherein steps (a), (b), and (c) and repetitions of steps (a), (b), and (c) occur over a time period of at least about 12 months.
31B. The method of any of embodiments 8B-30B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a bum, the presence of an immunosuppressive molecule, gastrointestinal irritation or any combination of the foregoing.
32B. The method of embodiment 31B wherein the subject's immunosuppression condition is ameliorated or the unwanted immune response is reduced.
33B. The method of embodiment 32B wherein the subject's innate immunity, specific immunity or both is enhanced.
34B. The method of embodiment 33B wherein the subject's innate immunity is enhanced.
35B. The method of embodiment 34B wherein the subject's specific immunity is enhanced.
36B. The method of embodiment 8B wherein step (c) comprises the a shorter dosing regimen than step (a).
37B. The method of embodiment 36B wherein step (a) comprises administering BrEA hemihydrate for 7 to about 24 days.
38B. The method of embodiment 37B wherein step (c) comprises administering BrEA hemihydrate for 4 to about 12 days.
39B. The method of embodiment 38B wherein step (b) comprises not administering BrEA hemihydrate for about 3 to about 120 days.
40B. The method of embodiment 39B wherein step (b) comprises not administering BrEA hemihydrate for about 4 to about 60 days.
41 B. The method of embodiment 40B wherein step (b) comprises not BrEA hemihydrate for about 5 to about 30 days.
42B. The method of embodiment 36B wherein step (d) comprises repeating steps (a), (b), and (c) at least once.
43B. The method of embodiment 42B wherein step (d) comprises repeating steps (a), (b), and (c) about 3 times to about 25 times.
44B. The method of embodiment 36B wherein steps (a), (b), and (c) and repetitions of steps (a), (b), and (c) occur over a time period of at least about 2 months.
45B. The method of embodiment 44B wherein steps (a), (b), and (c) and repetitions of steps (a), (b), and (c) occur over a time period of at least about 12 months.
46B. The method of any of embodiments 36B-45B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a bum, the presence of an immunosuppressive molecule, gastrointestinal irritation or any combination of the foregoing.
47B. The method of embodiment 46B wherein the subject's immunosuppression condition is ameliorated or the unwanted immune response is reduced.
48B. The method of embodiment 47B wherein the subject's innate immunity, specific immunity or both is enhanced.
49B. The method of embodiment 48B wherein the subject's innate immunity is enhanced.
50B. The method of embodiment 48B wherein the subject's specific immunity is enhanced.
51B. The method of embodiment 8B wherein step (c) comprises a longer dosing period than step (a).
52B. The method of embodiment 51B wherein step (a) comprises administering BrEA hemihydrate for 7 to about 24 days.
53B. The method of embodiment 52B wherein step (c) comprises administering BrEA hemihydrate for 4 to about 12 days.
54B. The method of embodiment 53B wherein step (b) comprises not administering BrEA hemihydrate for about 3 to about 120 days.
55B. The method of embodiment 54B wherein step (b) comprises not administering BrEA hemihydrate for about 4 to about 60 days.
56B. The method of embodiment 55B wherein step (b) comprises not administering BrEA hemihydrate for about 5 to about 30 days.
57B. The method of embodiment 51 B wherein step (d) comprises repeating steps (a), (b), and (c) at least once.
58B. The method of embodiment 57B wherein step (d) comprises repeating steps (a), (b), and (c) about 3 times to about 25 times.
59B. The method of embodiment 51 B wherein steps (a), (b), and (c) and repetitions of steps (a), (b), and (c) occur over a time period of at least about 2 months.
60B. The method of embodiment 59B wherein steps (a), (b), and (c) and repetitions of steps (a), (b), and (c) occur over a time period of at least about 12 months.
61B. The method of any of embodiments 51B-60B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a bum, the presence of an immunosuppressive molecule, gastrointestinal irritation or any combination of the foregoing.
62B. The method of embodiment 61 B wherein the subject's immunosuppression condition is ameliorated or the unwanted immune response is reduced.
63B. The method of embodiment 62B wherein the subject's innate immunity, specific immunity or both is enhanced or wherein the subject's Th1 immune reponse is enhanced or the subject's Th2 immune response is decreased.
64B. The method of embodiment 8B wherein the variations of steps (a), (b) and (c) comprise conducting a first dosing regimen of steps (a), (b) and (c) once, twice or three times, followed by one or more second dosing regimens of steps (a'), (b') and (c') wherein one or more of the (a'), (b') and (c') steps in the second dosing regimen is longer than the corresponding step in the first dosing regimen.
65B. The method of embodiment 8B wherein the variations of steps (a), (b) and (c) comprise conducting a first dosing regimen of steps (a), (b) and (c) once, twice or three times, followed by one or more second dosing regimens of steps (a'), (b') and (c') wherein one or more of the (a'), (b') and (c') steps in the second dosing regimen is shorter than the corresponding step in the first dosing regimen.
66B. The method of any of embodiments 1B-67B wherein BrEA hemihydrate is administered orally, intramuscularly, intravenously, subcutaneously, topically, vaginally, rectally, intracranially, intrathecally, intradermally, as an aerosol or by a buccal route.
67B. The method of embodiment 66B wherein BrEA hemihydrate is present in a solid formulation predominantly as a solid or BrEA hemihydrate is present in a liquid formulation predominantly as a solvate, colloid or a suspension or BrEA hemihydrate is present in a gel, cream or paste.
68B. The method of any of embodiments 2B-67B wherein the subject's viral infection, intracellular bacterial infection, extracellular bacterial infection, fungal infection, yeast infection, extracellular parasite infection, intracellular parasite infection, protozoan parasite, multicellular parasite, autoimmune disease, cancer, precancer, chemotherapy, radiation therapy, immunosuppressive therapy, anti-infective agent therapy, a wound, a bum, or the presence of an immunosuppressive molecule, gastrointestinal irritation or any combination of the foregoing is (a) a DNA virus infection or an RNA virus infection (HSV, CMV, HBV, HCV, HIV, SHIV, SIV); (b) a mycoplasma infection, a *Lisferia* infection or a *Mycobacterium* infection; (c) extracellular bacteria infection; (d) fungal infection; (e) a yeast infection *(Candida, Cryptococcus*); (d) protozoa (malaria, leishmania, cryptosporidium, toxoplasmosis, pneumocystis); (e) a multicellular parasite; (f) autoimmune diseases (SLE, RA, diabetes); (g) cancers (solid cancers selected from, e.g., ovarian, breast, prostate, glioma; disseminated cancers selected from lymphoma, leukemia, colon cancer, sarcoma); (h) precancers; (i) chemotherapies (adriamycin, cisplatin, mitomycin C); (j) radiation therapies; (k) immunosuppressive therapies; (I) anti-infective agent therapies; (m) wounds (surgical or otherwise); (n) 1^{st} degree, 2^{nd} degree or 3^{rd} degree bums; (o) immunosuppressive molecules; (p) gastrointestinal irritation (irritable bowel, Crohn's disease, chronic diarrhea); or (q) any combination of (a) through (p).
69B. The method of embodiment 68B wherein the RNA virus infection is a retroviral infection or a hepatitis virus infection.
71B. The method wherein BrEA hemihydrate is in a composition that comprises, (a) one or more nonaqueous liquid excipients, wherein the composition comprises less than about 3% v/v water; (b) a solid that comprises a pharmaceutically acceptable excipient; or (c) one or more liquid excipients, wherein the composition comprises more than about 3% v/v water.
75B. A method to treat involuntary weight loss, oral lesions, skin lesions, opportunistic infections, diarrhea or fatigue in an subject comprising intermittently administering BrEA hemihydrate to the subject (e.g., involuntary weight loss from viral infection, gastrointestinal infection, chemotherapy, anorexia).
76B. The method of embodiment 75B wherein the subject has an immunosuppression condition.
77B. The method of embodiment 76B wherein the subject is a human.
78B. The method of embodiment 77B wherein the subject is a human 1 day to 18 years old (e.g., 1 month to 6 years old).
79B. The method of any of embodiments 75B-78B wherein the subject's specific immunity remains impaired compared to a typical comparable control subject who does not have the subject's pathological condition.
80B. The method of embodiment 79B wherein the subject's CD4 cell count does not increase significantly during one or more courses of dosing (e.g., dosing for 1 week to about 2 weeks or more).
81B. The method of clam 80B wherein the subject's CD4 cell count is about 20 to about 100 CD4⁺ cells/mm³ or about 20 to about 75 CD4⁺ cells/mm³.
82B. The method of any of embodiments 1B-81B wherein the subject has a pathogen(s) infection or a malignancy and the pathogen(s) or malignancy does not become resistant to BrEA hemihydrate over a time normally associated with the development of measurable resistance in at least about 50% of subjects who are treated with a therapeutic treatment(s) other than BrEA hemihydrate.
83B. The method of embodiment 82B wherein the pathogen infection is an HIV, SIV, SHIV or HCV infection.

In other embodiments, the invention provides methods to modulate immune cells or immune responses in a subject. The following numbered embodiments describe some of these methods.
1C. A method to modulate a subject's innate immunity or to enhance a subject's Th1 immune response or to reduce a subject's Th2 immune responses comprising administering to the subject a BrEA hemihydrate.
2C. The method of embodiment 1C wherein the subject's innate immunity is enhanced.
3C. The method of embodiment 1C or 2C wherein the subject suffers from an innate immunity suppression condition, a suppressed Th1 immune response or an unwanted Th2 immune response.
4C. The method of embodiment 3C wherein the innate immunity suppression condition, the suppressed Th1 immune response or the unwanted Th2 response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a bum, the presence of an immunosuppressive molecule or any combination of the foregoing.
5C. The method of any of embodiments 1 C-3C wherein the subject's Th1 immune response is enhanced.
6C. The method of embodiment 1C wherein the subject's Th2 immune response is reduced.
7C. The method of embodiment 6C wherein the subject has a condition comprising an unwanted immune response (e.g., autoimmune disease, SLE, diabetes).
9C. The method of embodiment 6C or 7C wherein the subject is a vertebrate, a mammal, a primate or a human.
10C. The method of embodiment 9 wherein the vertebrate's, the mammal's the primate's or the human's specific immunity modulation is (i) an enhanced CTL or Th1 response to a virus infection or to a malignant cell *in vitro* or *in vivo*, (ii) enhanced antigen presentation or biological activity by dendritic cells or dendritic cell precursors, or (iii) enhanced killing of virus-infected or of malignant cells.
11C. The method of 10C wherein the vertebrate is a human, the virus infection is an HIV infection and the CTL or Th1 response comprises an enhanced response to one or more of the HIV's gag protein or to the HIV's gp120.
12C. The method of embodiment 1C, 4C, 10C or 11C wherein the subject's Th1 cells, tumor-infiltrating lymphocytes (TIL cells), NK cells, peripheral blood lymphocytes, phagocytes, monocytes, macrophage, neutrophils, eosinophils, dendritic cells or fibrocytes are activated as measured by, e.g., enhanced ³H-thymidine uptake compared to untreated controls or by an increase in the number of the cell type in circulation or demonstrable movement of the cell type from one tissue or compartment (e.g., skin) to another tissue or compartment (e.g., blood, lymph node, spleen or thymus).
13C. The method of embodiment 1C, 4C, 10C, 11C or 12C, wherein BrEA hemihydrate modulates transcription of one or more genes in the subject's NK cells, phagocytes, monocytes, macrophages, neutrophils, eosinophils, dendritic cells or fibrocytes are activated (e.g., as measured by increased protein kinase C activity or by modulation of a biological activity of a steroid receptor or an orphan nuclear hormone receptor).
14C. The method of embodiment 1 C wherein the BrEA hemihydrate enhances lysosome movement in one or more of the subject's NK cells, phagocytes, monocytes, macrophages, neutrophils, eosinophils, dendritic cells or fibrocytes.
15C. The method of embodiment 1C wherein BrEA hemihydrate enhances protein kinase C activity in one or more of the subject's NK cells, phagocytes, monocytes, macrophages, neutrophils, eosinophils, dendritic cells or fibrocytes (e.g., PKCα, PKCβ, PKCγ and PKCζ).
18C. The method of any of embodiments 1C-17C wherein the subject has an infection, a hyperproliferation disorder, a hypoproliferation condition, an immunosuppression condition, an unwanted immune response or wherein the subject has recently experienced or will shortly experience trauma, surgery or a therapeutic treatment wherein the therapeutic treatment is one other than the method of embodiment 1C.
19C. The method of embodiment 18C wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a bum, the presence of an immunosuppressive molecule, gastrointestinal irritation, or any combination of the foregoing.
20C. The method of embodiment 19C wherein the subject's immunosuppression condition is ameliorated or the unwanted immune response is reduced.
21C. The method of embodiment 19C wherein the subject's immunosuppression condition is associated with a viral infection.
22C. The method of embodiment 21C wherein the viral infection comprises a DNA virus or an RNA virus infection.
23C. The method of embodiment 22C wherein the RNA virus infection comprises a retroviral infection or a hepatitis virus infection.
24C. The method of any of embodiments 18C-23C wherein the subject suffers from one or more of chronic diarrhea, involuntary weight loss (usually at least about 5% or more), cachexia (usually at least about 5% or more), muscle wasting, one or more oral lesions (usually at least about 1 cm²), one or more genital lesions (usually at least about 1 cm²), skin lesions (usually at least about 1 cm²) or an opportunistic infection associated with AIDS.
25C. A method (e.g., to determine a biological activity of BrEA hemihydrate or to modulate transcription of a gene in a cell or cell-free transcription system) comprising: (a) contacting the BrEA hemihydrate with a cell or cell population *in vitro* or *in vivo;* (b) measuring one or more of (i) a complex between a binding partner and BrEA hemihydrate, (ii) proliferation of the cell or cell population, (iii) differentiation of the cell or cell population (iv) an activity of a protein kinase C, (v) a level of phosphorylation of a protein kinase C substrate, (vi) transcription of one or more target genes, (vii) enhancement or inhibition of the cellular response to steroids, e.g., glucocorticoids, (viii) inhibition of steroid-induced transcription, e.g., glucocorticoids, sex steroids, (ix) inhibition of retrovirus (e.g., HIV, SIV, FIV or SHIV) LTR-driven transcription, or (x) modulation of the numbers of an immune cell population in circulation *in vivo* (e.g., circulating peripheral blood lymphocytes in a mammal such as a primate or a human); and (c) optionally comparing the result obtained in step (b) with an appropriate control.
26C. The method of embodiment 25C wherein the binding partner is a steroid receptor, a transcription factor or a steroid hormone superfamily orphan receptor.
27C. The method of embodiment 25C wherein the biological activity determined is a modulating activity of BrEA hemihydrate for replication or cytopathic effects associated with a retrovirus, a hepatitis virus or a protozoan parasite.
28C. The method of embodiment 25C wherein the biological activity determined is a modulating activity of BrEA hemihydrate for replication, cytopathic effects associated with the retrovirus, the hepatitis virus or the protozoan parasite or the biological activity determined is metabolism (assay by ³H-thymidine uptake) of a cell or cell population comprising NK cells, phagocytes, monocytes, macrophages, basophils, eosinophils, fibrocytes, transformed cells, virus-infected cells, bacteria-infected cells or parasite-infected cells.
29C. The method of embodiment 25C wherein the target gene is a virus gene, a bacterial gene, a parasite gene, a gene associated with cancer.
30C. The method of embodiment 29C wherein the virus gene is a polymerase gene, a reverse transcriptase gene, an envelope gene, a protease gene or a gene associated with viral nucleic acid replication or a viral structural gene.
31C. The method of embodiment 30C wherein the polymerase gene encodes a DNA polymerase or encodes an RNA polymerase.
32C. The method of embodiment 30C wherein the reverse transcriptase gene encodes a human, primate, avian or feline retrovirus reverse transcriptase.
33C. A method comprising administering BrEA hemihydrate to a human or primate who has a retroviral infection and a CD4 count of 550 or less.
34C. The method of embodiment 33C wherein the human has a CD4 count of about 20 to about 100 or about 20 to about 80.
35C. The method of embodiment 33C wherein the human has a CD4 count of about 30 to about 150.
36C. The method of embodiment 33C wherein the human has a CD4 count of about 500 or less, about 450 or less, about 400 or less, about 350 or less, about 300 or less, about 250 or less, about 200 or less, about 150 or less, about 100 or less, about 50 or less or about 25 or less or about 20 or less.
37C. The method of any of embodiments 33C-36C wherein BrEA hemihydrate is present in a composition that comprises one or more nonaqueous liquid excipients and less than about 3% v/v water or any of the formulations as disclosed in the specification or any of the numbered embodiments above.
38C. The method of any of embodiments 33C-37C wherein the BrEA hemihydrate is administered according to an intermittent dosing protocol as disclosed in the specification or any of the numbered embodiments above.
39C. The method of any of embodiments 30C-45C wherein the human is coinfected with hepatitis C virus, hepatitis B virus, HSV-1, HSV-2, a malaria parasite, a *Pneumocystis* parasite, or a *Cryptosporidium* parasite.
40C. The method of embodiment 46C wherein level of the HCV is reduced in the human.
41 C. A method comprising administering BrEA hemihydrate to a subject, or to a nervous system cell(s) in tissue culture whereby BrEA hemihydrate binds to a receptor associated with a cell(s) in the nervous system and (1) elicits a biological response in the cell(s) in the nervous system or in the cell(s) in tissue culture and/or (2) elicits a neuronal response that is transmitted to a distant site(s) or cell(s) where the method optionally is used to screen a formula 1 compound(s), in particular BrEA hemihydrate, for its biological activity, to treat a pathological condition (e.g., pathogen infection such as a virus (HIV), a malignancy or a neurological disorder, e.g., AIDS associated dementia, Alzheimer's, Parkinson's, Multiple Sclerosis) in the subject or to determine the bioavailability or metabolism of BrEA hemihydrate to the subject or the cell(s) in the nervous system or in tissue culture, wherein metabolism is optionally determined by comparing the biological effect of a formula 1 compound(s) with a control compound, which can be a different formula 1 compound.
42C. The method of embodiment 41 wherein the receptor associated with the cell in the nervous system is a neurotransmitter receptor(s) (e.g., a γ-aminobutyric acid receptor such as type A, a NMDA receptor) and/or a steroid receptor (e.g., androgen receptor, estrogen receptor).
43C. The method of embodiment 41C or 42C wherein the cell(s) in the nervous system is a neuron(s), and astrocyte(s) and/or a glial cell(s).
44C. The method of embodiment 41C, 42C or 43C wherein the biological response in the cell(s) in the nervous system or in the cell(s) in tissue culture is increased or decreased transcription of a gene(s) (e.g., a neurotransmitter, vasopressin, a heat shock protein), increased or decreased secretion of a protein(s) (e.g., vasopressin), reduced damage from oxidative stress, enhanced nitric oxide release and/or enhanced neurite growth.
45C. The method of any of embodiments 1C-44C wherein the compound(s) of formula 1 is BrEA hemihydrate.
46C. A method to (a) modulate the expression of at least one immune cell antigen by an immune cell in a subject, wherein the immune cell antigen is selected from CD3, CD11c, CD14, CD16, CD19, CD25, CD38, CD56, CD62L, CD69, CD45RA, CD45RO, CD123, HLA-DR, IL-1, IL-2, IL-4, IL-6, IL-8, IL-10, TNFα, IGF₁ and γIFN, or (b) activate CD8⁺ T cells or CD8⁻ T cells in a subject, wherein the activation comprises at least transiently enhanced expression of CD25 or CD69 by the T cells, or (c) increase the proportion of CD8⁺ or CD8⁻ lymphokine activated killer cells in a subject's CD16+ cells (e.g., CD8⁺, CD16⁺, CD38⁺ or cells CD8⁻, CD16⁺, CD38⁺), or (d) increase the proportion of (i) CD8⁻, CD16⁺ natural killer cells, (ii) CD8⁺, CD16⁺ natural killer cells or (iii) CD8⁻, CD16⁺ cells that mediate antibody-dependent cell-mediated cytotoxicity, or (iv) CD8⁺, CD16⁺ cells that mediate antibody-dependent cell-mediated cytotoxicity, or (e) increase the proportion of dendritic cell precursors in a subject's circulating white blood cells (e.g., Lin⁻, HLA-DR⁺, CD123⁺ or Lin⁻ HLA-DR⁺, CD11c⁺ cells) or (f) increase the proportion of CD45RA⁺ T cells or CD45⁺, RO⁺ T cells in a subject's circulating white blood cells, or (g) change (increase or decrease) the proportion or relative numbers of CD62L⁺ T cells in a subject's circulating white blood cells, or (h) increase the proportion of CD8⁺ or CD4⁺ T cells that express CD62L in a subject's circulating CD8⁺ or CD4⁺ T cells, or (i) decrease the proportion of CD8⁺ or CD4⁺ T cells that express CD62L in a subject's circulating CD8⁺ or CD4⁺ T cells, or (j) increase the proportion of HLA-DR⁺, CD8⁺, CD38⁺ cells in a subject's circulating white blood cells, or (k) decrease the level of IL-4 or IL-10 that is expressed by or present in a subject's white blood cells or in a subject's plasma (or that is expressed after the subject's white cells are stimulated *in vitro),* (I) at least transiently increase the number of dendritic cell precursors or dendritic cells that are present in a subject's white blood cells or in a subject's plasma, or (m) enhance the capacity of CD4⁺ T cells to express IL-2, IL-12 or γIFN, the method comprising administering to the subject BrEA hemihydrate and a pharmaceutically acceptable excipient.
48C. The method of embodiment 46C or 47C wherein BrEA hemihydrate is administered to the subject daily over a period from one to about 15 days, e.g., for 1, 2, 3, 4, 5, 6, 7, 8, 9, 10, 11, 12, 13, 14, 15 or more days.
49C. The method of embodiment 48C wherein the expression of the immune cell antigen is detectably modulated for at least about 4-7 days after the last administration of BrEA hemihydrate to the subject, e.g., for at least 4, 5, 6, 7 or more days.
50C. The method of embodiment 48C or 49C wherein the expression of the immune cell antigen is detectable at least about 8-90 days after the last administration of BrEA hemihydrate, e.g., for at least about 8, 10, 12, 15, 20, 25, 28, 30, 35, 40, 42, 45, 49, 50, 55, 56, 60, 63, 65, 70, 75, 77, 80, 84, 85, 90, 91 95, 98, 100 or more days.
51C. The method of any of embodiments 46C-51C wherein the subject has an immunosuppression condition, a pathogen infection or a conditions associated with a deficient Th1 immune reponse or an excessive Th2 immune response.
52C. The method of embodiment 51 C wherein the pathogen infection is a viral infection, a bacterial infection, a yeast infection, a fungal infection or a viroid infection, e.g., wherein the pathogen infection is a viral infection such as a DNA virus infection or an RNA virus infection (e.g., an infection caused by a Hepadnavirus, a Parvovirus, a Papovavirus, an Adenovirus, a Herpesvirus, Retrovirus, a Flavivirus, a Togavirus, a Rhabdovirus, a Picomavirus, a Bunyavirus, a Reovirus, an Orthomyxovirus or a Paramyxovirus, such as a HIV1, HIV2, SIV, SHIV or another virus described herein or in the cited references).
53C. The method of embodiment 52C wherein the subject has an immunosuppression condition that is associated with or caused by a pathogen infection.
54C. The method of any of embodiments 46C-53C wherein the subject is a mammal, a human, a primate or a rodent.
55C. The method of any of embodiments 46C-54C wherein about 0.05 mg/kg/day to about 20 mg/kg/day is administered parenterally (e.g., by intravenous, subcutaneous, intramuscular, or intramedullary injection), topically, orally, sublingually or bucally to the subject, e.g., about 0.1 mg/kg/day, about 0.2 mg/kg/day, about 0.5 mg/kg/day, about 1.0 mg/kg/day, about 1.5 mg/kg/day, about 2 mg/kg/day, about 2.5 mg/kg/day, about 3.0 mg/kg/day, about 4 mg/kg/day or about 6 mg/kg/day, i.e., about 0.1-10 mg/kg/day, typically about 0.2-7 mg/kg/day.
56C. The method of embodiment 55C wherein the subject is concurrently taking one or more second therapeutic agents to treat a pathogen infection, e.g., a viral infection, such as a HIV-1 infection, a HIV-2 infection, a HAV infection, a HBV infection, a HCV infection, an Epstein Barr virus infection, a HSV-1 infection, a HSV-2 infection, human herpesvirus 6 infection, human herpesvirus 7 infection, human herpesvirus 8 infection, or a bacterial infection or a parasite infection, such as a malaria infection, leishmaniasis, cryptosporidiosis, toxoplasmosis, a mycoplasma infection, a *Trichomonas* infection, a *Chlamidya* infection, a *Pneumocystis* infection, a *Salmonella* infection, a *Listeria* infection, an *Escherichia coli* infection, a *Yersinia* infection, a *Vibrio* infection, a *Pseudomonas* infection, a *Mycobacterium* infection, a *Haemophilus* infection, a *Neisseria* infection, a *Staphylococcus* infection or a *Streptococcus* infection.
57C. The method of embodiment 56C wherein the one or more second therapeutic agents is a protease inhibitor, a reverse transcriptase inhibitor, a viral, bacterial or parasite DNA or RNA polymerase inhibitor, an antibacterial antibiotic or an antifungal agent, such as AZT, ddl, ddC, D4T, 3TC, a viral (e.g., HIV) fusion inhibitor, hydroxyurea, nelfinavir, saquinavir, ritonavir, indinavir, chloroquine, a chloroquine analog, amphotericin B, fluconazole, clotrimazole, isoniazid, dapsone, rifampin, cycloserine, erythromycin, a tetracycline antibiotic, vancomycin, ethambutol, pyrazinamide, a flurorquinolone (e.g., ciprofloxacin, norfloxacin), a cephalosporin antibiotic, a β-lactam antibiotic or an aminoglycoside antoibiotic (e.g., streptomycin, kanamycin, tobramycin).
58C. The method of any of embodiments 46C-57C wherein the subject is a human, a primate, a canine, a feline or a rodent.
59C. A composition comprising an effective amount of an immune cell subset modulatory compound of formula 1, in particular BrEA hemihydrate and a pharmaceutically acceptable carrier.
60C. The composition of embodiment 59C wherein the immune cell subset is (1) CD8⁺ T cells, (2) CD4⁺ T cells, (3) CD8⁺ lymphokine activated killer cells, (4) CD8⁻ lymphokine activated killer cells, (5) CD8⁻, CD16⁺ natural killer cells, (6) CD8⁺, CD16⁺ natural killer cells, (7) CD8⁻, CD16⁺ cells that mediate antibody-dependent cell-mediated cytotoxicity, (8) CD8⁺, CD16⁺ cells that mediate antibody-dependent cell-mediated cytotoxicity, (9) dendritic cells or dendritic cell precursors, (10) CD45RA⁺ T cells, (11) CD45RO⁺ T cells, (12) CD45RA⁺, CD45RO⁺ T cells, (13) CD8⁺, CD62L T cells, (11) CD4⁺, CD62L⁺ T cells or (14) HLA-DR⁺, CD8⁺, CD38⁺ T cells.
61C. A method to detect a biological response associated with the administration of BrEA hemihydrate to a subject comprising (1) obtaining a sample from the subject, (2) administering the BrEA hemihydrate to the subject to obtain a treated subject (3) obtaining a second sample from the treated subject, (4) within 24 hours of obtaining the sample, analyzing the sample to obtain control information for detecting the biological response, (5) within 24 hours of obtaining the second sample, analyzing the second sample for the presence or absence of a biological response to obtain experimental information and (6) optionally comparing the control information with the experimental information to detect the presence, absence, relative magnitude or absolute magnitude of the biological response
62C. The method of embodiment 61C wherein the BrEA hemihydrate further comprises a pharmaceutically acceptable carrier.
63C. The method of embodiment 61C or 62C wherein the biological response associated with the administration of BrEA hemihydrate to the subject is modulation of the expression of a cell surface antigen, an increased absolute or relative number of cells in an immune cell subset, a decreased absolute or relative number of cells in an immune cell subset or an unchanged absolute or relative number of cells in an immune cell subset.
64C. The method of embodiment 63C wherein the immune cell subset is CD8⁺ T cells, CD4⁺ T cells, CD8⁺ lymphokine activated killer cells, CD8⁻, CD16⁺ natural killer cells, circulating dendritic cell precursors, circulating dendritic cells, tissue dendritic cell precursors, tissue dendritic cells, CD8⁺ lymphokine activated killer cells, CD8⁻ lymphokine activated killer cells, CD8⁻, CD16⁺ natural killer cells, CD8⁺, CD16⁺ natural killer cells, CD8⁻, CD16⁺ cells that mediate antibody-dependent cell-mediated cytotoxicity, CD8⁺, CD16⁺ cells that mediate antibody-dependent cell-mediated cytotoxicity, CD45RA⁺ T cells, CD45RA⁺, CD45RO⁺ T cells, CD45RO⁺ T cells, CD8⁺, CD62L T cells, CD4⁺, CD62L⁺ T cells or HLA-DR⁺, CD8⁺, CD38⁺ T cells, monocytes or macrophages.
65C. The method of embodiment 64C wherein the biological response is at least transient modulation of an immune cell antigen or an immune accessory cell antigen (e.g., an adhesion molecule at the surface of endothelial cells or a cytokine receptor at the surface of T cells or B cells).
66C. The method of embodiment 65C wherein the immune cell antigen is a protein, glycoprotein or cell surface antigen usually or only expressed by lymphoid cells (lymphocytes or white blood cells or their precursors, e.g., T cells, B cells, monocytes, macrophage, LAK cells, NK cells, dendritic cells).
67C. The method of embodiment 65C wherein the immune cell antigen is a CD molecule, an interleukin or a cytokine, optionally selected from CD16, CD25, CD38, CD62L, CD69, CD45RA, CD45RO, IL-1, IL-2, IL-4, IL-6, IL-8, IL-10, TNFα, IGF₁ and γIFN.
68C. The method of any of embodiments 61C-67C wherein the subject is a human, a primate, a canine, a feline or a rodent.
69C. A method to alter the Th1-Th2 balance in a subject comprising administering an effective amount of BrEA hemihydrate to a subject whereby the subject's expression or secretion of IL-4 or IL-10 is detectably modulated.
70C. The method of embodiment 30 wherein the subject's expression or secretion of IL-4 or IL-10 is decreased and the Th1-Th2 balance in the subject's Th1 immune responses to an infection or immunosuppression condition is detectably enhanced.

Examples. The following examples further illustrate the invention and they are not intended to limit it in any way.

**Example 1. BrEA formulation.** Two lots of a non-aqueous BrEA formulation were made at a BrEA concentration of 50 mg/mL in 25% polyethylene glycol 300, 12.5% dehydrated ethyl alcohol, 5% benzyl benzoate, and 57.5% propylene glycol as follows. BrEA was obtained from Procyte, Inc. The remaining excipients are shown below.

| **Excipient** | **Specification** | **Supplier Lot No.** | **Final Product Concentration** |
|---|---|---|---|
| Propylene glycol | USP | Arco Chemical HOC-61220-01104 | 57.5% (v:v) |
| | | | |
| Polyethylene glycol 300 | NF | Union Carbide 695752 | 25% (v:v) |
| | | | |
| Dehydrated alcohol | USP | McCormick Distilling 97K10 | 12.5% (v:v) |
| | | | |
| Benzyl benzoate | USP | Spectrum Pharmaceuticals MG025 | 5% (v:v) |

The formulation was prepared by suspending BrEA in polyethylene glycol 300, and sequentially adding propylene glycol, benzyl benzoate, and dehydrated ethyl alcohol to form a solution, which was diluted to the final desired volume with additional propylene glycol. The procedure is described below.

The calculated amount of polyethylene glycol 300 was added to a compounding vessel. Then , while mixing, the calculated amount of BrEA was added to the vessel, and mixed for at least 5 minutes to form a smooth, creamy liquid propylene glycol was added to the vessel, and mixed for a minimum of 5 minutes to form a uniform suspension. The calculated amount of benzyl benzoate is added to the vessel, and mixed for approximately 5 minutes to form a translucent liquid suspension. Dehydrated alcohol was added to the vessel, and mixed for approximately 5 minutes to form a clear, colorless solution. Propylene glycol was then added to achieve the desired final formulation, and mixed for approximately 5 minutes. The drug solution was transferred to a volume dispensing device set to deliver 1.2 mL per vial. Under nitrogen pressure, the solution was filtered through two 0.2 µm polyvinylidene fluoride filters in series into 2 cc amber glass vials. The vials were capped with Teflon-coated, butyl-rubber stoppers and crimp sealed. Materials used in the product vials are listed below.

| **Material** | **Source** | **Product Code** | **Description** |
|---|---|---|---|
| Vial | Wheaton | 2702-B51BA | Tubing vial, 2 mL/13 mm, glass, type 1 amber |
| Stopper | Omniflex | V9239 FM257/2 | 13 mm, Teflon coated, butyl rubber stopper |
| Seal | West | 4107 | Flip seal, 13 mm, mist gray bridge |

Product specifications were examined by one or more of the following assays.

| **Test** | **Specification** | **Method** |
|---|---|---|
| Physical Examination | Clear colorless solution with slight alcoholic odor | |
| Volume recovery | NLT* 1.0 mL | USP23<1> |
| Specific gravity | TBD | USP23<841> |
| Assay for active component | 90-110% of label | HPLC |
| Sterility | sterile | USP23<71> |
| Endotoxin | <0.1 EU/mg | USP23<85> |
| Particulate matter | ≥ 10 µm NMT** 6000/cnt ≥ 25 µm NMT 600/cnt | USP23<788> |

| | | |
|---|---|---|
| * NLT - no less than | | |
| **NMT - no more than | | |

| **Lot Analysis** | | | |
|---|---|---|---|
| **Test** | **Specification** | **Lot 1** | **Lot 2** |
| Physical Examination | Clear colorless solution with slight alcoholic odor | Positive | Positive |
| Volume recovery | NLT 1.0 mL | 1.15 mL | -- |
| Specific gravity | TBD | 1.0411 | -- |
| Assay for active component | 90-110% of label | 103.10 % | 104.25% |

| | | | |
|---|---|---|---|
| Sterility | sterile | sterile | -- |
| Endotoxin | <0.1 EU/mg | 0.024 EU/mg | -- |
| Particulate matter | ≥ 10 µm NMT 6000/cnt | 26 | -- |
| | ≥ 25 µm NMT 600/cnt | 15 | |

**Example 2. BrEA drug substance and BrEA formulation stability.** An accelerated stability study of 6 months duration is conducted using BrEA and the formulations from example 1. Samples are taken at 1, 2, 3, 4, 5, and 6 month time points and compared with the specifications listed in example 1. Real time stability (25° C, 60% relative humidity) is conducted using BrEA formulation Lots 1 and 2, with sampling time points at 3, 6, 9, 12, 18, 24, and 36 months. After 3 months of storage at 40° C and 75% relative humidity, the assay potency of BrEA is at least 95% of the label value. The results from the stability testing indicate that BrEA is stable for at least 3 months at elevated temperature and humidity in the Lot 1 and 2 formulations.

**Example 3. Primate intermittent dosing protocol.** Pig-tail Macaque monkeys infected with the SHIV₂₂₉ retrovirus were treated with a BrEA formulation as described in example 1. SHIV₂₂₉ is a recombinant retrovirus containing HIV and SIV sequences. J. Thompson et al., abstract #75, 16^{th} *Annual Symposium on Nonhuman Primate Models for AIDS*, October 7-10, 1998, Atlanta, GA, M. Agy et al., abstract #67, 16^{th} *Annual Symposium on Nonhuman Primate Models for AIDS*, October 7-10, 1998, Atlanta, GA. In monkeys, it establishes an aggressive infection that leads to severe symptoms of end-stage disease in infected untreated animals at about 180-210 days after infection. Four pig-tail macaques (2/group) received subcutaneous injections of the formulation at 1 or 2 mg/kg body weight for 10 consecutive days (Protocol 1). On week 8, 3 of the 4 monkeys were retreated and 2 treatment naive monkeys were treated with 5 mg/kg of the formulation on an every other day basis for a period of 20 days (Protocol 2). On week 19, all primates receiving treatment began a 3 course treatment regimen with 3 mg/kg the BrEA formulation once daily for 10 consecutive days, repeated every four weeks for a total of 3 treatment courses (Protocol 3).

The animals were infected with 1-100 TCID₅₀ units administered intravenously or intrarectally. Viral titers in the first group of animals ranged from 10⁶ to 10⁸ before dosing began. All animals demonstrated an initial rise in plasma viral SHIV RNA. After a period of 2 to 3 weeks, titers began to decline and 3 of the 4 animals showed a response to therapy with average viral titers of 0.76 log below baseline at weeks 4 to 5 after initiation of treatment. By week 8, titers in all animals had retumed to baseline values. Blood glucose levels dropped significantly, alkaline phosphatase levels were elevated and SGOT/GGT values trended towards the high end of normal. No other significant changes were observed in any of the parameters monitored. The CD4 levels in all monkeys remained less than 100 cells/mm³ at the end of the first protocol.

Three of the five monkeys on the second regimen (Protocol 2) responded to the BrEA therapy with a greater depth and duration of response than observed at the lower dose levels. In the responding animals, the average decline below baseline was 1.47 log. The non-responding animal from Protocol 1 responded when administered the BrEA formulation in Protocol 2. Two animals did not respond, one each from the treatment experienced and treatment naive groups. The third regimen (Protocol 3) is ongoing and animals are being monitored.

The monkeys on this study were salvaged from an infectivity study and the first cohort of four monkeys on study (Protocol 1) were expected to live only a few weeks past the initiation of these experiments as they were beginning to deteriorate due to disease related causes. One animal died at day 356 from a toxic reaction to the anesthetic used during acquisition of a blood sample for analysis. At the time of this application, the remaining monkeys are receiving multiple rounds of therapy appear to be in good clinical health. Their survival was greater than 380 days from the time of infection. Treatment by intermittent dosing of the BrEA formulation was used. Three control monkeys were infected with 1-10,000 SHIV₂₂₉ TCID₅₀ units and did not receive treatment These animals are considered the no treatment arm of a survival study. The mean time to death for pig-tailed macaques infected with SHIV₂₂₉ was 193 days. Monkeys receiving therapy remained in good clinical health for over 350 days with CD4 levels less than 20 cells/mm³ and without opportunistic infections or disease-related symptoms, other than a mild anemia in one animal.

These results show completely unexpected therapeutic responses by the primates infected with the SHIV retrovirus, which is quite virulent. The results show that the majority of subjects in these treatment protocols not only had significantly prolonged survival compared to untreated controls, but also the clinical symptoms associated with retroviral infection improved dramatically, despite the fact that CD4 counts remained low, i.e., less than about 100 CD4 cells/mm³ initially and less than about 20 CD4 cells/mm³ later in the treatment protocols. To date, results such as this, i.e., (1) good clinical health in a majority of subjects having low CD4 levels (less than about 150 cells/mm³, especially less than about 75 cells/mm³) and (2) no clinical sign of viral resistance to treatment despite intermittent dosing over a prolonged time period, are unprecedented in primates, humans or any other animal. The SHIV₂₂₉ model is extremely pathogenic in pig-tailed macaques. Events that occur in this model over several weeks would typically take several years in humans infected with HIV. Treatment of monkeys infected with this virus and treated with commonly used antiretrovirals, e.g., AZT, 3TC or a protease inhibitor, are not expected to significantly affect the course of disease progression. The clinical condition of the animals continues to improve, e.g., weight gain is about 8-15% per animal. These results show that the treatment using the intermittent dosing protocol is highly effective despite the apparent impairment of the subject's specific immunity, as shown by the low CD4 counts. Increased CD4 counts may be attained using immune stimulators such as IL2 or they may increase spontaneously in some subjects such as humans, depending on the treatment protocol, the duration of dosing or the subject's initial medical condition. The antiviral effects shown here appear to function at least in part by enhancing the subject's immune responses, e.g., enhanced immune response by phagocytic cells (NK cells, monocytes and/or macrophages), and/or enhancing any residual specific immune responses, if any, that the subject may be able to muster.

**Example 4. Human treatment protocol.** A dose escalation clinical trial is performed using a nonaqueous formulation containing BrEA or another formula 1 compound(s) that is prepared essentially as described in example 1. The patients are treatment naïve or treatment experienced and about 3-10 patients are examined at each dose level. The initial dose is 25 mg of BrEA or another formula 1 compound(s) that is administered parenterally , e.g., s.c. or i.m. The dose is administered once or once or twice per day for 1-12 days, followed by no dosing for at least 7 days (e.g., 7 to 90 days). Subsequent doses are administered once or once per day for 1-12 days, followed by no dosing for at least 7 days (e.g., 7 to 90 days). Other dose levels tested are 20 mg, 50 mg, 75 mg, 100 mg, 150 mg, 200 mg, 250 mg and 300 mg with each dose administered once per day as a single dose or as two, three or more subdivided doses. An efficacy dosing trial is performed using the same dosing protocol as the dose escalation trial or it may alternatively comprise dosing once or twice every other day for 3-17 days, followed by no dosing for 7-90 days and then repeating the dosing once or twice every other day for 3-17 days. This protocol is repeated indefinitely (e.g., at least about 3-18 months) using the optimal dose(s) obtained from the dose escalation trial, e.g., about 10-200 mg/day of a formula 1 compound.

**Example 5. Animal pharmacological studies.** Nonclinical studies were conducted using an oral and a subcutaneous formulation of BrEA. Rats were orally administered ¹⁴C BrEA solubilized in different excipients to determine the levels of drug in blood and various tissues. The results of these preliminary pharmacokinetics studies indicated that the absorption of BrEA by oral administration is about 0.1 to 15%, with at least about 80% excreted in the feces.

The nonaqueous BrEA formulation of example 1 was administered as a single subcutaneous dose to rabbits. More than 90% of the drug left the injection site within 24 hours of administration, and reached a maximum concentration in the plasma of about 1.2 % of the injected dose at eight to twelve hours post administration. The circulating half-life of the drug in the plasma was about twelve hours. The drug did not accumulate to a significant extent in any major organ and was primarily excreted in the urine.

BrEA was administered subcutaneously to rats using the formulation of example 1. Approximately 90% of the drug left the injection site within 24 hours of administration, and reached a maximum concentration in the plasma of about 0.2 % of the injected dose at one hour post administration. Elimination from the plasma was biphasic, with half-lives of about 12 and 72 hours respectively. BrEA did not accumulate to a significant extent in any major organ, and was excreted primarily in the feces. A study is also performed in Rhesus Monkeys with the example 1 formulation to determine plasma pharmacokinetics.

A pharmacokinetic analysis of ¹⁴C BrEA in plasma was conducted in two female Rhesus Monkeys. Trace labeled compound (16α-bromo-3-beta-hydroxy-5α-[4-¹⁴C]-androstan-17-one [50 mCi/mmole]) was used at a dose of 1 mg/kg as a subcutaneous injection in the scapular region using an injection volume of 1 mL/kg. The BrEA was formulated in 25% polyethylene glycol 300, 12.5% absolute ethanol, 5% benzyl benzoate, and qs with propylene glycol. 40 µCi were injected per animal. Blood samples were taken at 0, 0.5, 1, 2, 4, 8, and 24 hours for determination of ¹⁴C activity. The radioactivity in the plasma rose to near peak concentration in 8 hours and remained at approximately the same level through the end of the study at 24 hours.

A pharmacokinetic analysis of ¹⁴C BrEA was conducted in New Zealand White rabbits. Twenty µCi of ¹⁴C 16α-bromo-3-beta-hydroxy-5α-[4-¹⁴C]-androstan-17-one (50 mCi/mmole) plus 1 mg/kg unlabeled BrEA was administered to each of three New Zealand White rabbits as a subcutaneous injection in the scapular region using an injection volume of 1 mL/kg. The drug was formulated in 25% polyethylene glycol 300, 12.5% absolute . ethanol, 5% benzyl benzoate, and qs with propylene glycol. Blood samples were taken at 0.5, 1, 2, 4, 8, 12, 24 hours for all three animals, and at 48 hours for two of the animals. Twenty-four and 48 hours after administration, one and two animals respectively, were sacrificed, and the following organs/tissues were collected: brain, heart, kidneys, liver, lungs, skeletal muscle, spleen, and injection site muscle and skin. In addition to the organs and tissues, urine and feces were collected as well as the cage wash. BrEA did not accumulate to a significant degree in any of the organs listed above. Of the organs, the greatest mass of drug was observed in the liver, containing approximately 0.8% and 0.12% of the injected dose at 24 and 48 hours, respectively (average 0.13%).

| **Percentage of Drug in Organs (Rabbits)** | | | |
|---|---|---|---|
| **Organ or Tissue** | **Animal 201 24 hours** | **Animal 301 48 hours** | **Animal 302 48 hours** |
| Brain | 0.005 | 0.002 | 0 |
| Heart | 0.008 | 0.003 | 0.002 |
| Kidneys | 0.155 | 0.055 | 0.050 |
| Liver | 0.76 | 0.145 | 0.125 |
| Lungs | 0.029 | 0.019 | 0.011 |
| Spleen | 0.002 | 0 | 0 |
| Skeletal muscle (sample wt. in grams) | 0.002 (3.8 g) | 0 (6 g) | 0 (5 g) |
| Skin (sample wt. in grams) | 0.008 (8 g) | 0.002 (6 g) | 0.004 (9 g) |

The average percentages of the administered dose in whole blood was calculated by multiplying the concentration of drug in whole blood by the assumed volume of blood in the animals, 200 mL. The amount of drug in the blood reaches a maximum at around 8 hours, and a small amount was still evident at 48 hours. The amount of BrEA in whole blood was consistently lower than in plasma, suggesting the drug is not taken up to an appreciable extent by red blood cells.

*In vivo* experiments were conducted to determine the bioavailability of BrEA via oral administration using different formulations. BrEA was (1) solubilized in soya oil, vitamin E oil, a mixture of vitamin E and cremophore or (2) BrEA was micronized and combined with or without a surfactant These formulations are described below. The formulations were administered orally to rats and BrEA levels were determined in the blood, liver, spleen, kidney, and the lymph nodes. In the studies using micronized BrEA, the brain was evaluated for drug uptake. Twenty-four hour urine and feces were collected when BrEA was solubilized in vitamin E and soya oils and vitamin E mixed with Cremophore. The data from these studies indicate that BrEA enters into the lymphatics but is eliminated rapidly from the other tissues. The amount of ¹⁴C radioactivity recovered in the feces 24 hours after administration was 78 to 83%. A brief summary of each experiment is provided below and the results are provided in Table 6.

BrEA (5 mg in 1.0 mL of soya oil or vitamin E oil) supplemented with ¹⁴C-labelled BrEA was administered intragastrically to rats. Solubilization of BrEA in the vitamin E or soya oil was facilitated with 50 µL ethanol. Animals (3/time point) were assayed at 1.5, 3, 5.5, and 24 hours after administration and the ¹⁴C-radioactivity was measured in the blood, liver, spleen, kidney, lymph nodes and 24 hour feces and urine. The results indicate that, on the basis of ¹⁴C-radioactivity, some of the BrEA is taken into the lymphatic system. The uptake is greater with soya oil than vitamin E oil in the blood, liver, and lymph nodes.

BrEA (5 mg in 1.0 mL of a vitamin E and cremophore) supplemented with ¹⁴C-labelled BrEA was administered intragastrically to rats. Solubilization of BrEA in the vitamin E-cremophore mixture was facilitated by the adding 60 µL ethanol. Animals (4/time point) were sacrificed at 2, 3, 5.5, and 24 hours and ¹⁴C-radioactivity was measured in the blood, liver, spleen, kidney, lymph nodes and 24 hour feces and urine. The results indicate that a small portion of the drug is taken up by the lymphatic system. Judging from the values in plasma, liver and lymph nodes, it appears that drug uptake is slower compared with soy oil or vitamin E and its presence in the tissues is more persistent.

Rats, in groups of three males, were orally administered 1.0 mL of 0.9% NaCl containing 10 or 32 mg BrEA micronized with a surfactant, Synperonic PE/F 127 (2.5% wt/wt). Rats were examined at 1.5, 5 and 24 hours after administration. Blood, liver, spleen, kidney, lymph nodes, and brain were assayed for ¹⁴C radioactivity. The levels of BrEA in the blood, in comparison to the experiments with BrEA in Vitamin E oil and soya, were higher, 0.3% at 1.5 hours, and increased after 5 hours to 0.8% and 0.9% of the 10 and 32 mg dose, respectively. Additionally, the values in the lymph nodes were similar to those measured at 1.5 hours and the levels were sustained at 5 hours (5.3 and 5.0%) and 24 hours (3.7 and 3.1%) for the 10 and 32 mg dose, respectively (refer to Table 6).

In a repeated dose experiment, rats were intragastrically administered 1.0 mL 0.9% NaCl containing 2 mg BrEA micronized with Synperonic PE/F 127 (2.5% wt/wt) every 6 to 16 hours. Rats (3/time point) were sacrificed at 40, 72, 84, 90 and 96 hours after the first administration. Blood, liver, spleen, kidney and lymph nodes were assayed for ¹⁴C radioactivity. Higher levels in the blood, liver, kidneys and lymph nodes were noted in this experiment over previous studies.

Rats, in groups of three males, were orally administered 1.0 mL of 0.9% NaCl containing 2, 4 or 10 mg BrEA micronized without a surfactant. Rats were sacrificed at 1.5, 5 and 24 hours after administration and blood, liver, spleen, kidney, lymph nodes and brain were assayed for ¹⁴C radioactivity. The concentration of BrEA micronized without a surfactant in the observed tissues was lower than BrEA plus a surfactant.

**Example 6. Inhibition of parasites *in vitro.*** For *in vitro* antimalarial testing, micro-titer plates were used. The concentration of drugs were prepared as pMol/well according to WHO standard procedures (WHO, 1990). The test compound was dissolved in 15% DMSO in sterile RPMI-1640. Both chloroquine sensitive (e.g., WS/97) and resistant (e.g., MN/97) isolates of *Plasmodium* species are used.

A schizont inhibition assay was performed as follows. The micro-titer plates were predosed with various concentrations of the test compound. 50 µL of parasitised erythrocyte suspension in RPMI-1640 (0.2 mL erythrocyte + 0.3 mL serum + 4-5 ml RPMI-1640) were dispensed in microtiter wells that contained various concentrations of drug. Triplicate readings were made for each concentration.

A ³H-hypoxanthine incorporation assay was performed as follows. The testing was carried out according to the procedure of Desjardins et al. 1979. After 30 hr culture at 37 degrees C, the same microtiter plates from schizont inhibition assays with another triplicate wells were pulsed with ³H-hypoxanthine for overnight. The cell suspensions were washed twice on millipore glass fiber filter with Millipore filter apparatus. The filter discs were counted for DPM by a Beckman LS6000 β-scintillation counter. The activity of the drug was measured by plotting DPM against concentration of drug.

| Activity of compounds against Chloroquine sensitive T996/86 *P. falciparum in vitro* | | | | |
|---|---|---|---|---|
| Concentration (µM) | DHEA* | BrEA* | Etienic Acid Methyl Ester* | Etianic Acid Methyl Ester* |
| 30 | 65.6 | 98 | 60 | 61.5 |
| 15 | 44 | 60.1 | 45.7 | 47.4 |
| 7.5 | 38.3 | 50 | 40.9 | 45.3 |
| 3.25 | 37.2 | 43.7 | 46 | 41.4 |
| 1.875 | 23.2 | 40.9 | 41 | 43.4 |
| 0.938 | 37.2 | 31.8 | 43.3 | 47.1 |
| IC₅₀ | 19.0 µM | 7. 5 µM | 19.5 µM | 17.5 µM |

| | | | | |
|---|---|---|---|---|
| * - % inhibition | | | | |

| Concentration (nM) Chloroquine | % Inhibition Chloroquine |
|---|---|
| 200 | 95.9 |
| 100 | 94.6 |
| 50 | 97.3 |
| 25 | 94.5 |
| 12.5 | 86.8 |
| 6.25 | 27.2 |
| IC₅₀ | 9.0 nM |

The activity of 16α-chloroepiandrosterone and 16α-bromodehydro-epiandrosterone against chloroquine sensitive T996.86 and chloroquine resistant KI *P. falciparum in vitro* is shown below.

| | T996.86 | KI |
|---|---|---|
| 16-chloroepiandrosterone IC₅₀ | -9.25 pg/mL | -9.25 µg/mL |
| DHEA-Br IC₅₀ | -25.0 pg/mL | -25.0 µg/mL |

Other formula 1 compounds, e.g., any compound in compound group 1 through 25-6 are used in a similar manner to inhibit *Plasmodium* parasites.

**Example 7. Four-day *in vivo* protocol for inhibition of *Plasmodium berghei*.** The 4-day suppressive test has been widely used and it can be performed within a 1 week period. The test consists of the inoculation of parasitised erythrocytes on the first day of the experiment (D₀), followed by an injection of the test compound, which is also administered on the 2^{nd}, 3^{rd} and 4^{th} days of the protocol. On the 5^{th} day, blood films are taken and antimalarial activity is assessed either by calculating parasitemia, or by scoring parasite numbers on a predetermined scale (i.e., 1-5). Peters (*Ann. Trop. Med. Parasitol*. 64: 25-40, 1970) described a basic procedure using this 4-day test.

The protocol is summarized as follows. Five female TO mice were used per test group. *P. berghei* HP15 ANKA parasites were collected by cardiac puncture using a heparinised syringe from a donor mouse having a 30+% parasitaemia. The blood was diluted with diluting agent (50% HIFCS+50% sterile PBS) to a final concentration of 1% parasitaemia or 1X10⁷ infected erythrocytes per 0.2 mL of the infecting suspension. Each mouse was inoculated intravenously, which produced a more uniform infection rate than intraperitoneal administration of 0.2 mL of the infecting suspension. Test compounds were prepared at doses of 100 mg/kg in (16.7% DMSO + 83.3% Celacol). The steroid formulations were administered intraperitoneally 2 hours after parasite inoculation. The compounds were administered once a day starting on D₀, and continued on the following three days. Blood films were made from tail blood on the day after the last dosing of compound and the blood was fixed with 100% methanol and stained with 10% Giemsa. Parasitaemias were scored on a scale of 0-5, where 5 is equal to the control.

An inoculum of 1% parasitaemia 1x10⁷ erythrocytes/mL, 0.2 mL per mouse (female strain TO mice), was delivered by intravenous injection. Drug administration commenced 2 hours after inoculation on Day 1 and continued for 3 days. The results are shown below from blood films from all 20 mice on Day 5 when parasitaemias were assessed.

| Compound | Treatment | Parasitaemia Score (0 - 5) |
|---|---|---|
| BrEA | 100mg/kg x 4 days i.p.* | 1 |
| Etienic Acid | 100mg/kg x 4 days i.p. | 2 |
| DHEA | 100mg/kg x 4 days i.p. | 1 |
| Chloroquine | 3 mg/kg x 4 days i.p. | 1 |
| control | N/A | 5 |

| | | |
|---|---|---|
| * i.p. = intraperitoneal injection | | |

In a similar protocol, mice are inoculated with a solution containing 1 x 10⁷ erythrocytes/mL by I.V. injection. Two hours later give drug is delivered by I.V. injection. BrEA or another formula 1 compound is given (0.2 mL I.V. or S.C.) once a day for 4 days. Tail snips are used to obtain blood after the study. Mice infected with *P. berghei* were used to obtain infected cells. Parasites are harvested from cardiac mouse blood, and uninfected mice are infected using 0.2 ml of blood with 14% parasitaemia per mouse I.V. Two hours later, the first dose of BrEA (100 mg/kg I.V. or S.C.) is delivered to the infected animals. The BrEA formulation was a sterile solution containing 15 mg/mL of BrEA in 45% hydroxypropyl-β-cyclodextrin and 0.9% saline. At 1, 2, 3 and 4 days after the infection of the animals, BrEA (100 mg/kg I.V. or S.C.) is delivered to the infected animals. No deaths occurred in the group receiving I.V. BrEA at day 30, but all control animals were dead by day 10. All animals treated with BrEA by S.C. delivery were dead by Day 11.

**Example 8. Rat *in vitro* and *In vivo* study.** In the *in vitro* protocol the parasite *(Plasmodium falciparum*, chloroquine sensitive strain WT and chloroquine resistant strain Dd2) level is adjusted to 1% and the hemocrit is adjusted to 7% with medium. Using a 96 well plate, 50 µL of parasite and 100 µL of drug mixed with media are added to each well and the procedure is done in triplicate. The plate is placed in a chamber containing a physiological gas mixture and incubated at 37°C. The media/drug mixture is changed at 24, 48 and 72 hours. On day 5 (96 hours) slides of each well are made, stained with Gemsia and 500 red blood cells are counted for each slide. The triplicates are averaged and data are reported in percent inhibition.

in the *in vivo* protocol, Lewis rats weighing 80-85 grams were given a standardized IP injection of parasite (*Plasmodium berghei*). Rats were then intravenously injected 2 hours later with one of the treatments described in the table below, returned to their housing, fed standard lab chow and allowed free access to water. Animals were weighed and treated again 24, 48, and 72 hours after the first treatment and again returned to their housing and they were allowed free access to food and water. The animals were weighed again and then bled using a 26-gauge needle on day 5, 11 and 28 post inoculation. Hemocrits were measured and blood smears are prepared for each rat. The blood smears were then stained using Gemsia and the level of parasitemia (defined as the percent of red cells with parasites) were determined. Animals were again returned to their housing and observed twice daily for evidence of progressive disease, defined as listlessness and or adverse drug reaction, which is defined as a loss of 20% of original body weight, for a total of 28 days. If either progressive disease or drug reaction is noted, the animals are euthanized.

The BrEA formulation was a sterile solution containing 15 mg/mL of BrEA in 45% hydroxypropyl-β-cyclodextrin and 0.9% saline.

| Group 1 | Group 2 | Group 3 | Group 4 |
|---|---|---|---|
| Control 0.9% | Chloroquine | BrEA Low Dose | BrEA High |
| saline | Control 40mg/kg | 30 mg/kg | Dose 60 mg/kg |

The intravenous injections were given on days 0, 1, 2 and 3 and the results are shown below. The results showed that treatment *in vivo* with a formulation comprising BrEA reduced parasitemia to a level comparable to that seen with the chloroquine ("Clq") control. The results are summarized below.

| | % RBC parasitemia |
|---|---|
| Day 4 | |
| saline control | 16% |
| chloroquine control | 10% |
| low dose BrEA | 9% |
| high dose BrEA | 7% |

| | % RBC parasitemia |
|---|---|
| Day 11 | |
| saline control | 36% |
| chloroquine control | 16% |
| low dose BrEA | 12% |
| high dose BrEA | 11% |

**Example 9. Human clinical study - parasite infection.** Response to drug treatment was graded as per World Health Organization criteria (WHO 1973) in infected patients. Evaluation of therapeutic response was determined using the parasitic and fever clearance times. Parasite clearance was expressed as three indices; the time for the parasite count to fall by 50% of the pre-treatment (baseline) value (PC₅₀), (ii) the time for the parasite count to fall by 90 % of the baseline value (PC₉₀) and (iii) the time for the parasite count to fall below the level of microscopic detection (parasite clearance time PCT) (N.J. White and S. Krishna *Trans. R. Soc. Trop. Med. Hyg*. 83: 767-777, 1989; White et al., *J. Infect. Dis.* 165: 599-600, 1992; White et al., *J. Infect. Dis.* 166: 1195-1196, 1992). The fever clearance time was defined as the time from drug administration till the oral or rectal temperature fell to or below 37.2° C and remained so for at least 48 h.

Venous blood (5mL) was obtained from two patients before treatment and at 4, 6, 8, 12, 18, 20, 24, 30 and 36 h after treatment or at 4 or 6-hourly intervals after treatment until there was complete clearance of peripheral parasitemia. Blood was collected aseptically and transferred to 10 mL syringes containing 2 mL of acid citrate dextrose (ACD) for *in vitro* culture. Prior to incubation, the plasma was separated from the red blood cells and the red blood cells were washed twice. Parasites were cultured by modification of standard in vitro culture techniques (W. Trager and J.B. Jensen, *Science* 193:673-675, 1976; A.M. Oduola et al., *J. Protozool.* 39: 605-608, 1992). Samples were dispensed into sterile centrifuge cubes within 10 min of collection and spun down. The supernatant plasma was stored while the packed cells were washed twice with culture medium (washing medium, RPMI-1640 medium, containing 25 mM HEPES buffer and 25 mmol/L NaOH). The buffy coat was removed by vacuum aspiration. A 1:10 fold dilution was done for each blood sample with complete washing medium [CMP (washing medium supplemented with 10 % human plasma)]. One milliliter each of the sample was transferred into 2 wells of a 24 well micro culture plate. Cultures were incubated at 37 degrees C in an atmosphere of 5% CO₂, 5% O₂ and 90% N₂ premixed gas. The culture medium was changed daily and thin blood smears were prepared for microscopy at 24 and 48 h after the culture has been set up. The culture samples were diluted with unparasitized washed type A Rh-positive red blood cells if the proportion of parasitized red blood cells was more than 2%.

Microscopy. During the *in vivo* study, thin and thick blood films were fixed with dehydrated methanol (100%) and heat, respectively, were stained with 10% Giemsa for 20 min. Parasitemia was quantified in thin films by counting 2000 red blood cells in clear contiguous fields and finding the proportion that was parasitized. In thick films, parasitemia was quantified by counting parasites against leukocytes. A film was declared negative if no parasites were found after examination of 200 microscope fields of a thick smear. During *in vitro* and *ex vivo* study, pretreatment thin and thick smears were, graded for ring stages by the method of Jiang as modified by Li et al. (J.B. Jiang et al, *Lancet* 2(8293): 285-288, 1982; K. Silamut and N.J. White *Trans. R. Soc. Trop.Med. Hyg*. 87: 436-443, 1993; X.L. Li et al, *Chi. J. Parasitol. Dis*. 12: 296, 1994). Approximately 5000 erythrocytes were counted in clear contiguous fields 24 and 48 h after incubation of blood obtained at each time point and graded for maturity into tiny rings, small rings, large rings, pigmented trophozoites and schizonts. Functional viability was estimated as the percentage of asexual ring forms capable of maturing to pigmented trophozoites or schizonts after 24-48 h of *in vitro* culture (W.M. Watkins et al., *Trans. R. Soc. Trop. Med. Hyg*. 87: 75-78, 1993).

Calculation of parameters. The patients presented with acute symptomatic severe non-cerebral pure *P. falciparum* malaria. They had oral fluid intolerance, body temperatures greater than 39°C, greater than 5000 parasites per micro liter of blood, asexual parasitemia and they had a negative urine test for antimalarial drugs. They were administered 25 mL intravenously every four hours with BrEA suspended in sterile 45% β-cyclodextrin in saline at a concentration of 25 mg/mL. This regimen was continued for four days. Parasitemia quantification and clinical examination were done once every 6 hours for the first 72 hours, followed by daily assessment of the parameters up to day 7 (168 hrs) and thereafter on day 14.

Blood films were Giemsa-stained and parasitemia quantification was done in thick films by counting 2000 parasites against leukocytes, and the thin films by finding the proportion of infected red blood cells. Response to drug treatment was graded according to WHO criteria. Evaluation of therapeutic response was done using the parasitic and fever clearance times. Parasite clearance was expressed as three indices: The time for the parasite count to fall by 50% of the pre-treatment (baseline) value (PC₆₀); to fall by 90% of the baseline value (PC₉₀); and to fall below the level of microscopic detection (parasite clearance time) PCT.

The fever clearance time was defined as the time from drug administration until the oral/rectal temperature fell to below 37.2 degrees C and remained so for greater than 48 hours. The parasite clearance rate at day 14 was 100%. The clinical response thus included an effect on parasitemia in both patients and amelioration of one or more symptoms of infection.

| Intravenous BrEA Malaria Patient Trial | | |
|---|---|---|
| | Patient A | Patient B |
| Fever clearance time | 12 hrs | 18 hrs |
| Parasite clearance times | | |
| Time to 50% clearance | 18 hrs | 24 hrs |
| Time to 90% clearance | 24 hrs | 48 hrs |
| Time to 100% clearance | 48 hrs | 64 hrs |

**Example 10. Cellular studies *in vitro*.** The effect of BrEA on pentosephosphate shunt (PPS) activity in normal human RBC was examined using whole cells. Since glucose-6-phosphate dehydrogenase ("G6PD") is the limiting enzyme of the PPS, PPS flux measurement is considered to better reflect G6PD activity in the whole cell compared to G6PD activity measurement in a cell lysate. G6PD activity measured in a cell lysate is typically about 1100-fold higher than the PPS flux in whole resting unstimulated RBC (G6PD activity in cell lysate: 165; PPS flux 0.142 micromoles/hour/ml RBC). PPS flux and G6PD activity in the whole RBC depends on a number of factors (the concentration of NADPH, NAD, and ATP, and intracellular pH), which are kept constant if the measurement is performed in the lysate and may vary in the whole RBC. Levels of G6PD activity in cells is considerably above normal basal needs and inhibition of overall G6PD activity might have no or minor consequence on PPS flux in the whole cell. For example, RBC with the Mediterranean G6PD mutant with about 1-3 percent residual activity compared with normal individuals have no impairment in basal PPS flux, but show impaired flux when flux through PPS is stimulated by methylene blue addition. A series of experiments were perfromed using varying amounts of BrEA and PPS flux was measured in unstimulated basal RBC and in methylene-blue (MB)-stimulated RBC.

The data below shows PPS flux (micromoles/hour/ml RBC) in basal unstimulated, and MB-stimulated normal RBC. Different concentrations of BrEA (0.3, 3.5 and 7 micromolar, final) were supplemented to suspensions of washed RBC suspended in RPMI, pH 7.4 at 10% hematocrit, whereby PPS flux was immediately measured without further incubation and without further washings. A minor inhibition of MB-stimulated PPS flux was observed with BrEA at 7 µM.

| | PPS flux |
|---|---|
| control, unstimulated RBC | 230 |
| DMSO control, unstimulated RBC | 270 |
| DMSO control, MB stimulated RBC | 5090 |
| 0.3 µM BrEA, unstimulated | 250 |
| 0.3 µM BrEA, MB stimulated | 5000 |
| 3.5 µM BrEA, unstimulated | 270 |
| 3.5 µM BrEA, MB stimulated | 4950 |
| 7 µM BrEA, unstimulated | 295 |
| 7 µM BrEA, MB stimulated | 4660 |

The data below shows average values of 3 experiments, where basal, unstimulated, and MB-stimulated PPS flux (micromoles/hour/ml RBC) was measured in normal RBC. In these experiments, different concentrations of BrEA ∼0.8, 8 and 80 micromolar, final) were supplemented to suspensions of washed RBC suspended in RPMI, pH 7.4 at 10% hematocrit. After a 90-min incubation at 37° C with and without BrEA. PPS flux was measured. The results showed a dose-dependent inhibition of MB-stimulated PPS flux. Inhibition was 10% at 8 micromolar (p=0.006 vs control+DMSO) and 25% at 80 micromolar (p=0.002 vs control+DMSO).

| | PPS flux |
|---|---|
| control, unstimulated RBC | 430 |
| control, MB stimulated RBC | 5410 |
| DMSO control, unstimulated RBC | 480 |
| DMSO control, MB stimulated RBC | 4890 |
| 0.8 µM BrEA, unstimulated | 410 |
| 0.8 µM BrEA, MB stimulated | 4930 |
| 8 µM BrEA, unstimulated | 450 |
| 8 µM BrEA, MB stimulated | 4430 |
| 80 µM BrEA, unstimulated | 450 |
| 80 µM BrEA, MB stimulated | 3660 |

**Example 11. Inhibition of parasite growth.** The effect of Epi (16α-bromo-epiandrosterone) on parasite (*Plasmodium falciparum*) growth was shown. EPI was active at a concentration of 1 µM.

| | Parasitemia after treatment | | | |
|---|---|---|---|---|
| | Time 0 | 24hrs | 48hrs | 72hrs |
| control + DMSO | 5% | 5.40% | 3.10% | 5.20% |
| Epi 1 µM | 5% | 5.70% | 5,50% | 1,60% |
| Epi 10 µM | 5% | 5,60% | 0.90% | 0 |
| Epi 100 µM | 5% | 0 | 0 | 0 |
| Epi 500 µM | 5% | 0 | 0 | 0 |
| | | | | |
| control + DMSO | 2% | 8.80% | 11 % | 8% |
| Epi 50 nM | 2% | 9.90% | 9.20% | 8.30% |
| Epi 1 µM | 2% | 5.80% | 6.10% | 2.10% |
| Epi 2.5 µM | 2% | 7.30% | 5.80% | 3.20% |
| Epi 5 µM | 2% | 5.40% | 6% | 1.80% |
| Epi 10 µM | 2% | 4.20% | 3% | 0 |
| Epi 50 µM | 2% | 0 | 0 | 0 |

Parasitemias were determined by standard methods (microscopic inspection of at least 500 cells, stained with Diff-Quick™ (Baxter). Parasites were cultured under standard conditions in RPMI-1640 supplemented with Hepes/Glucose (10 mM), glutamine (0.3 g/liter) and 10% human plasma. The hematocrit was 1%.

**Example 12. Stimulation of phagocytosis.** The capacity of BrEA to influence phagocytosis of *Plasmodium* parasite-infected RBC is examined using adherent human monocytes. The parasitemia level is about 8-10% and human monocytes are obtained from buffy coats from blood as follows. Peripheral blood mononuclear cells are separated from freshly collected platelet-poor buffy coats discarded from blood samples of heafthy adult donors of both sexes. Separated cells are washed once with luke-warm PBS supplemented with 10 mM glucose (PBS-G) and resuspended at 5 x 10⁶ cells/mL in ice-cold RPMI 1640 medium supplemented with 23 mM NaHCO₃ and 25 mM Hepes, pH 7.4 (RMBH). Dynabeads M450 Pan B and Pan T (Dynal) are added to cells in a 4:1 ratio for 20 min at 4°C. B-Iymphocytes and T-Iymphocytes are removed as specified by the manufacturer. The remaining monocytes are washed 2 times in RMBH, resuspended in AIM V cell culture medium (Gibco) at 1 x 10⁶ cell/mL. The monocyte layer is collected, washed with PBS-G at 37°C and resuspended in AIM V medium at 1 x 10⁶ cells/mL. Purified cells are >90% monocytes as assessed by CD14 expression.

Phagocytosis of opsonized parasitized RBC (PE) is determined as follows. Phagocytosis of fresh-serum opsonized PE is initiated by mixing 10 PE/monocyte. Suspensions are briefly centrifuged (150 x g for 5 sec at room temperature) to improve contact between PE and monocytes. To avoid attachement of monocytes after centrifugation and during the whole incubation period, cells are kept in suspension at 5 x 10⁶ cells/5 mL AIM V medium in 6 cm diameter teflon bottom dishes (Heraeus) in a humidified incubator (95% air, 5% CO₂) at 37°C. On average, at least 90% of the monocytes phagocytose PE, as assessed by microscopic inspection. Control cells are kept under similar conditions without phagocytosis. Quantitative assessment of phagocytosis is performed by a previously described bioluminescence method (E. Schwarzer, et al., *Br. J. Haematol*. 1994 88: 740-745).

Erythrocyte treatments and parasite cultures are as follows. Fresh blood (Rh+) is used to isolate erythrocytes (RBC). Washed RBC are infected with schizont/trophozoite parasite stages (Palo Alto strain, mycoplasma-free). Stage specific parasites are isolated by the Percoll-mannitol method. Briefly, normal schizont-stage parasitized RBC (SPE) separated on Percoll-mannitol gradient (parasitemia > 95% SPE) are mixed with RBC suspended in growth medium (RPMI 1640 medium containing 25 mmol/L Hepes, 20 mmol/L glucose, 2 mmol/L glutamine, 24 mmol/L NaHCO₃, 32 mg/L gentamicin and 10% AB or A human serum, pH 7.30) to start synchronous cultures at selected hematocrit values. The inoculum parasitemia is adjusted to 20% normal SPE for isolation of ring parasitized RBC (RPE) and to 5% normal SPE for isolation of trophozoite-stage parasitized RBC (TPE). At 14-18 hours after inoculum parasites are at ring-stage in the first cycle; at 34-33 hours, parasites are at trophozoite-stage in the first cycle; and at 40-44 hours after inoculum parasites are at schizont-stage in the first cycle. RPE, TPE and SPE are separated on Percoll-mannitol gradients. The parasitemia is usually 8-10% RPE, and >95% TPE. Nonparasitized and parasitized RBC are counted electronically. To assess total parasitemia and relative contribution of RPE, TPE and SPE, slides are prepared from cultures at indicated times, stained with Diff-Quik™ parasite stain and about 400-1000 cells are examined microscopically.

The effect of a formula 1 compound such as BrEA in parasitized RBC is examined using various concentrations of the compound, e.g., BrEA, e.g., 0.5 µM, 1 µM, 10 µM, 25 µM and 50 µM. Trophozoite-parasitized RBC, schizont-parasitized RBC or ring-parasitized RBC are examined as described.

**Example 13. Human malaria clinical trial.** The clinical trial protocol that incorporates about 15-20 patients is established. For a phase I, I/II or II trial, the patients are mildly infected with one or more *Plasmodium* parasites and they are mildly symptomatic (less than about 8-10% parasitemia of RBC). Before treatment, the patients are optionally tested for infection with HIV, HCV, TB, and *Cryptosporidium*. Patients with one or more co-infections are given standard care for the coinfection. The patients are hospitalized for treatment for one week. Two or more dose groups, e.g., 25, 50 or 100 mg/day of BrEA administered parenterally, e.g., by intramuscular, subcutaneous or intravenous injection, on 3, 4 or 5 days of the week when patients are dosed. Dosing is on consecutive days or on an intermittent schedule, e.g., 2, 3 or 4 doses with one dose administrered every other day.

The formulation containing BrEA is as described herein, e.g., the formulation of example 1 or a formulation that comprises 100 mg/mL BrEA, PEG300 ∼30% v/v, propylene glycol 30% v/v, benzyl benzoate 30% v/v and benzyl alcohol 2% v/v. At day 5-7, if less than about 50% reduction in parasitemia is observed, the patients are given standard care for malaria (mefloquine). During the week of treatment and for 1, 2 3, or more weeks there after, blood samples are taken periodically for evaluation of parasitemia, pharmacokinetics, plasma cytokines (e.g., IL-2, IL-4, IL-10, IGF1, γIFN, GM-CSF), and intracellular cytokines (e.g., IL-2, IL-4, IL-10, IGF1, γIFN, GM-CSF). The patients are optionally treated again at about 2 to 12 weeks after the initial dosing, using the same or a similar protocol as that used in the initial dosing protocol.

An exemplary open-label study of a BrEA formulation administered intramuscularly to semi-immune patients with uncomplicated malaria is conducted. The formulation comprises 100 mg/mL BrEA, PEG300 ∼30% v/v, propylene glycol 30% v/v, benzyl benzoate 30% v/v and benzyl alcohol 2%. Patients will remain at the hospital as in-patients for the first 7 days of the study. Patients will receive one daily intramuscular administration of 50 mg or 100 mg of BrEA for 5 consecutive days. Daily evaluation for the first 7 days, and up to study day 14, may include parasitemia evaluation (twice daily), chemistry, hematology and drug levels (pharmacokinetic evaluation). If, after study day 7, the parasitemia levels decrease from the screening value and the patient is clinically stable, the patient may be followed on a daily basis for parasitemia (twice daily) for up to an additional 7 days as hospital in-patients. If a patient becomes clinically unstable at any time during the study, the patient will be discontinued and may be offered the standard treatment for malaria. Patients deficient in glucose-6-phosphate dehydrogenase enzyme may be excluded, since BrEA inhibits the enzyme. Other considerations that may lead to exclusion of patients from the trial include patients diagnosed with any of the following: severe anemia (hematocrit < 21 % or hemoglobin < 7 g/dL); renal or liver failure by history and/or laboratory results respiratory distress as evidenced by dyspnea or respiratory rate ≥ 30 per minute; hypotension (systolic blood pressure < 90 mm Hg); tachycardia (heart rate > 130 beats/minute); pregnant or breast-feeding women; significant active co-morbid illness (acute medical diagnosis requiring specific therapy; patients with parasitemia >10% on peripheral smear.

Blood samples may be collected from each patient for future clinical evaluation such as the determination of activation markers or immunological analyses (e.g., assay for intracellular or extracellular interleukins IL-1β, IL-2, IL-4, IL-6, IL-10 and IL-12, γIFN and TNFα).

**Example 14. Liposome formulation.** Liposomes suitable for parenteral administration are prepared as follows. 400 mg of phosphatidyl choline and 80 mg of BrEA are dissolved in chloroform and methanol (2:1 v/v) and the solution is dried by rotary evaporation under reduced presure. The resulting film is rehydrated by adding 8.0 mL of a 0.9% w/v NaCl solution and agitating the solution. The sizes of the liposomes are optionally measured, e.g., by photon correlation spectroscopy (Malvern Zetasizer 3000 or equivalent). The liposomes are optionally sized by, e.g., sonication to reduce the average size below 400 mn, or by filtration using suitable filters. Similar procedures are used to prepare liposome preparations that contain a formula 1 compound at about 15-100 mg/mL. The formulation is used to deliver the compound orally or parenterally (I.M., S.C., I.V.).

**Example 15. Cyclodextrin formulation.** A cyclodextrin formulation containing BrEA is prepared as follows. 45 g of hydroxypropyl-β-cyclodextrin is added to 1 L of sterile physiological saline and the mixture is stirred for about 4-24 hours, until a clear solution is obtained. Non-micronized BrEA is added to give a concentration of 20 mg/mL and the mixture is stirred until a clear solution is obtained. The solution is sterilized by filtration using a 0.2 µm pore size filter and dispensed into sterile containers. Similar procedures are used to prepare cyclodextrin formulations that contain a formula 1 compound at about 15-100 mg/mL. The formulation is used to deliver the compound orally, parenterally (I.M., S.C., I.V.) or by a buccal or sublingual route..

**Example 16. Suppository formulation.** A suppository formulation containing a formula 1 compound such as BrEA is prepared as follows. Sufficient non-micronized BrEA is measured to obtain a desired number of units that comprise 500 mg each of BrEA. The BrEA is blended with with a suppository base, e.g., triglyceride from edible vegetable oil, to provide desired characteristics, e.g., a free fatty acid content of about 0.1% w/w, a saponification value of about 242, an iodine value of about 3, moisture at about 0.1% w/w and a closed capillary melting point of about 35°C.

**Example 17. Human HCV clinical trial.** A female patient infected with HIV and HCV was dosed I.V. with BrEA for 3 consecutive days using a formulation that contained 20 mg/mL BrEA in 45% w/v hydroxypropyl-β-cyclodextrin and saline. Four mL of the formulation (80 mg BrEA) was administered to the patient every 4 hours during the 3 day treatment period. The patient's predosing HCV level was 6.5 Log₁₀ as measured by PCR and the HCV level was 6.2 Log₁₀ on the first day of dosing, 5.5 Log₁₀ on the 3^{rd} day of dosing and 4.9 Log₁₀ three days after the last dose was administered. HIV RNA levels as measured by PCR was 5.2 Log₁₀ (predosing), 5.8 Log₁₀ (first day), 5.9 Log₁₀ (third day) and 5.4 Log₁₀ (day 6). The NK cell counts (cells/mm³) were 28, 41 and 38 at predosing, day 0 and day 3.

**Example 18. Formulation.** A formulation comprising 100 mg/mL BrEA, ∼30% v/v PEG300, 30% v/v propylene glycol, 30% v/v benzyl benzoate and 2% v/v benzyl alcohol was prepared by suspending BrEA in polyethylene glycol 300, and sequentially adding propylene glycol and benzyl benzoate, to form a solution, which was diluted to the final desired volume with additional propylene glycol. The procedure is described below.

The calculated amount of polyethylene glycol 300 was added to a compounding vessel. Then, while mixing, the calculated amount of BrEA was added to the vessel, and mixed for at least 5 minutes to form a smooth, creamy liquid propylene glycol was added to the vessel, and mixed for a minimum of 5 minutes to form a uniform suspension. The calculated amount of benzyl benzoate is added to the vessel, and mixed for approximately 5 minutes to form a translucent liquid suspension. Propylene glycol was then added to achieve the desired final formulation, and mixed for approximately 5 minutes. The drug solution was transferred to a volume dispensing device set to deliver 1.2 mL per vial. Under nitrogen pressure, the solution was filtered through two 0.2 µm polyvinylidene fluoride filters in series into 2 cc amber glass vials. The vials were capped with Teflon-coated, butyl-rubber stoppers and crimp sealed.

**Example 19. Opportunistic infection clinical protocol.** A double blind, randomized, placebo controlled study of 100 mg of BrEA administered intramuscularly to late stage HIV-infected patients at risk for opportunistic infections (Ols). HIV-1 seropositive patients with a CD4 cell count ≤100 cells/mm³, HIV RNA at 1x10⁶ copies/mL and a Kamofsky score of at least 60 are identified for potential inclusion into the protocol. Patients in all clinical protocols must understand and sign a written informed consent form prior to screening evaluations.

BrEA in the formulation of example 16 is used. Administration of drug or vehicle will be for 3 to 5 consecutive days followed by about 35-90days of observation, e.g., 37 days of observation. An exemplary treatment regimen comprises 5 days of treatment followed by 37 days of observation, which is repeated for a total of 7 courses over 42 weeks. The incidence rate of Ols as well as the time to resolution or control of the Ols will be monitored and compared to a placebo control group. The patients may be monitored monthly for 2 or 3 months after completion of the study for follow-up. The incidence of Ols or conditions associated with AIDS are monitored, e.g., as tuberculosis (TB), candadiasis, Pneumocystis pneumonia (PCP), diarrhea, or Kaposi's sarcoma, may be evaluated as protocol endpoints. If a patient is diagnosed with one or more of the protocol specified opportunistic infections, the protocol regimen a treatment for the Ol will be initiated, e.g., Fluconazole for Candidiasis or for PCP, trimethoprim and sulfamethoxazole or Dapsone. A similar protocol is used with other formula 1 compounds.

**Example 20. Human HIV clinical protocol.** Patients infected with HIV are dosed with an i.m. injection of 25-200 mg of BrEA using a formulation containing 100 mg/mL BrEA, PEG300 ∼30% v/v, propylene glycol 30% v/v, benzyl benzoate 30% v/v and benzyl alcohol 2% v/v. The pateints are dosed once per day for 5 consecutive days followed by a period of about 28 days or longer with no BrEA treatment. The patients were them provided with one more course of 5 consecutive days of dosing with BrEA, followed by a non-dosing period of at least about 28 days. Up to 5 rounds of 5-day treatments, followed by at least 28 days of no dosing were provided. Immunological responses were then assayed using blood or plasma samples from the patients by flow cytometry and other known analytical methods. Immune cell subsets or other measured markers were assayed within 24 hours of obtaining the sample from each patient. Labeled antibodies, e.g., anti-CD antigen antibodies conjugated with fluorescent dyes (FITC, phycoerythrin, allophycocyanin or PerCP), were prepared and used essentially according to standard protocols using commercially available reagents, see, e.g., PharMingen, 1998 Research Products Catalog, technical protocols at pages 732-774, human cell surface molecules at pages 182-295 and mouse, rat and hamster cell surface molecules at pages 2-173 and cytokine and chemokine reagents at pages 344-489.

The clinical protocol is a phase I/II, open-label, randomized study of 3 dose levels of BrEA administered intramuscularly to HIV-infected patients who are treatment naïve. There will be 3 treatment groups and each group will consist of 2 parts (Parts A and B). Patients will receive the same dosage of BrEA throughout Parts A and B of the study. If a patient experiences an antiviral response (an HIV RNA titer at least 0.5 log below the average of the screening and baseline values) or benefits (any decrease in HIV RNA titers below the average of the screening and baseline values) from the treatment received during Parts A and B of the study, the patient may continue receiving 5-day treatment courses of the BrEA formulation of example 2 at the dose initially received. This treatment course may be repeated up to six times.

All patients may be monitored for levels of HIV RNA (Chiron Quantiplex™ branched chain DNA assay), T-cell subsets [CD4/CD8], proviral HIV DNA (PBMC), interleukins [IL-2, 4, 6, 8, 10, and 12] (serum), γIFN (serum), insulin-like growth factor [IGF-1] (serum) and tumor necrosis factor [TNF] (serum) throughout the study. PBMC quantitative co-culture (cells) may be conducted on a subset of patient samples. Assays for additional activation markers may be conducted. Analysis of chemistry and hematology panels and urinalysis is planned. Additionally, patients co-infected with hepatitis B and/or C viruses, malaria or tuberculosis may be monitored regularly for viral titers or microbiological cultures. Serial blood and urine samples will be collected from a subset of patients for pharmacokinetic determination after the first dose on Part A and the last dose on Part B.

Treatment may consist of more than one intramuscular injection. Intramuscular injections may be administered in different locations (i.e., left or right upper arms or thighs or buttocks) and a single 100 mg or 200 mg dose of BrEA may be delivered to patients in two or more subdoses of less than 100 mg (e.g., 50 mg).

There are two segments of this study, Segment 1 and 2. Both segments consist of two parts, Part A and Part B. The first 12 patients enrolled on the study will be assigned to the design described in Segment 1. The remaining 24 patients will be assigned to Segment 2 of the study. The design of each segment is provided below.

Part A will consist of a single intramuscular injection of a BrEA formulation. The day the patient receives the injection will be study day 1. Patients participating in the pharmacokinetic subgroup will have serial blood and urine samples collected, beginning on study day 1. Part B of the study begins on study day 8 (Segment 1) or study day 15 (Segment 2).

Segment 1 Part B consists of 5 consecutive daily intramuscular injections of the formulation of example 1 at the same dose as received in Part A of the study. The day the patient receives the first dose will be on about study day 8-12. The 5-day treatment course is followed by an approximate 28-day observation period (or approximately 32 days from a first dose on day 8 to the initiation of a second treatment course on day 40). During the observation period, patients will be asked to return to the clinic on a weekly basis for various tests. Patients participating in the pharmacokinetic subgroup will have serial blood and urine samples collected, beginning approximately on study day 12-17.

Segment 2 Part B consists of 5 consecutive daily intramuscular injections of the formulation of example 2 at the same dose the patient received during Part A of the study. The day the patient receives the first dose will be about at study day 15. The 5-day treatment course is followed by an approximate 45 day observation period (or approximately 49 days from the first dose on study day 15 to the initiation of the next treatment course on study day 64). During the observation period, patients will be asked to return to the clinic on a weekly basis for various tests. Patients participating in the pharmacokinetic subgroup will have serial blood and urine samples collected, beginning approximately on study day 19.

Randomization in this dose escalation study is as follows. When 4 of the 12 patients per treatment group have completed 5 days of daily dosing on Part B and have not experienced a serious drug-related adverse event, enrollment into the next higher dose level will occur, after consultation between the sponsor and investigators.

The first four patients enrolled will be assigned to the 50 mg dose group. If no serious drug-related adverse events are experienced, the next 8 subjects will be randomized to either the 50 mg or 100 mg dose level in a 1:1 fashion. If no serious drug-related adverse events occur in patients receiving 100 mg, then the next 24 patients will be randomized to either the 50, 100, or 200 mg dose group in a 1:2:3 fashion.

If 4 of the 12 patients in a dose group experiences a serious drug-related event (Grade III or IV), 2 additional patients will be enrolled at the same dose level. Additionally, patient enrollment on to the next dose level, if enrolling, will be temporarily on hold until safety is assessed. If one of the 2 additional patients experiences a serious drug-related event, dosing in this dose level will discontinue. Upon consultation with the sponsor and investigators, additional patients may be enrolled at a dose between the dose-limiting group and the next lower dose group to determine the maximum tolerated dose (MTD). Enrollment of additional patients at a specific dose level will be determined in a protocol amendment.

The results indicated that a single 50 mg or 100 mg dose of BrEA increased the numbers of activated CD8⁺ and CD4⁺ T cells (e.g., CD8⁺, CD69⁺, CD25⁻ cells) that were circulating in the patient's blood. Also, the circulating numbers of dendritic precursor cells, NK cells, LAK cells and cells that mediate ADCC (antibody-dependent cell-mediated cytotoxicity mediated by the CD8⁺, CD16⁻ immune cell subset) functions were increased. Further increases were usually observed on dosing for 5 consecutive days.

Some of the results are summarized below. Course 1, 2 and 3 refer to each 5 consecutive day treatment regimen of one daily injection with BrEA (50 or 100 mg BrEA per injection). In the diagrams below, HE2000 refers to the formulation containing 100 mg/mL BrEA, PEG300 ∼30% v/v, propylene glycol 30% v/v, benzyl benzoate 30% v/v and benzyl alcohol 2% v/v. The data shown below was obtained from patient blood samples at baseline (on the day dosing was initiated) and at various times after the patients received at least one dose of BrEA. The results showed significant increases in immune cell populations and cytokine expression profiles associated with Th1 responses. The patients in this protocol initially had CD4 counts of at least 200 per mm³ and a serum HIV RNA load of 5,000 to 1 x 10⁶ RNA copies/mL. After dosing with one course of BrEA (5 consecutive daily i.m. injections), all patients showed increases in levels of immune cells including activated CD8 T cells (e.g., CD8⁺, CD69⁺, CD25⁻), LAK cells (e.g., CD8⁺, CD16⁺, CD38⁺), NK cells (e.g., CD8⁻, CD16⁺), ADCC cells (e.g., CD8⁻, CD16⁺) and dendritic cells (Lin⁻, HLA-DR⁺, CD11c⁺ or Lin⁻, HLA-DR⁺, CD123⁺). Average CD4 IL-10 production dropped from a median of 66% to 4% of the cells, while CD4 IFNγ went from a median of 8% to 63%, leading to a Th2 to a Th1 shift in cytokine production.

In the diagrams below baseline data is indicated by "BL" or by "pre".

| **Increased immunophenotypes after BrEA therapy** | | | | |
|---|---|---|---|---|
| Phenotype | Baseline^{a} | Course 1 | Course 2 | Course 3 |
| CD8+CD69+CD25- | 18 | 54 | 56 | 75 |
| n= | (13) | (13) | (9) | (4) |
| ^{b}p= | | <0.001 | <0.001 | 0.04 |
| | | | | |
| CD8+CD16+CD38+ | 8 | 27 | 28 | 25 |
| n= | (10) | (10) | (4) | (4) |
| p= | | <0.001 | 0.047 | 0.02 |
| | | | | |
| CD8-CD16+ | 53 | 253 | 288 | 249 |
| n= | (12) | (12) | (4) | (2) |
| p= | | <0.001 | 0.02 | 0.04 |
| | | | | |
| Lin- HLA-DR+ | | | | |
| CD11c+/CD123+ | 3.2 | 17.7 | 11.4 ^{c} | 14.7^{c} |
| n= | (10) | (10) | (5) | (4) |
| p= | | <0.001 | 0.02 | 0.04 |
| | | | | |
| IL2+CD4^{d} | 3.14^{e} | 29.25 | 31.42 | 13.59 |
| n= | 13 | 13 | 3 | 4 |
| p= | | <0.001 | 0.09 | 0.04 |
| | | | | |
| IL10+CD4^{d} | 66 | 20.9 | 8.9 | 15.3 |
| n= | 13 | 13 | 5 | 3 |
| p= | | 0.005 | 0.005 | 0.03 |
| | | | | |
| Th1 Response^{d} | 17 | 66 | 64 | 53 |
| n= | (13) | (13) | (5) | (5) |
| p= | | 0.001 | 0.033 | 0.025 |

| | | | | |
|---|---|---|---|---|
| ^{a}Median values of cells/µL | | | | |
| ^{b}paired value t test | | | | |
| ^{c}Test not available at baseline for patients receiving second and third courses, baseline value from initiation of 2^{nd} course = 6.4 | | | | |
| ^{d}% of CD4 | | | | |
| ^{e}Baseline values from day 8 (preceding the first five-day treatment) | | | | |

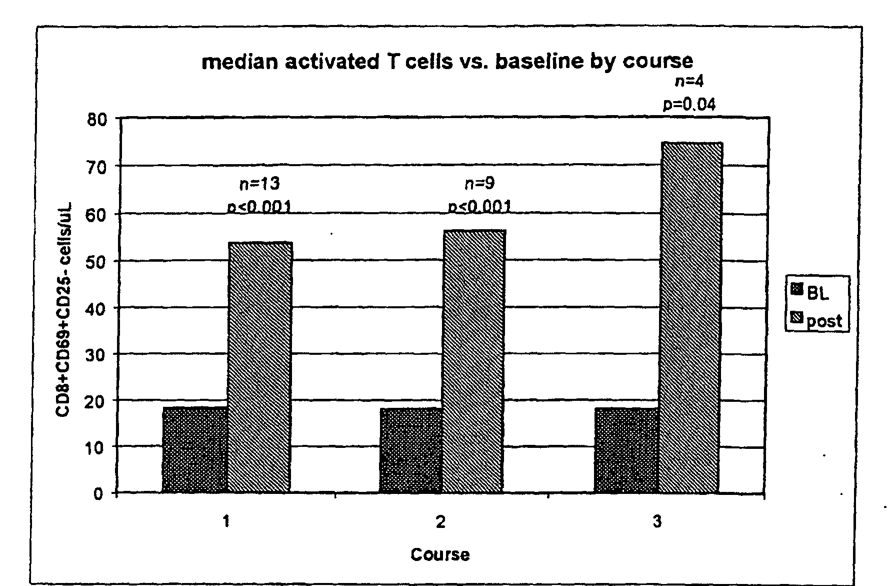

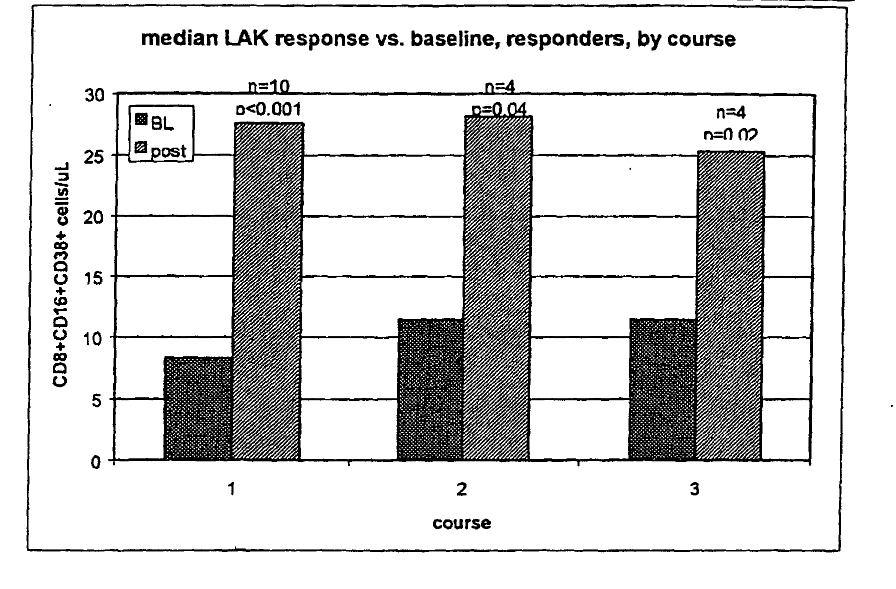

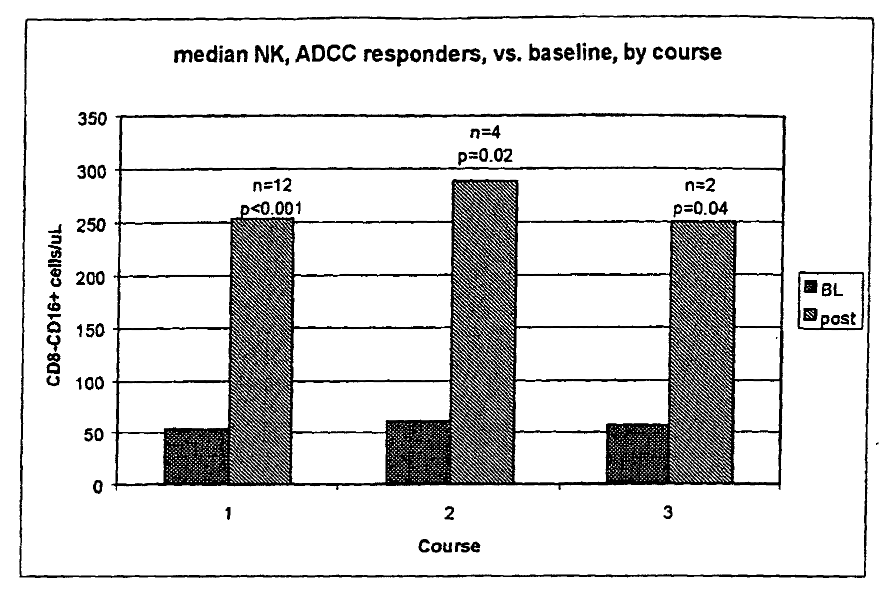

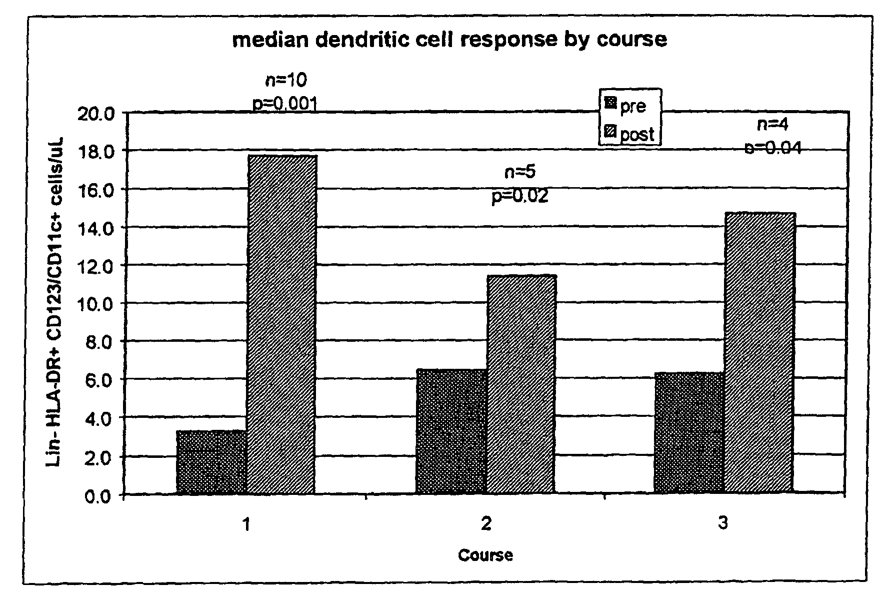

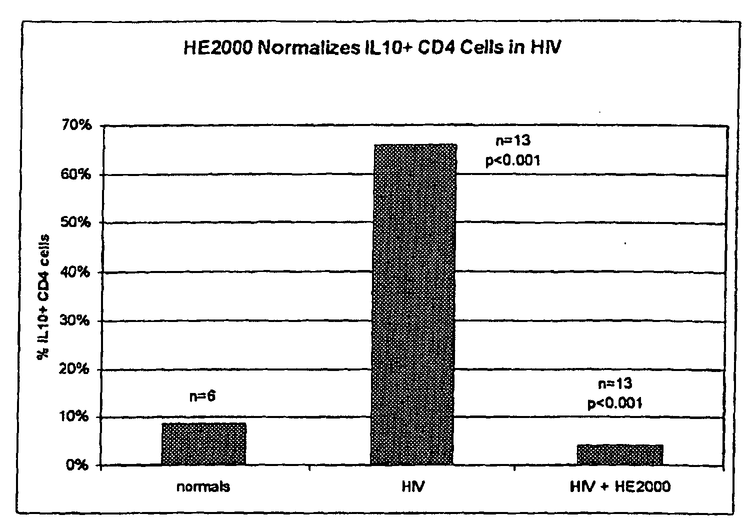

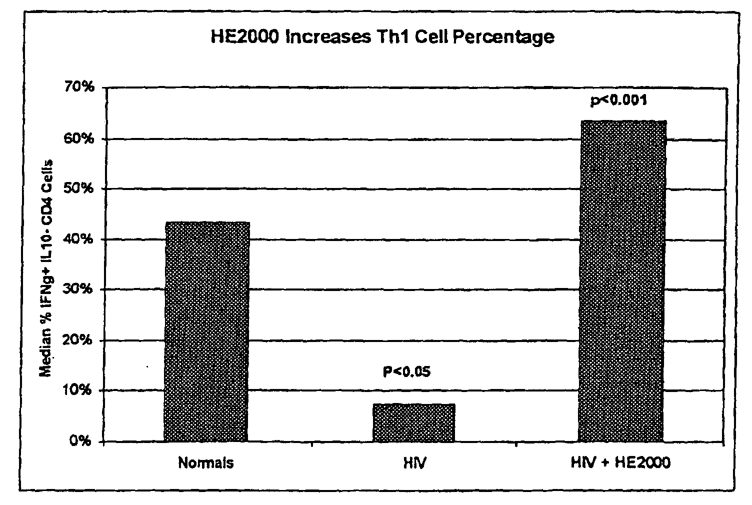

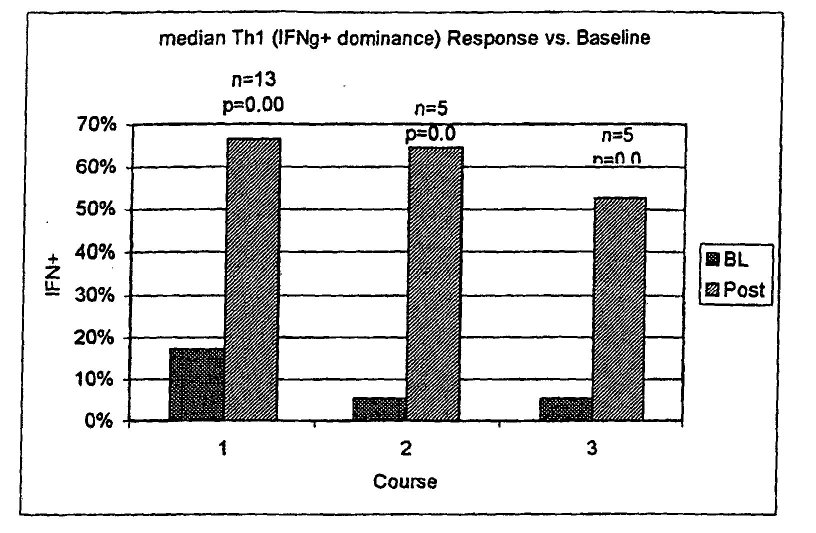

**Example 21. Treatment of symptoms of HIV infection.** Two HIV infected patients with chronic diarrhea were dosed with BrEA as follows. A BrEA formulation (40 mg/mL BrEA in 25% v/v PEG 300, 12.5% v/v ethanol, 5% v/v benzyl benzoate, ∼57.5% v/v propylene glycol) was delivered subcutaneously. The patients received 60 mg of BrEA in 1.5 mL daily for 10 days. During the period of dosing, the diarrhea ceased. After the 10-day dosing period ended, diarrhea resumed. In other patients receiveing oral BrEA, diarrhea also went into remission.

**Example 22. Subcutaneous formulation.** A BrEA formulation was prepared essentially as described herein. The formulation contained 50 mg/mL BrEA, 40% v/v PEG 200, 2% v/v benzyl alcohol, 2% v/v benzyl benzoate and ∼ 66% v/v propylene glycol (qs). The formulation is particularly suitable for subcutaneous administration of the compound.

**Example 23. Preparation of BrEA hemihydrate - procedure 1.** Crude BrEA was prepared by bromination of epiandrosterone, followed by crystallization from methanol. The hemihydrate was prepared by dissolving 25 g of crude BrEA in 75 mL of refluxing ethanol with moderate agitation. To the BrEA solution 12.5 mL of water was slowly added while maintaining the solution at reflux with agitation. Agitation of the solution was maintained and the solution was then allowed to cool to about 20-25°C and kept at about 20-25°C for about 15 minutes to obtain a suspension of BrEA hemihydrate crystals. The crystals were recovered by filtration, washed with a solution of 25 mL of water:ethanol (5:1 v/v) at about 20-25°C and then vacuum dried for about 13 hours at 50-60°C until the product weight was constant The crystals were primarily rod and needle shaped, with smaller amounts of other shapes such as tablets.

The procedure gave 22.5 g of BrEA hemihydrate (yield 90%) with a water content of 2.6% w/w by KF analysis, a purity of 100% by HPLC area analysis, an FTIR spectrum with carbonyl peaks at 1741 cm⁻¹ and 1752 cm⁻¹. The FTIR scan of anhydrous BrEA shows a single carbonyl peak at 1749 cm⁻¹. The DSC scan showed three endotherms. One had a broad shallow peak with an onset at about 109-110°C and ending at about 150°C. This broad DSC peak is consistent with the loss of water from the hemihydrate crystals as the temperature of the sample increased. The second endotherm at about 83-100°C is consistent with the loss of the small amount of residual ethanol from the sample. A DSC scan of anhydrous BrEA does not have the broad endotherm that is observed with the hemihydrate. Also consistent with the loss of water from the hemihydrate over the 100-150°C range is a sharp third endotherm peak in the hemihydrate DSC scan at about 163-164°C, which is the melting point of anhydrous BrEA. The FTIR was obtained using USP method <197>, where the BrEA hemihydrate sample was prepared in KBr. The DSC thermogram was obtained by scanning from 25°C to 250°C with a heating rate of 10°C/minute.

**Example 24. Preparation of BrEA hemihydrate - procedure 2.** The hemihydrate was prepared by dissolving 10 g of crude BrEA in 40 mL of refluxing acetone with moderate agitation. To the BrEA solution 4.0 mL of water was slowly added while maintaining the solution at reflux with agitation. Agitation of the solution was maintained and the solution was then allowed to cool to about 20-25°C and kept at about 20-25°C for about 15 minutes to obtain a suspension of BrEA hemihydrate crystals. The crystals were recovered by filtration, washed with a solution of 6.0 mL of water:acetone (10:1 v/v) at about 20-25°C and then vacuum dried overnight (about 13-15 hours) at 50-60°C until the product weight was constant. The procedure gave 7.0 g of BrEA hemihydrate (yield 70%) with a water content of 2.6% w/w by KF analysis and an FTIR spectrum with carbonyl peaks at 1741 cm⁻¹ and 1752 cm⁻¹.

**Example 25. Analysis of BrEA hemihydrate particle size.** BrEA hemihydrate crystals were prepared essentially as described herein and sized using a particle sizing apparatus (Malvern instruments). The analysis model used was for a polydisperse sample and a volume distribution type. The analysis showed a range of crystal diameter sizes from about 0.5 µm to about 880 µm. About 90% of the crystals had a diameter of about 20 µm to about 220 µm and the majority of the crystals had a diameter of about 30-200 µm. The mean crystal diameter was about 93 µm. The specific surface area of the crystals was about 0.25 m²/g.

## Claims

1. 16α-Bromo-3β-hydroxy-5α-androstan-17-one hemihydrate.

2. 16α-bromo-3β-hydroxy-5α-androstan-17-one hemihydrate according to claim 1, **characterized by** one or more of (1) an absorption endotherm onset as measured by differential scanning calorimetric analysis of about 100°C +/-2°C, (2) two carbonyl absorption bands at about 1741 cm⁻¹ and 1750 cm⁻¹ as measured by Fourier transform infrared absorption spectroscopy, (3) a water content of about 2.4% w/w to about 2.6% w/w as measured by Karl Fisher titration and (4) 1, 2, 3 or more of the XRD peaks at Theta values of about 17.8 ° +/- 0.2°, 23.8 +/- 0.2°, 24.2 ° +/- 0.2°, 26.9 °-27.2 ° +/- 0.2°, 28.6 ° +/-0.2°, 30.1 *°* +/- 0.2° and 32.2 ° +/- 0.2° Theta wherein the XRD peaks are determined using Cu-Kα radiation.

3. 16α-bromo-3β-hydroxy-5α-androstan-17-one hemihydrate of claim 1, **characterized in that** it is milled to an average particle size of 0.01-200 µm.

4. 16α-bromo-3β-hydroxy-5α-androstan-17-one hemihydrate of claim 1, **characterized in that** is milled to an average particle size of 0.1-10 µm.

5. A composition comprising 16α-bromo-3β-hydroxy-5α-androstan-17-one hemihydrate and an excipient suitable for human pharmaceutical use or for veterinary use.

6. The composition of claim 5 in the form of a unit dosage that comprises 3 to 1000 mg of 16α-bromo-3β-hydroxy-5α-androstan-17-one hemihydrate.

7. The composition of claim 5 or 6 in the form of a tablet, capsule, lozenge, solution, suspension, gel or colloid.

8. The composition of claim 5 or 6 in the form of a tablet, capsule, lozenge, solution, suspension, gel or colloid wherein the unit dosage is suitable for oral, parenteral, topical, buccal or sublingual administration to a subject.

9. A method to make 16α-bromo-3β-hydroxy-5α-androstan-17-one hemihydrate comprising contacting water with a solution comprising 16α-bromo-3β-hydroxy-5α-androstan-17-one and a C1-C6 alcohol.

10. The method of claim 9 wherein the C1-C6 alcohol is ethanol.

11. The method of claim 9 wherein the solution comprises 5-25% w/w water, 30-45% w/w ethanol and 30-45% w/w of 16α-bromo-3β-hydroxy-5α-androstan-17-one.

12. The method of claim 9 wherein the solution comprises 18-22% w/w water, 37-43% w/w ethanol and 37-43% w/w of 16α-bromo-3β-hydroxy-5α-androstan-17-one.

13. The method of claim 12 wherein the solution is at a temperature of about -20°C to about 45°C.

14. Use of 16α-bromo-3β-hydroxy-5α-androstan-17-one hemihydrate to prepare a medicament for the treatment of a subject having or susceptible to an immunosuppression condition or an unwanted immune response, either or both of which are associated with (1) a pathogen infection selected from a viral infection, an intracellular bacterial infection, an extracellular bacterial infection, a fungal infection, a yeast infection, an extracellular parasite infection, an intracellular parasite infection, a protozoan parasite infection and a multicellular parasite infection, (2) an autoimmune disease, (3) a malignancy or a precancer, (4) a chemotherapy, a radiation therapy, an immunosuppressive therapy, an anti-infective agent therapy, a wound, a burn, the presence of an immunosuppressive molecule, gastrointestinal irritation or inflammation, or (5) any combination of the foregoing.

15. Use according to claim 14, wherein immunosuppression condition or unwanted immune response, is associated with malignancy or a precancer.

16. Use according to claim 15, wherein the malignancy or precancer is ovarian cancer, breast cancer, prostate cancer, benign prostatic hyperplasia, a glioma, a lymphoma, a leukemia, colon cancer, a sarcoma, non-small cell lung cancer, bronchogenic carcinoma, renal cell cancer, renal cell carcinoma, melanoma, pancreatic or gastric adenocarcinoma, human papillomavirus-associated cervical intraepithelial neoplasia, cervical carcinoma, hepatoma, cutaneous T-cell lymphoma or a cancer or precancer arising in the throat, esophagus, stomach, intestine, colon, ovary, lung, breast or central nervous system.

17. Use according to claim 14, wherein immunosuppression condition or unwanted immune response, is associated with a viral infection, an intracellular bacterial infection, an extracellular bacterial infection, a fungal infection, a yeast infection, an extracellular parasite infection, an intracellular parasite infection, a protozoan parasite infection and a multicellular parasite infection.

18. Use according to claim 17, wherein the viral infection, intracellular bacterial infection, extracellular bacterial infection, fungal infection, yeast infection, an extracellular parasite infection, intracellular parasite infection, protozoan parasite infection or multicellular parasite infection is an infection caused by a group, species or genus selected from a Retrovirus, a Togavirus, a Flavivirus, a Hepadnavirus, a Herpesvirus, a Papillomavirus, HIV-1, HIV-2, HHV-6, HHV-8, hepatitis B virus, hepatitis C virus, HSV-1, HSV-2, CMV, *Escherichia, Haemophilus, Legionella pneumonia, Listeria, Mycoplasma, Neisseria, Pseudomonas, Rickettsia, Salmonella, Shigella, Streptococcus, Staphylococcus, Yersinia, Pneumocystis, Candida, Cryptococcus, Aspergillus, Cryptosporidium, Toxoplasma, Trichomonas, Chlamidya, Pneumocystis, Trypanosoma, Leishmania, Plasmodium*, a gastrointestinal helminth, a Microsporidium parasite or an Isosporum parasite.

19. Use according to claim 18 wherein a medicament comprising an antiviral agent, an antibacterial agent, an antifungal agent or an antiparasitic agent is optionally administered in conjunction with the medicament of claim 14.

20. Use according to claim 19 wherein the antiviral agent, antibacterial agent or antifungal agent is a reverse transcriptase inhibitor, a protease inhibitor, an antibiotic, a fusion inhibitor, γ-interferon, amantadine, rimantadine, ribavirin, chloroquine or a chloroquine analog.

21. Use according to claim 19 or 20 wherein the antiviral agent is selected from AZT, 3TC, D4T, ddl, ddC, adefovir dipivoxil, 9-[2-(R)-[[bis[[(isopropoxycarbonyl)-oxy]methoxy]phosphinoyl]methoxy]propyl]adenine, (*R*)-9-[2-(phosphonomethoxy)propyl]adenine, adefovir, indinavir, nelfinavir, ritonavir, crixivan, sequanavir and hydroxyurea.

22. Use according to claim 21 wherein the antibacterial agent or antifungal agent is selected from amphotericin B, fluconazole, clotrimazole, isoniazid, dapsone, rifampin, cycloserine, erythromycin, a tetracycline antibiotic, vancomycin, ethambutol, pyrazinamide, a flurorquinolone optionally selected from ciprofloxacin and norfloxacin, a cephalosporin antibiotic, a β-lactam antibiotic or an aminoglycoside antibiotic optionally selected from streptomycin, kanamycin and tobramycin.

23. Use according to claim 14, wherein the immunosuppression condition or unwanted immune response, is associated with inflammation.

24. Use according to claim 23, wherein the inflammation is allergic bronchopulmonary aspergillosis in cystic fibrosis patients, atopic asthma, allergic respiratory disease, allergic rhinitis, atopic dermatitis, subepithelial fibrosis in airway hyperresponsiveness, chronic sinusitis, perennial allergic rhinitis, Crohn's disease, ulcerative colitis, inflammatory bowel disease or fibrosing alveolitis.

25. Use according to claim 14, wherein the immunosuppression condition or unwanted immune response, is associated with an autoimmune disease.

26. Use according to claim 25, wherein the autoimmune disease is systemic lupus erythematosus, osteoporosis, multiple sclerosis, myasthenia gravis, Grave's disease, rheumatoid arthritis or osteoarthritis.

27. Use according to claim 14-26 wherein the medicament is administered to the subject using an intermittent dosing protocol.

28. Use according to claim 27 wherein the subject is a mammal.

29. Use of 16α-bromo-3β-hydroxy-5α-androstan-17-one hemihydrate to prepare a medicament for the modulation of a cytokine or interleukin that facilitates Th1 immune or Th2 immune responses in a subject.

30. Use according to claim 29 wherein the cytokine or interleukin is IL-1, IL-2, IL-4, IL-6, IL-8, IL-10, IL-12, γIFN or TNFα.

31. Use according to claim 30 wherein the cytokine or interleukin is IL-12.

32. Use according to claim 30 wherein the cytokine or interleukin is IL-1.

33. Use according to claim 30 wherein the cytokine or interleukin is IL-6.

34. Use according to claim 30 wherein the cytokine or interleukin is IL-10.

35. Use according to claim 30 wherein the cytokine or interleukin is TNFα.

36. Use according to claim 29-35 wherein the medicament is administered to the subject using an intermittent dosing protocol and the subject is a mammal.

37. Use of 16α-bromo-3β-hydroxy-5α-androstan-17-one hemihydrate to prepare a medicament for the enhancement of Lin⁻ HLA-DR⁺ CD123⁺ dendritic cells, Lin⁻ HLA-DR⁺ CD11c⁺ dendritic cells, CD8⁺CD16⁺CD38⁺ lymphokine activated killer cells, CD8⁻CD16⁺CD38⁺ lymphokine activated killer cells, CD8⁻ CD16⁺ natural killer cells, CD8⁺CD16⁺ natural killer cells, CD8⁻CD16⁺ cells that mediate antibody-dependent cell-mediated cytotoxicity, CD8⁺CD16⁺ cells that mediate antibody-dependent cell-mediated cytotoxicity in a subject, CD45RA⁺ T-cells or CD45⁺RO⁺ T-cells in a subject.

38. Use according to claim 37 wherein the subject has an immunosuppression condition or an unwanted immune response, either or both of which are associated with (1) a pathogen infection selected from a viral infection, an intracellular bacterial infection, an extracellular bacterial infection, a fungal infection, a yeast infection, an extracellular parasite infection, an intracellular parasite infection, a protozoan parasite infection or a multicellular parasite infection, (2) an autoimmune disease, (3) a malignancy or a precancer, (4) a chemotherapy, a radiation therapy, an immunosuppressive therapy, an anti-infective agent therapy, a wound, a burn, the presence of an immunosuppressive molecule, gastrointestinal irritation or inflammation, or (5) any combination of the foregoing.

39. Use according to claim 37 or 38 wherein the medicament is administered to the subject using an intermittent dosing protocol and the subject is a mammal.

40. Use according to claim 37 or 38, wherein immunosuppression condition or unwanted immune response, is associated with malignancy or a precancer.

41. Use according to claim 40, wherein the malignancy or precancer is ovarian cancer, breast cancer, prostate cancer, benign prostatic hyperplasia, a glioma, a lymphoma, a leukemia, colon cancer, a sarcoma, non-small cell lung cancer, bronchogenic carcinoma, renal cell cancer, renal cell carcinoma, melanoma, pancreatic or gastric adenocarcinoma, human papillomavirus-associated cervical intraepithelial neoplasia, cervical carcinoma, hepatoma, cutaneous T-cell lymphoma or a cancer or precancer arising in the throat, esophagus, stomach, intestine, colon, ovary, lung, breast or central nervous system.

42. Use according to claim 37 or 38, wherein immunosuppression condition or unwanted immune response, is associated with a viral infection, an intracellular bacterial infection, an extracellular bacterial infection, a fungal infection or a yeast infection.

43. Use according to claim 42, wherein the viral infection, intracellular bacterial infection, extracellular bacterial infection, fungal infection or yeast infection is an infection caused by a group, species or genus selected from a retrovirus, a flavivirus, a papillomavirus, HIV-1, HIV-2, HHV-6, HHV-8, hepatitis B virus, hepatitis C virus, HSV-1, HSV-2, CMV, *Escherichia, Legionella pneumonia, Listeria, Mycoplasma, Neisseria, Pseudomonas, Rickettsia, Salmonella, Shigella, Streptococcus, Staphylococcus, Yersinia, Trichomonas, Chlamidya, Pneumocystis, Candida, Cryptococcus, Aspergillus, Cryptosporidium, Toxoplasma,* and *Pneumocystis*.

44. Use according to claim 37 or 38, wherein the immunosuppression condition or unwanted immune response, is associated with inflammation.

45. Use according to claim 44, wherein the inflammation is allergic bronchopulmonary aspergillosis in cystic fibrosis patients, atopic asthma, allergic respiratory disease, allergic rhinitis, atopic dermatitis, subepithelial fibrosis in airway hyperresponsiveness, chronic sinusitis, perennial allergic rhinitis, Crohn's disease, ulcerative colitis, inflammatory bowel disease or fibrosing alveolitis.

46. Use according to claim 37 or 38, wherein the immunosuppression condition or unwanted immune response, is associated with an autoimmune disease.

47. Use according to claim 46, wherein the autoimmune disease is systemic lupus erythematosus, osteoporosis, multiple sclerosis, myasthenia gravis, Grave's disease, rheumatoid arthritis or osteoarthritis.

48. Use of 16α-bromo-3β-hydroxy-5α-androstan-17-one hemihydrate to prepare a medicament for the modulation of a subject's innate immunity or the enhancement of a subject's Th1 immune responses.

49. Use according to claim 48 wherein the subject has an innate immunity suppression condition or a suppressed Th1 immune response.

50. Use of 16α-bromo-3β-hydroxy-5α-androstan-17-one hemihydrate to prepare a medicament to increase the number of neutrophils, dendritic cells, monocytes, macrophages or NK cells in a subject's blood.

51. Use according to claim 50 wherein the subject has an innate immunity suppression condition or a suppressed Th1 immune response.

52. Use according to claim 51 wherein the innate immunity suppression condition or the suppressed Th1 immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a burn, the presence of an immunosuppressive molecule or any combination of the foregoing.

53. Use according to claim 52 wherein the subject has or is subject to developing an innate immunity suppression condition or a suppressed Th1 immune response that is associated with a chemotherapy, a radiation therapy, an immunosuppressive therapy or an anti-infective agent therapy.

54. Use according to claim 53 wherein the innate immunity suppression condition or suppressed Th1 immune response is associated with a chemotherapy, a radiation therapy or an immunosuppressive therapy, wherein the therapy is optionally selected from adriamycin treatment, cisplatin treatment, mitomycin C treatment, amphotericin B treatment, γ-radiation therapy, nucleoside analog treatment for viral infection or cancer, cyclosporin treatment and corticosteroid treatment.

55. Use according to claim 50, 51 or 52 wherein the dendritic cells are Lin⁻ HLA-DR⁺ CD123⁺ dendritic cells or Lin⁻ HLA-DR⁺ CD11c⁺ dendritic cells.

56. 16α-Bromo-3β-hydroxy-5α-androstan-17-one hemihydrate for use as a medicament.

## Patentansprüche

1. 16α-Brom-3β-hydroxy-5α-androstan-17-onhemihydrat.

2. 16α-Brom-3β-hydroxy-5α-androstan-17-onhemihydrat nach Anspruch 1, **gekennzeichnet durch** eine oder mehrere der folgenden Bedingungen (1) einen mittels Differentialscanningcalorimetrieanalyse gemessenen Absorptionsendothermenbeginn von ungefähr 100 °C +/- 2 °C, (2) zwei Carbonyl-Absorptionsbanden bei ungefähr 1741 cm⁻¹ und 1750 cm⁻¹, gemessen mittels Fourier-transform-Infrarotabsorptionsspektroskopie, (3) einen Wassergehalt von ungefähr 2,4 Gew.-% bis ungefähr 2,6 Gew.-%, gemessen mittels Karl-Fisher-Titration und (4) 1, 2, 3 oder mehr der XRD-Peaks bei Theta-Werten von ungefähr 17,8° +/- 0,2°, 23,8° +/- 0,2°, 24,2° +/- 0,2°, 26,9° - 27,2° +/- 0,2°, 28,6° +/-0,2°, 30,1° +/- 0,2° und 32,2° +/- 0,2° Theta, wobei die XRD-Peaks unter Verwendung einer Cu-Kα-Strahlung bestimmt wurden.

3. 16α-Brom-3β-hydroxy-5α-androstan-17-onhemihydrat nach Anspruch 1, **dadurch gekennzeichnet, dass** es auf eine mittlere Teilchengröße von 0,01-200 µm gemahlen ist.

4. 16α-Brom-3β-hydroxy-5α-androstan-17-onhemihydrat nach Anspruch 1, **dadurch gekennzeichnet, dass** es auf eine mittlere Teilchengröße von 0,1-10 µm gemahlen ist.

5. Zusammensetzung, umfassend 16α-Brom-3β-hydroxy-5α-androstan-17-onhemihydrat und einen Arzneimittelträger, der für eine humanpharmazeutische Anwendung oder für eine Veterinäranwendung geeignet ist.

6. Zusammensetzung nach Anspruch 5 in der Form einer Unit Dosage, die 3 bis 1000 mg an 16α-Brom-3β-hydroxy-5α-androstan-17-onhemihydrat umfasst.

7. Zusammensetzung nach Anspruch 5 oder 6 in der Form einer Tablette, einer Kapsel, einer Pastille, einer Lösung, einer Suspension, eines Gels oder eines Kolloids.

8. Zusammensetzung nach Anspruch 5 oder 6 in der Form einer Tablette, einer Kapsel, einer Pastille, einer Lösung, einer Suspension, eines Gels oder eines Kolloids, wobei die Unit Dosage geeigent ist für eine orale, parenterale, topische, bukkale oder sublinguale Verabreichung an einen Patienten.

9. Verfahren zur Herstellung von 16α-Brom-3β-hydroxy-5α-androstan-17-onhemihydrat, umfassend ein In-Kontakt-Bringen von Wasser mit einer Lösung, die 16α-Brom-3β-hydroxy-5α-androstan-17-on und einen C1-C6-Alkohol umfasst.

10. Verfahren nach Anspruch 9, wobei der C1-C6-Alkohol Ethanol ist.

11. Verfahren nach Anspruch 9, wobei die Lösung 5-25 Gew.-% an Wasser, 30-45 Gew.-% an Ethanol und 30-45 Gew.-% an 16α-Brom-3β-hydroxy-5α-androstan-17-on umfasst.

12. Verfahren nach Anspruch 9, wobei die Lösung 18-22 Gew.-% an Wasser, 37-43 Gew.-% an Ethanol und 37-43 Gew.-% an 16α-Brom-3β-hydroxy-5α-androstan-17-on umfasst.

13. Verfahren nach Anspruch 12, wobei die Lösung eine Temperatur von ungefähr -20 °C bis ungefähr 45 °C besitzt.

14. Verwendung von 16α-Brom-3β-hydroxy-5α-androstan-17-onhemihydrat zur Herstellung eines Medikaments zur Behandlung eines Patienten, der einen Immunosuppressions-Zustand oder eine unerwünschte Immunantwort aufweist oder dafür anfällig ist, wovon eine oder beide in Verbindung stehen mit (1) einer pathogenen Infektion, die ausgewählt ist aus einer Virusinfektion, einer intrazellulären Bakterieninfektion, einer extrazellulären Bakterieninfektion, einer Pilzinfektion, einer Hefeinfektion, einer extrazellulären Parasiteninfektion, einer intrazellulären Parasiteninfektion, einer Protozoenparasiteninfektion und einer multizellulären Parasiteninfektion, (2) einer Autoimmunerkrankung, (3) einer Malignität oder einer Präkanzerose, (4) einer Chemotherapie, einer Strahlentherapie, einer Immunsuppressionstherapie, einer Antiinfektivatherapie, einer Wunde, einer Verbrennung, der Gegenwart eines immunosuppressiven Moleküls, einer gastrointestinalen Irritation oder Entzündung oder (5) irgendeiner Kombination des vorher erwähnten.

15. Verwendung nach Anspruch 14, wobei der Immunosuppressions-Zustand oder die unerwünschte Immunantwort in Zusammenhang stehen mit einer Malignität oder einer Präkanzerose.

16. Verwendung nach Anspruch 15, wobei die Malignität oder Präkanzerose Ovarialkrebs, Brustkrebs, Prostatäkrebs, gutartige Prostatahyperplasie, ein Gliom, ein Lymphom, eine Leukämie, Dickdarmkrebs, ein Sarkom, nicht-kleinzelliges Lungenkarzinom, Bronchialkarzinom, Nierenzellenkrebs, Nierenzellenkarzinom, Melanom, Pankreas- oder Magenadenokarzinom, mit dem Human-Papilloma-Virus in Zusammenhang stehende zervikale intraepitheliale Neoplasie, Zervixkarzinom, Hepatom, kutanes T-Zell-Lymphom oder ein Krebs oder eine Präkanzerose ist, die im Rachen, Esophagus, Magen, Darm, Dickdarm, Ovarium, Lunge, Brust oder Zentralnervensystem entsteht.

17. Verwendung nach Anspruch 14, wobei der Immunosuppressions-Zustand oder die unerwünschte Immunantwort in Zusammenhang steht mit einer Virusinfektion, einer intrazellulären Bakterieninfektion, einer extrazellulären Bakterieninfektion, einer Pilzinfektion, einer Hefeinfektion, einer extrazellulären Parasiteninfektion, einer intrazellulären Parasiteninfektion, einer Protozoenparasiteninfektion und einer multizellulären Parasiteninfektion.

18. Verwendung nach Anspruch 17, wobei die Virusinfektion, intrazelluläre Bakterieninfektion, extrazelluläre Bakterieninfektion, Pilzinfektion, Hefeinfektion, eine extrazelluläre Parasiteninfektion, intrazelluläre Parasiteninfektion, Protozoenparasiteninfektion oder multizelluläre Parasiteninfektion eine Infektion ist, die verursacht wird durch eine Gruppe, Spezies oder Gattung, ausgewählt aus einem Retrovirus, einem Togavirus, einem Flavivirus, einem Hepadnavirus, einem Herpesvirus, einem Papiiloma-virus, HIV-1, HIV-2, HHV-6, HHV-8, Hepatitis-B-Virus, Hepatitis-C-Virus, HSV-1, HSV-2, CMV, *Escherichia, Haemophilus, Legionella pneumonia, Listeria, Mycoplasma, Neisseria, Pseudomonas, Rickettsia, Salmonella, Shigella, Streptococcus, Staphylococcus, Yersinia, Pneumocystis, Candida, Cryptococcus, Aspergillus, Cryptosporidium, Toxoplasma, Trichomonas,* *Chlamidya, Pneumocystis, Trypanosoma, Leishmania, Plasmodium*, einem gastrointestinalen Helminthen, einem Microsporidiumparasiten oder einem Isosporumparasiten.

19. Verwendung nach Anspruch 18, wobei gegebenenfalls in Kombination mit dem Medikament gemäß Anspruch 14 ein Medikament verabreicht wird, das ein antivirales Mittel, ein antibakterielles Mittel, ein antifungales Mittel oder ein antiparasitäres Mittel umfasst.

20. Verwendung nach Anspruch 19, wobei das antivirale Mittel, antibakterielle Mittel oder antifungale Mittel ein Inhibitor der reversen Transcriptase, ein Proteasinhibitor, ein Antibiotikum, ein Fusionsinhibitor, γ-Interferon, Amantadin, Rimantadin, Ribavirin, Chlorochin oder ein Chlorochinanalog ist.

21. Verwendung nach Anspruch 19 oder 20, wobei das antivirale Mittel ausgewählt ist aus AZT, 3TC, D4T, ddI, ddC, Adefovir Dipivoxil, 9-[2-(*R*)-[[Bis[[(isopropoxycarbonyl)oxy]methoxy] phosphinoyl]methoxy] propyl] adenin, (*R*)-9-[2-(Phosphonomethoxy)propyl]adenin, Adefovir, Indinavir, Nelfinavir, Ritonavir, Crixivan, Sequanavir und Hydroxyharnstoff.

22. Verwendung nach Anspruch 21, wobei das antibakterielle Mittel oder antifungale Mittel ausgewählt ist aus Amphotericin B, Fluconazol, Clotrimazol, Isoniazid, Dapson, Rifampin, Cycloserin, Erythromycin, einem Tetracyclin-Antibiotikum, Vancomycin, Ethambutol, Pyrazinamid, einem Fluorchinolon, das gegebenenfalls ausgewählt ist aus Ciprofloxacin und Norfloxacin, einem Cephalosporin-Antibiotikum, einem β-Lactam-Antibiotikum oder einem Aminoglycosid-Antibiotikum, das gegebenenfalls ausgewählt ist aus Streptomycin, Kanamycin und Tobramycin.

23. Verwendung nach Anspruch 14, wobei der Immunosuppressions-Zustand oder die unerwünschte Immunantwort in Zusammenhang stehen mit einer Entzündung.

24. Verwendung nach Anspruch 23, wobei die Entzündung eine allergische bronchopulmonale Aspergillose bei Patienten mit zystischer Fibrose, atopisches Asthma allergische Atemwegserkrankung, allergische Rhinitis, atopische Dermatitis, subepitheliale Fibrose bei Luftweg-Überreagibilität, chronischer Sinusitis, perenniale allergische Rhinitis, Crohn-Krankheit, Colitis ulcerosa, entzündliche Darmerkrankung oder fibrosierende Alveolitis ist.

25. Verwendung nach Anspruch 14, wobei der Immunosuppressions-Zustand oder die unerwünschte Immunantwort in Zusammenhang mit einer Autoimmunerkrankung stehen.

26. Verwendung nach Anspruch 25, wobei die Autoimmunerkrankung eine systemische Lupus erythematodes, Osteoporose, Multiple Sclerose, Myasthenia gravis, Graves-Krankheit, rheumatoide Arthritis oder Osteoarthritis ist.

27. Verwendung nach Anspruch 14-26, wobei das Medikament dem Patienten unter Verwendung eines intermittierenden Dosierungsprotokolls verabreichte wird.

28. Verwendung nach Anspruch 27, wobei der Patient ein Säugetier ist.

29. Verwendung von 16α-Brom-3β-hydroxy-5α-androstan-17-onhemihydrat zur Herstellung eines Medikaments zur Modulation eines Zytokins oder Interleukins, das Th1-Immun- oder Th2-Immunantworten in einem Patienten fördert.

30. Verwendung nach Anspruch 29, wobei das Zytokins oder Interleukin IL-1, IL-2, IL-4, IL-6, IL-8, IL-10, IL-12, γIFN oder TNFα ist.

31. Verwendung nach Anspruch 30, wobei das Zytokins oder Interleukin IL-12 ist.

32. Verwendung nach Anspruch 30, wobei das Zytokins oder Interleukin IL-1 ist.

33. Verwendung nach Anspruch 30, wobei das Zytokins oder Interleukin IL-6 ist.

34. Verwendung nach Anspruch 30, wobei das Zytokins oder Interleukin IL-10 ist.

35. Verwendung nach Anspruch 30, wobei das Zytokins oder Interleukin TNFα ist.

36. Verwendung nach Anspruch 29-35, wobei das Medikament dem Patienten unter Verwendung eines intermittierenden Dosierungsprotokolls verabreichte wird und der Patient ein Säugetier ist.

37. Verwendung of 16α-Brom-3β-hydroxy-5α-androstan-17-on-hemihydrat zur Herstellung eines Medikaments zur Steigerung von Lin⁻HLA-DR⁺CD123⁺ dendritischen Zellen, Lin⁻HLA-DR⁺CD11c⁺ dendritischen Zellen, CD8⁺CD16⁺CD38⁺ Lymphokin-aktivierten Killerzellen, CD8⁻CD16⁺CD38⁺ Lymphokin-aktivierten Killerzellen; CD8⁻CD16⁺ natürlichen Killerzellen, CD8⁺CD16⁺ natürlichen Killerzellen, CD8⁻CD16⁺ Zellen, die eine Antikörper-abhängige zellvermittelte Zytotoxizität vermitteln, CD8⁺CD16⁺ Zellen, die eine Antikörper-abhängige zellvermittelte Zytotoxizität in einem Patienten vermitteln, CD45RA⁺ T-Zellen oder CD45⁺RO⁺ T-Zellen in einem Patienten.

38. Verwendung nach Anspruch 37, wobei der Patient einen Immunosuppressions-Zustand oder eine unerwünschte Immunantwort aufweist oder dafür anfällig ist, wovon eine oder beide in Verbindung stehen mit (1) einer pathogenen Infektion, die ausgewählt ist aus einer Virusinfektion, einer intrazellulären Bakterieninfektion, einer extrazellulären Bakterieninfektion, einer Pilzinfektion, einer Hefeinfektion, einer extrazellulären Parasiteninfektion, einer intrazellulären Parasiteninfektion, einer Protozoenparasiteninfektion und einer multizellulären Parasiteninfektion, (2) einer Autoimmunerkrankung, (3) einer Malignität oder einer Präkanzerose, (4) einer Chemotherapie, einer Strahlentherapie, einer Immunsuppressionstherapie, einer Antiinfektivatherapie, einer Wunde, einer Verbrennung, der Gegenwart eines immunosuppressiven Moleküls, einer gastrointestinalen Irritation oder Entzündung oder (5) irgendeiner Kombination des vorher erwähnten.

39. Verwendung nach Anspruch 37 oder 38, wobei das Medikament dem Patienten unter Verwendung eines intermittierenden Dosierungsprotokolls verabreichte wird und der Patient ein Säugetier ist.

40. Verwendung nach Anspruch 37 oder 38, wobei der Immunosuppressions-Zustand oder die unerwünschte Immunantwort in Zusammenhang stehen mit einer Malignität oder einer Präkanzerose.

41. Verwendung nach Anspruch 40, wobei die Malignität oder Präkanzerose Ovarialkrebs, Brustkrebs, Prostatakrebs, gutartige Prostatahyperplasie, ein Gliom, ein Lymphom, eine Leukämie, Dickdarmkrebs, ein Sarkom, nicht-kleinzelliges Lungenkarzinom, Bronchialkarzinom, Nierenzellenkrebs, Nierenzellenkarzinom, Melanom, Pankreas- oder Magenadenokarzinom, mit dem Human-Papilloma-Virus in Zusammenhang stehende zervikale intraepitheliale Neoplasie, Zervixkarzinom, Hepatom, kutanes T-Zell-Lymphom oder ein Krebs oder eine Präkanzerose ist, die im Rachen, Esophagus, Magen, Darm, Dickdarm, Ovarium, Lunge, Brust oder Zentralnervensystem entsteht.

42. Verwendung nach Anspruch 37 oder 38, wobei der Immunosuppressions-Zustand oder die unerwünschte Immunantwort in Zusammenhang steht mit einer virusinfektion, einer intrazellulären Bakterieninfektion, einer extrazellulären Bakterieninfektion, einer Pilzinfektion oder, einer Hefeinfektion.

43. Verwendung nach Anspruch 42, wobei die Virusinfektion, intrazelluläre Bakterieninfektion, extrazelluläre Bakterieninfektion, Pilzinfektion oder Hefeinfektion eine Infektion ist, die verursacht wird durch eine Gruppe, Spezies oder Gattung, ausgewählt aus einem Retrovirus, einem Flavivirus, einem Papillomavirus, HIV-1, HIV-2, HHV-6, HHV-8, Hepatitis-B-Virus, Hepatitis-C-Virus, HSV-1, HSV-2, CMV, *Escherichia, Legionella pneumonia, Listeria, Mycoplasma, Neisseria, Pseudomonas, Rickettsia, Salmonella, Shigella, Streptococcus, Staphylococcus, Yersinia, Trichomonas, Chlamidya, Pneumocystis, Candida, Cryptococcus, Aspergillus, Cryptosporidium, Toxoplasma* und *Pneumocystis*.

44. Verwendung nach Anspruch 37 oder 38, wobei der Immunosuppressions-Zustand oder die unerwünschte Immunantwort in Zusammenhang mit einer Entzündung stehen.

45. Verwendung nach Anspruch 44, wobei die Entzündung eine allergische bronchopulmonale Aspergillose bei Patienten mit zystischer Fibrose, atopisches Asthma allergische Atemwegserkrankung, allergische Rhinitis, atopische Dermatitis, subepitheliale Fibrose bei Luftweg-Überreagibilität, chronischer Sinusitis, perenniale allergische Rhinitis, Crohn-Krankheit, Colitis ulcerosa, entzündliche Darmerkrankung oder fibrosierende Alveolitis ist.

46. Verwendung nach Anspruch 37 oder 38, wobei der Immunosuppressions-Zustand oder die unerwünschte Immunantwort in Zusammenhang mit einer Autoimmunerkrankung stehen.

47. Verwendung nach Anspruch 46, wobei die Autoimmunerkrankung eine systemische Lupus erythematodes, Osteoporose, Multiple Sclerose, Myasthenia gravis, Graves-Krankheit, rheumatoide Arthritis oder Osteoarthritis ist.

48. Verwendung of 16α-Brom-3β-hydroxy-5α-androstan-17-onhemihydrat zur Herstellung eines Medikaments zur Modulation der angeborenen Immunität eines Patienten oder zur Steigerung der Th1-Immunantworten eines Patienten.

49. Verwendung nach Anspruch 48, wobei der Patient einen Suppressionszustand der angeborenen Immunität oder eine unterdrückte Th1-Immunantwort besitzt.

50. Verwendung von 16α-Brom-3β-hydroxy-5α-androstan-17-onhemihydrat zur Herstellung eines Medikaments zur Erhöhung der Anzahl der Neutrophilen, dendritischen Zellen, Monozyten, Makrophagen oder NK-Zellen im Patientenblut.

51. Verwendung nach Anspruch 50, wobei der Patient einen Suppressionszustand der angeborenen Immunität oder eine unterdrückte Th1-Immunantwort besitzt.

52. Verwendung nach Anspruch 51, wobei der Suppressionszustand der angeborenen Immunität oder die unterdrückte Th1-Immunantwort in Verbindung stehen mit einer Virusinfektion, einer intrazellulären Bakterieninfektion, einer extrazellulären Bakterieninfektion, einer Pilzinfektion, einer Hefeinfektion, einer extrazellulären Parasiteninfektion, einer intrazellulären Parasiteninfektion, einer Protozoenparasiteninfektion und einer multizellulären Parasiteninfektion, einer Autoimmunerkrankung, eines Karzinoms, einer Präkanzerose, einer Chemotherapie, einer Strahlentherapie, einer Immunsuppressionstherapie, einer Antiinfektivatherapie, einer Wunde, einer Verbrennung, der Gegenwart eines immunosuppressiven Moleküls oder irgendeiner Kombination des vorher erwähnten.

53. Verwendung nach Anspruch 52, wobei der Patient einen Suppressionszustand der angeborenen Immunität oder eine unterdrückte Th1-Immunantwort besitzt oder deren Entstehung unterliegt, welche in Zusammenhang stehen mit einer Chemotherapie, einer Strahlentherapie, einer Immunsuppressionstherapie oder einer Antiinfektivatherapie.

54. Verwendung nach Anspruch 53, wobei der Suppressionszustand der angeborenen Immunität oder die unterdrückte Th1-Immunantwort in Verbindung stehen mit einer Chemotherapie, einer Strahlentherapie oder einer Immunsuppressionstherapie, wobei die Therapie gegebenenfalls ausgewählt ist aus einer Adriamycin-Behandlung, Cisplatin-Behandlung, Mitomycin C Behandlung, Amphotericin B Behandlung, γ-Strahlentherapie, Nucleosidanaloga-Behandlung für Virusinfektion oder Krebs, Cyclosporin-Behandlung und Corticosteroid-Behandlung.

55. Verwendung nach Anspruch 50, 51 oder 52, wobei die dendritischen Zellen Lin⁻HLA⁻DR⁺CD123⁺ dendritische Zellen oder Lin⁻HLA⁻DR⁺CD11c⁺ dendritische Zellen sind.

56. 16α-Brom-3β-hydroxy-5α-androstan-17-onhemihydrat zur Verwendung als ein Medikament.

## Revendications

1. Semi-hydrate de 16α-bromo-3β-hydroxy-5α-androstan-17-one.

2. Semi-hydrate de 16α-bromo-3β-hydroxy-5α-androstan-17-one selon la revendication 1, **caractérisé par** l'un ou plusieurs de (1) un début d'endotherme d'absorption mesuré par analyse calorimétrique différentielle d'environ 100°C +/- 2°C, (2) deux bandes d'absorption carbonyle à environ 1741 cm⁻¹ et 1750 cm⁻¹ mesurées par spectroscopie infrarouge d'absorption à transformée de Fourier, (3) une teneur en eau d'environ 2,4% poids/poids à environ 2,6% poids/poids mesurée par titrage Karl Fisher et (4) 1, 2, 3 ou plus pics XRD à des valeurs Thêta d'environ 17,8° +/- 0,2°, 23,8° +/- 0,2°, 24,2°+/- 0,2°, 26,9° à 27,2° +/- 0,2°, 28,6° +/- 0,2°, 30,1° +/- 0,2° et 32,2° +/- 0,2° Thêta, dans lequel les pics XRD sont déterminées en utilisant le rayonnement Cu-Kα.

3. Semi-hydrate de 16α-bromo-3β-hydroxy-5α-androstan-17-one selon la revendication 1, **caractérisé en ce qu'**il est broyé à une taille moyenne de particules de 0,01 à 200 µm.

4. Semi-hydrate de 16α-bromo-3β-hydroxy-5α-androstan-17-one selon la revendication 1, **caractérisé en ce qu'**il est broyé à une taille moyenne de particules de 0,1 à 10 µm.

5. Composition comprenant du semi-hydrate de 16α-bromo-3β-hydroxy-5α-androstan-17-one et un excipient convenant pour une utilisation pharmaceutique humaine ou pour une utilisation vétérinaire.

6. Composition selon la revendication 5, sous la forme d'un dosage unitaire qui comprend 3 à 1000 mg de semi-hydrate de 16α-bromo-3β-hydroxy-5α-androstan-17-one.

7. Composition selon la revendication 5 ou 6 sous la forme d'un comprimé, d'une capsule, d'une tablette, d'une solution, d'une suspension, d'un gel ou d'un colloïde.

8. Composition selon la revendications 5 ou 6 sous la forme d'une tablette, d'un comprimé, d'une pastille, d'une tablette, d'une solution, d'une suspension, d'un gel ou d'un colloïde, dans laquelle le dosage unitaire convient pour une administration orale, parentérale, topique, buccale ou sublinguale à un sujet.

9. Procédé de fabrication de semi-hydrate de 16α-bromo-3β-hydroxy-5α-androstan-17-one comprenant la mise en contact d'eau avec une solution comprenant du 16α-bromo-3β-hydroxy-5α-androstan-17-one et un alcool en C1-C6.

10. Procédé selon la revendication 9, dans lequel l'alcool en C1-C6 est de l'éthanol.

11. Procédé selon la revendication 9, dans lequel la solution comprend 5 à 25% poids/poids d'eau, 30 à 45% poids/poids d'éthanol et 30 à 45% poids/poids de 16α-bromo-3β-hydroxy-5α-androstan-17-one.

12. Procédé selon la revendication 9, dans lequel la solution comprend 18 à 22% poids/poids d'eau, 37 à 43% poids/poids d'éthanol et 37 à 43% poids/poids de 16α-bromo-3β-hydroxy-5α-androstan-17-one.

13. Procédé selon la revendication 12, dans lequel la solution est à une température d'environ -20°C à environ 45°C.

14. Utilisation de semi-hydrate de 16α-bromo-3β-hydroxy-5α-androstan-17-one pour préparer un médicament pour le traitement d'un sujet atteint ou susceptible d'être atteint d'immunosuppression ou de réaction immunitaire non souhaitée, étant associées chacune ou toutes deux à (1) une infection pathogène choisie parmi une infection virale, une infection bactérienne intracellulaire, une infection bactérienne extracellulaire, une infection fongique, une infection provoquée par des levures, une infection parasite extracellulaire, une infection parasite intracellulaire, une infection parasite protozoaire, et une infection parasite multicellulaire, (2) une maladie auto-immune, (3) une malignité ou un pré-cancer, (4) une. chimiothérapie, une radiothérapie, une thérapie immunosuppressive, une thérapie par agent anti-infectieux, une plaie, une brûlure, la présence d'une molécule immunosuppressive, une irritation ou une inflammation gastro-intestinale, ou (5) n'importe quelle combinaison de ce qui précède.

15. Utilisation selon la revendication 14, dans laquelle la condition d'immunosuppression ou de réaction immunitaire non souhaitée est associée à une malignité ou un pré-cancer.

16. Utilisation selon la revendication 15, dans laquelle la malignité ou le pré-cancer est un cancer ovarien, un cancer du sein, un cancer de la prostate, une hyperplasie bénigne de la prostate, un gliome, un lymphome, une leucémie, un cancer du côlon, un sarcome, un cancer du poumon à grandes cellules, un carcinome bronchogénique, un cancer des cellules rénales, un carcinome des cellules rénales, un mélanome, un adénocarcinome pancréatique ou gastrique, une néoplasie intraépithéliale cervicale associée à un virus de papillome humain, un carcinome cervical, un hépatome, un lymphome cutané des lymphocytes T ou un cancer ou un pré-cancer survenant dans la gorge, l'oesophage, l'estomac, l'intestin, le côlon, les ovaires, les poumons, le sein ou le système nerveux central.

17. Utilisation selon la revendication 14, dans laquelle la condition d'immunosuppression ou de réaction immunitaire non souhaitée est associée à une infection virale, une infection bactérienne intracellulaire, une infection bactérienne extracellulaire, une infection fongique, une infection provoquée par des levures, une infection parasite extracellulaire, une infection parasite intracellulaire, une infection parasite protozoaire et une infection parasite multicellulaire.

18. Utilisation selon la revendication 17, dans laquelle l'infection virale, l'infection bactérienne intracellulaire, l'infection bactérienne extracellulaire, l'infection fongique, l'infection provoquée par des levures, une infection parasite extracellulaire, une infection parasite intracellulaire, une infection parasite protozoaire ou une infection parasite multicellulaire est une infection provoquée par un groupe, des espèces ou un genre choisi parmi un rétrovirus, un togavirus, un flavivirus, un hépadnavirus, un virus d'herpès, un virus de papillome, HIV-1, HIV-2, HHV-6, HHV-8, le virus de l'hépatite B, le virus de l'hépatite C, HSV-1, HSV-2, un cytomégalovirus CMV, *Escherichia, Haemophilus, Legionella pneumophila, Listeria, Mycoplasma, Neisseria, Pseudomonas, Rickettsia, Salmonella, Shigella, Streptococcus, Staphylococcus, Yersinia, Pneumocystis, Candida, Cryptococcus, Aspergillus, Cryptosporidium, Toxoplasma, Trichomonas, Chlamydia,* *Pneumoncystis, Trypanosoma, Leishmania, Plasmodium*, un helminthe gastro-intestinal, un parasite Microsporidium ou un parasite Isosporum.

19. Utilisation selon la revendication 18, dans laquelle un médicament comprenant un agent antiviral, un agent antibactérien, un agent antifongique ou un agent antiparasite est facultativement administré en conjonction avec le médicament de la revendication 14.

20. Utilisation selon la revendication 19, dans laquelle l'agent antiviral, l'agent antibactérien ou l'agent antifongique est un inhibiteur de transcriptase inverse, un inhibiteur de protéase, un antibiotique, un inhibiteur de fusion, un γ-interféron, une amantadine, une rimantadine, une ribavirine, une chloroquine ou un analogue de chloroquine.

21. Utilisation selon la revendication 19 ou 20, dans laquelle l'agent antiviral est choisi parmi AZT, 3TC, D4T, ddl, ddC, l'adéfovir dipivoxil, 9[2-(R)-[[bis[[(isopropoxycarbonyl)-oxy]méthoxy]phosphinoyl]méthyoxy]propyl]adénine, (R)-9[2-(phosphonométhoxy)propyl]adénine, l'adéfovir, l'indinavir, le nelfinavir, le rilonavir, le crixivan, le séquanavir et l'hydroxycarbamide.

22. Utilisation selon la revendication 21, dans laquelle l'agent antibactérien ou l'agent antifongique est choisi parmi l'amphotéricine B, le fluconazole, le clotrimazole, l'isoniazide, le dapsone, la rifampine, la cyclosérine, l'érythromicine, un antibiotique tétracycline, le vancomycine, l'éthambutol, la pyrazinamide, un fluorquinolone choisi parmi la ciprofloxacine et la norfloxacine, un antibiotique céphalosporine, un antibiotique β-lactame ou un antibiotique aminoglycoside facultativement choisi parmi la streptomycine, la kanamycine, et la tobramycine.

23. Utilisation selon la revendication 14, dans laquelle la condition d'immunosuppression ou de réaction immune non souhaitée est associée à une inflammation.

24. Utilisation selon la revendication 23, dans laquelle l'inflammation est une aspergillose broncho-pulmonaire allergique chez des patients atteints de mucoviscidose, de l'asthme allergique, une maladie respiratoire allergique, une rhinite allergique, une dermatite allergique, une fibrose sous-épithéliale lors d'hyper-réaction des voies aériennes, une sinusite chronique, une rhinite allergique apériodique, une maladie de Crohn, une colite ulcérative, une maladie inflammatoire du côlon ou une alvéolite fibreuse.

25. Utilisation selon la revendication 14, dans laquelle la condition d'immunosuppression ou de réaction immunitaire non souhaitée est associée à une maladie auto-immune.

26. Utilisation selon la revendication 25, dans laquelle la maladie auto-immune est un lupus érythémateux disséminé, une ostéoporose, une sclérose en plaques, une myasthénie gravis, une maladie de Graves, une polyarthrite rhumatoïde ou de l'arthrose.

27. Utilisation selon les revendications 14 à 26, dans laquelle le médicament est administré au sujet en utilisant un protocole de dosage intermittent.

28. Utilisation selon la revendication 27, dans laquelle le sujet est un mammifère.

29. Utilisation de semi-hydrate de 16α-bromo-3β-hydroxy-5α-androstan-17-one pour préparer un médicament pour la modulation d'une cytokine ou d'une interleukine qui facilite les réactions immunes Th1 ou Th2 chez un sujet.

30. Utilisation selon la revendication 29, dans laquelle la cytokine ou l'interleukine est IL-1, IL-2, IL-4, IL-6, IL-8, IL-10, IL-12, γlFN ou TNFα.

31. Utilisation selon la revendication 30, dans laquelle la cytokine ou l'interleukine est IL-12.

32. Utilisation selon la revendication 30, dans laquelle la cytokine ou l'interleukine est IL-1.

33. Utilisation selon la revendication 30, dans laquelle la cytokine ou l'interleukine est IL-6.

34. Utilisation selon la revendication 30, dans laquelle la cytokine ou l'interleukine est IL-10.

35. Utilisation selon la revendication 30, dans laquelle la cytokine ou l'interleukine est TNFα.

36. Utilisation selon les revendications 29 à 35, dans laquelle le médicament est administré au sujet en utilisant un protocole de dosage intermittent et le sujet est un mammifère.

37. Utilisation de semi-hydrate de 16α-bromo-3β-hydroxy-5α-androstan-17-one pour préparer un médicament pour l'amélioration des cellules dendritiques Lin⁻ HLA-DR⁺ CD123⁺, des cellules dendritiques Lin⁻ HLA-DR⁺ CD11c⁺, des lymphocytes LAK CD8⁺CD16⁺CD38⁺, des lymphocytes LAK CD8⁺CD16⁺CD38⁺, des cellules tueuses naturelles CD8⁻CD16⁺, des cellules tueuses naturelles CD8⁺CD16⁺, des cellules CD8⁻CD16⁺ qui facilitent la cytotoxicité facilitée par les cellules et dépendant des anticorps, des cellules CD8⁺CD16⁺ qui facilitent la cytotoxicité facilitée par les cellules et dépendant des anticorps chez un sujet, des lymphocytes T CD45RA⁺ ou des lymphocytes T CD45⁺RO⁺ chez un sujet.

38. Utilisation selon la revendication 37, dans laquelle le sujet possède une condition d'immunosuppression ou de réaction immune non souhaitée, associées chacune ou toutes deux à (1) une infection pathogène choisie parmi une infection virale, une infection bactérienne intracellulaire, une infection bactérienne extracellulaire, une infection fongique, une infection provoquée par des levures, une infection parasite extracellulaire, une infection parasite intracellulaire, une infection parasite protozoaire, ou une infection parasite multicellulaire, (2) une maladie auto-immune, (3) une malignité ou un pré-cancer, (4) une chimiothérapie, une radiothérapie, une thérapie immunosuppressive, une thérapie par agent anti-infectieux, une plaie, une brûlure, la présence d'une molécule immunosuppressive, une irritation ou une inflammation gastro-intestinale, ou (5) n'importe quelle combinaison de ce qui précède.

39. Utilisation selon la revendication 37 ou 38, dans laquelle le médicament est administré au sujet en utilisant un protocole de dosage intermittent et dans laquelle le sujet est un mammifère.

40. Utilisation selon la revendication 37 ou 38, dans laquelle la condition d'immunosuppression ou de réaction immune non souhaitée est associée à une malignité ou un pré-cancer.

41. Utilisation selon la revendication 40, dans laquelle la malignité ou le pré-cancer est cancer ovarien, un cancer du sein, un cancer de la prostate, une hyperplasie bénigne de la prostate, un gliome, un lymphome, une leucémie, un cancer du côlon, un sarcome, un cancer du poumon à grandes cellules, un carcinome brochogénique, un cancer des cellules rénales, un carcinome des cellules rénales, un mélanome, un adénocarcinome pancréatique ou gastrique, une néoplasie intraépithéliale cervicale associée à un virus de papillome humain, un carcinome cervical, un hépatome, un lymphome cutané des lymphocytes T ou un cancer ou un pré-cancer survenant dans la gorge, l'oesophage, l'estomac, l'intestin, le côlon, les ovaires, les poumons, le sein ou le système nerveux central.

42. Utilisation selon la revendication 37 ou 38, dans laquelle la condition d'immunosuppression ou de réaction immune non souhaitée est associée à une infection virale, une infection bactérienne intracellulaire, une infection bactérienne extracellulaire, une infection fongique ou une infection provoquée par des levures.

43. Utilisation selon la revendication 42, dans laquelle l'infection virale, l'infection bactérienne intracellulaire, l'infection bactérienne extracellulaire, l'infection fongique ou l'infection provoquée par des levures est une infection provoquée par un groupe, des espèces ou un genre choisi parmi un rétrovirus, un togavirus, un flavivirus, un virus de papillome, HIV-1, HIV-2, HHV-6, HHV-8, le virus de l'hépatite B, le virus de l'hépatite C, HSV-1, HSV-2, le cytomégalovirus CMV, *Escherichia, Legionella pneumonia, Listeria, Mycoplasma, Neisseria, Pseudomonas, Rickettsia, Salmonella, Shigella, Streptococcus, Staphylococcus, Yersinia, Trichomonas, Chlamidya, Pneumocystis, Candida, Cryptococcus, Aspergillus, Cryptosporidium, Toxoplasma et Pneumocystis*.

44. Utilisation selon la revendication 37 ou 38, dans laquelle la condition d'immunosuppression ou de réaction immunitaire non souhaitée est associée à une inflammation.

45. Utilisation selon la revendication 44, dans laquelle l'inflammation est une aspergillose broncho-pulmonaire allergique chez des patients atteints de mucoviscidose, de l'asthme allergique, une maladie respiratoire allergique, une rhinite allergique, une dermatite allergique, une fibrose sous-épithéliale lors d'hyper-réaction des voies aériennes, une sinusite chronique, une rhinite allergique apériodique, une maladie de Crohn, une colite ulcérative, une maladie inflammatoire du côlon ou une alvéolite fibreuse.

46. Utilisation selon la revendication 37 ou 38, dans laquelle la condition d'immunosuppression ou de réaction immunitaire non souhaitée est associée à une maladie auto-immune.

47. Utilisation selon la revendication 46, dans laquelle la maladie auto-immune est un lupus érythémateux disséminé, une ostéoporose, une sclérose en plaques, une myasthénie gravis, une maladie de Graves, une polyarthrite rhumatoïde ou de l'arthrose.

48. Utilisation de semi-hydrate de 16α-bromo-3β-hydroxy-5α-androstan-17-one pour préparer un médicament pour la modulation d'une immunité naturelle d'un sujet où l'amélioration des réactions immunes Th1 d'un sujet.

49. Utilisation selon la revendication 48, dans laquelle le sujet possède une condition de suppression d'immunité naturelle ou une réaction immunitaire Th1 supprimée.

50. Utilisation de semi-hydrate de 16α-bromo-3β-hydroxy-5α-androstan-17-one pour préparer un médicament pour augmenter le nombre de neutrophiles, de cellules dendritiques, de monocytes, de macrophages ou de cellules tueuses naturelles dans le sang d'un sujet.

51. Utilisation selon la revendication 50, dans laquelle le sujet possède une condition de suppression d'immunité naturelle ou une réaction immunitaire Th1 supprimée.

52. Utilisation selon la revendication 51, dans laquelle la condition de suppression d'immunité naturelle ou la réaction immunitaire Th1 supprimée est associée à une infection virale, une infection bactérienne intracellulaire, une infection bactérienne extracellulaire, une infection fongique, une infection provoquée par des levures, une infection parasite extracellulaire, une infection parasite intracellulaire, un parasite protozoaire, et un parasite multicellulaire, une maladie auto-immune, un cancer, un pré-cancer, une chimiothérapie, une radiothérapie, une thérapie immunosuppressive, une thérapie par agent anti-infectieux, une plaie, une brûlure, la présence d'une molécule immunosuppressive ou n'importe quelle combinaison de ce qui précède.

53. Utilisation selon la revendication 52, dans laquelle le sujet présente ou est soumis au développement d'une condition de suppression d'immunité naturelle ou d'une réaction immunitaire Th1 supprimée qui est associée à une chimiothérapie, une radiothérapie, une thérapie immunosuppressive ou une thérapie par agent anti-infectieux.

54. Utilisation selon la revendication 53, dans laquelle la condition de suppression d'immunité naturelle ou de réaction immunitaire Th1 supprimée est associée à une chimiothérapie, une radiothérapie, ou une thérapie immunosuppressive, dans laquelle la thérapie est facultativement choisie parmi un traitement à l'adriamycine, un traitement à la cisplatine, un traitement à la mitomycine C, un traitement à l'amphotéricine B, une thérapie par rayonnement γ, un traitement par analogue de nucléoside pour une infection virale ou un cancer, un traitement à la cyclosporine ou un traitement aux corticostéroïdes.

55. Utilisation selon les revendications 50, 51 ou 52, dans laquelle les cellules dendritiques sont des cellules dendritiques Lin⁻HLA-DR⁺CD123⁺ ou des cellules dendritiques Lin⁻HLA-DR⁺CD11c⁺.

56. Semi-hydrate de 16α-bromo-3β-hydroxy-5α-androstan-17-one pour utilisation comme médicament.
